# EUROPEAN PATENT APPLICATION

(11) **EP 1 400 588 A1**
(43) Date of publication of application: **24.03.2004**
(21) Application number: 02741299.8
(22) Date of filing: 25.06.2002
(51) Int. Cl.: C12N 15/09, C12Q 1/68, G01N 33/53

(54) **POLYNUCLEOTIDE PROBE AND PRIMER ORIGINATING IN HEPATITIS E VIRUS OF JAPANESE, CHIPS HAVING THE SAME, KITS HAVING THE SAME AND METHOD OF DETECTING HEPATITIS E VIRUS USING THE SAME**

(30) Priority: 25.06.2001 JP 2001191837
(71) Applicant: Kabushiki Kaisha Toshiba, Tokyo 105-8001 (JP)
(72) Inventor: TAKAHASHI, Kazuaki, Asao-ku, Kawasaki-shi, Kanagawa 215-0021 (JP); MISHIRO, Shunji, Bunkyo-ku, Tokyo 113-0021 (JP); OOTA, Yasuhiko, Nakano-ku, Tokyo 165-0026 (JP); HASHIMOTO, Michie, Tokyo 150-0001 (JP); MAEKUBO, Hiroshi, Chuo-ku, Sapporo-shi, Hokkaido 640-952 (JP)
(74) Representative: Andrews, Timothy Stephen
(86) International application number: PCT/JP2002/006365
(87) International publication number: WO 2003/000887

(57) **Abstract**

A polynucleotide probe including a sequence comprising at least eight nucleotides, the polynucleotide probe being used for detecting polynucleotides of hepatitis E virus.The sequence comprising at least eight nucleotides is hybridized with the polynucleotide of the hepatitis E virus, thereby, due to the hybridization, allowing the detection of the hepatitis E virus.

## Description

### Technical Field

The present invention relates to a novel method for detecting hepatitis E virus. The present invention also relates to a novel strain of hepatitis E virus recovered from Japanese, a novel strain of hepatitis E virus from a patient with fulminant hepatitis; and polynucleotide derived therefrom, which is important for establishing the novel method for detecting the RNA genome of hepatitis E virus.

### Background Art

Hepatitis E virus (which will be referred to as "HEV" hereinafter) which replicates in the liver of a patient is voided to feces rather than staying in blood. Accordingly, HEV is transmitted mainly by feco-oral route. Thus, HEV infection sometimes happens as a local outbreak caused by contamination of a water system. Due to such a manner of infection, hepatitis E caused by HEV is frequently observed in regions where the sanitary environment is not satisfactory, such as Asia and Africa. On the contrary, HEV infection is relatively rare in the industrially advanced countries such as Japan, US and Europe. Most of the hepatitis E cases, which are occasionally reported in these advanced countries, are found in travelers who have been to the regions where the disease is endemic and recently come back to their own countries. Accordingly, hepatitis E is generally recognized in the advanced countries as an "imported disease".

HEV was genetically cloned by Reyes of Genelabs Co., in the United States, in 1990, for the first time. The entire nucleotide sequence of the HEV genome was then revealed by the study thereafter. The HEV strains whose full-genome sequence had been analyzed were what is called the "Mexico" strain and the "Burma" strain. Thereafter, the sequences of the genomes derived from the "India" strain, the "Pakistan" strain, the "Nepal" strain, the "Burma" strain, the "China" strain, the "US" strain and the like have been sequentially been revealed. However, the nucleotide sequence of the HEV gene derived from the "Japan" strain has not been revealed yet.

The conventional method of diagnosing HEV infection includes: a method of conducting a PCR method by using a primer designed on the basis of the nucleotide sequences of the aforementioned various known HEV strains, for detecting the RNA genome of the virus; and a method of using, as an antigen, a peptide/protein which has been synthesized/expressed on the basis of the amino acid sequence of the known HEV strains, for detecting an antibody specific thereto.

In the conventional method or technique of diagnosing HEV infection, if an unknown HEV strain having a line different from the known HEV strains exists in the sample to be tested, there is a significant possibility that the unknown virus cannot be detected.

### Disclosure of Invention

An object of the present invention is to provide a method which enables "wide-range" detection of HEV belonging to various strains. Another object of the present invention is to provide polynucleotide for widely detecting HEV belonging to various strains and polynucleotide for determining the strain of the detected HEV.

Yet another object of the present invention is to provide polynucleotide derived from a novel HEV strain endemic to Japan, polynucleotide derived from a novel strain of fulminant HEV, and polypeptides coded by these nucleotide sequences.

A further object of the present invention is to provide a method for carrying out a drug design, which drug design is achieved by utilizing the genetic information of the novel HEV described above and the genetic information of the conventional known HEV.

The inventors of the present invention have molecularly isolated, for the first time in the world, the HEV strains (specifically the HEV Japan JRA1 strain, the JKN-Sap strain, the JMY-Haw strain, JKK-Sap strain and JAK-Sai strain) from Japanese patients, isolated HEV Japan JSN-FH strain as a novel HEV strain from a case of fulminant hepatitis, and determined the genome sequence of the obtained virus. The present invention has been achieved on the basis of these discoveries.

According to a first aspect of the present invention, there is provided a polynucleotide probe including a sequence comprising at least eight nucleotides, the polynucleotide probe being used for detecting the genomic polynucleotide of hepatitis E virus, characterized in that:
(1) the sequence comprising at least eight nucleotides is hybridized with the polynucleotide of the hepatitis E virus, thereby, due to the hybridization, detecting the hepatitis E virus; and
(2) the sequence comprising at least eight nucleotides is obtained from a sequence selected from the group consisting of nucleotide sequences disclosed at SEQ No. 11, SEQ No. 44, SEQ No. 45, SEQ No. 46, SEQ No. 47 and SEQ No. 48 and complementary strands thereof.

According to a second aspect of the present invention, there is provided a pair or plural pairs of primer for PCR for amplifying polynucleotide of hepatitis E virus, the at least a pair of primer for PCR each independently having a sequence comprising at least eight nucleotides, characterized in that:
(1) the sequence comprising at least eight nucleotides is hybridized with the genomic polynucleotide of the hepatitis E virus, thereby, due to the hybridization, amplifying a portion of the polynucleotide of the hepatitis E virus; and
(2) the sequence comprising at least eight nucleotides is obtained from a sequence selected from the group consisting of nucleotide sequences disclosed at SEQ No. 11, SEQ No. 44, SEQ No. 45, SEQ No. 46, SEQ No. 47 and SEQ No. 48 and complementary strands thereof.

According to a third aspect of the present invention, there is provided a method of detecting presence of hepatitis E virus in a sample, comprising:
(1) obtaining a sample from an object;
(2) reacting the sample obtained in the obtaining of (1) with the polynucleotide probe according to the first aspect;
(3) detecting a double strand produced as a result of the reaction of the reacting of (2);
(4) determining whether or not hepatitis E virus is present in the sample, on the basis of the detection result of the detecting of (3).

According to a fourth aspect of the present invention, there is provided a method for detecting the presence of hepatitis E virus in a sample, comprising:
(1) obtaining a sample from an object;
(2) reacting the sample obtained in the obtaining of (1) with a pair of primers for PCR according to the second aspect of the invention and a polymerase, under a condition in which amplification is effected in an appropriate manner
(3) detecting presence of a product obtained as a result of amplification by the reaction of the reacting of (2);
(4) determining whether or not hepatitis E virus is present in the sample, on the basis of the detection result of the detecting of (3).

According to a fifth aspect of the present invention, there is provided a method for determining the genotype of hepatitis E virus in a sample, comprising:
(1) reacting a sample with a pair of primers for PCR according to a second aspect of the invention and a polymerase, under a condition in which amplification is effected in an appropriate manner;
(2) determining length of a product obtained as a result of amplification by the reaction of the reacting of (1) ;
(3) determining genotype of hepatitis E virus present in the sample, on the basis of the detection result of the determining of (2).

According to a sixth aspect of the present invention, there is provided a probe assay kit, including the polynucleotide probe according to the first aspect.

According to a seventh aspect of the present invention, there is provided a PCR assay kit, including the pair or plural pairs of primer for PCR according to the second aspect.

According to an eighth aspect of the present invention, there is provided a chip for detecting a nucleotide sequence, on which the polynucleotide probe according to the first aspect has been immobilized.

### Brief Description of Drawings

FIG. 1 is a view showing a phylogenetic tree of a hepatitis E virus strain.
FIG. 2 is a view showing a genetic organization of HEV JRA1.
FIG. 3A and FIG. 3B are views showing a portion of the nucleotide sequence of HEV JRA1 and a portion of the nucleotide sequence of the "Mexico" strain in a manner that the former is compared with the latter.
FIG. 4A and FIG. 4B are, in addition to FIGS. 3A and 3B, views showing a portion of the nucleotide sequence of HEV JRA1 and a portion of the nucleotide sequence of the "Mexico" strain in a manner that the former is compared with the latter.
FIG. 5A and FIG. 5B are, in addition to FIGS. 4A and 4B, views showing a portion of the nucleotide sequence of HEV JRA1 and a portion of the nucleotide sequence of the "Mexico" strain in a manner that the former is compared with the latter.
FIG. 6 is a view showing a primer and a probe according to embodiments of the present invention.
FIG. 7 is a view showing a portion of the nucleotide sequence of HEV JSN-FH and a portion of the nucleotide sequence of the "Mexico" strain in a manner that the former is compared with the latter.
FIG. 8 is, in addition to FIG. 7, a view showing a portion of the nucleotide sequence of HEV JSN-FH and a portion of the nucleotide sequence of the "Mexico" strain in a manner that the former is compared with the latter.
FIG. 9 is, in addition to FIG. 8, a view showing a portion of the nucleotide sequence of HEV JSN-FH and a portion of the nucleotide sequence of the "Mexico" strain in a manner that the former is compared with the latter.
FIG. 10 is, in addition to FIG. 9, a view showing a portion of the nucleotide sequence of HEV JSN-FH and a portion of the nucleotide sequence of the "Mexico" strain in a manner that the former is compared with the latter.
FIG. 11 is, in addition to FIG. 10, a view showing a portion of the nucleotide sequence of HEV JSN-FH and a portion of the nucleotide sequence of the "Mexico" strain in a manner that the former is compared with the latter.
FIG. 12 is, in addition to FIG. 11, a view showing a portion of the nucleotide sequence of HEV JSN-FH and a portion of the nucleotide sequence of the "Mexico" strain in a manner that the former is compared with the latter.
FIG. 13 is a view showing the ORF regions of various HEV strains in a manner that the ORF regions are compared with one another.
FIG. 14 is a view showing a phylogenetic tree of the HEV strain.
FIG. 15 is a view showing the sequence of the ORF1 of various HEV strains in a manner that the ORF1 regions are compared with one another.
FIG. 16 is, in addition to FIG. 15, a view showing the sequence of the ORF1 of various HEV strains in a manner that the ORF1 regions are compared with one another.
FIG. 17 is, in addition to FIG. 16, a view showing the sequence of the ORF1 of various HEV strains in a manner that the ORF1 regions are compared with one another.
FIG. 18 is, in addition to FIG. 17, a view showing the sequence of the ORF1 of various HEV strains in a manner that the ORF1 regions are compared with one another.
FIG. 19 is a view showing the data which indicates the presence of RNA of HEV in a serum sample collected from a patient, which data is shown combined with changes in the liver function test values.
FIG. 20 is a chart showing results of HEV detection, obtained from samples of patients of non A, B, C acute hepatitis by using the primer according to embodiments of the present invention.
FIG. 21 is a chart showing results of HEV detection, which was successively conducted for a patient, according to embodiments of the present invention.
FIG. 22 is a view showing a result of detection of the amplified HEV genome by electrophoresis, according to embodiments of the present invention.
FIGS. 23A to 23E are views showing the relationship between the types of a probe, the genotype of HEV and absorbance.

### Best Mode for Carrying Out of the Invention

### I. Terms

The term "polynucleotide" used in the present specification inclusively represents, for convenience, polynucleotide, oligonucleotide and the like. Further, the term "polynucleotide" used in the present specification represents a substance resulted from phosphoric ester-bonding of no less than two nucleoside. Nucleoside generally includes deoxyribonucleoside and ribonucleoside, without being limited thereto. "Oligonucleotide" represents a substance obtained by polymerizing, by means of phosphodiester bonding, phosphoric esters of a few to dozens of nucleoside (i.e., nucleotide). Oligonucleotide generally includes oligoribonucleotide and oligodeoxyribonucleotide, without being limited thereto. The polynucleotide according to embodiments of the present invention may be either virus-genome RNA recovered from the HEV JRA1 strain or DNA (or the like) obtained from the virus-genome RNA. Further, "polynucleotide" of the present invention may include artificially synthesized nucleic acid such as peptide nucleic acid, morpholino nucleic acid, methylphosphonate nucleic acid and S-oligo nucleic acid.

The "polypeptide" used in the present specification generally represents a peptide composed of no less than two amino acids, an oligopeptide or a protein. The "polypeptide" used in the present specification includes both synthesized peptide and expressed protein, without being limited thereto. The "polypeptide" may be either a simple protein composed of only amino acids or a composite protein containing components other than amino acids.

The term "object" used in the present specification may be one of any mammal or the like, including human, dog, cat, cow, goat, pig, sheep and monkey. In the present embodiment, a human is the most suitable object.

The term "sample" used in the present specification represents a biosample such as blood, serum, stool, liver and lymph, collected from an individual or biont as the object. The "sample" of the present invention may also be obtained by subjecting a biosample to any necessary preparatory treatment such as homogenization, extraction or the like. Such a preparatory treatment will be easily selected by one skilled in the art, in accordance with the biosample used as the object.

The term "open reading frame" used in the present specification represents a region of a polynucleotide sequence which codes a polypeptide. This region represents at least a portion of the coding sequence.

The term "hepatitis E virus" used in the present specification represents the virus as the primary cause of epidemic non-A, non-B hepatitis, which spreads by the medium of drinking water, is transmitted by way of digestive tract and is observed mainly in Asia and Africa. The hepatitis E virus is RNA virus and classified into genus calicivirus of the family caliciviridae. The term "HEV" used in the present specification comprehensively represents a virus belonging to the genotypes I, II, III and IV, commonly used for virus classification. Further, according to embodiments the present invention, any strain which belongs to HEV and has not been isolated/identified can be detected and/or identified or classified into a genotype. Accordingly, the term "HEV" used in the present specification can include an unknown HEV and a virus which is detected and/or identified or classified into a genotype according to embodiments the present invention. "HEV" in the present specification includes a wild type, a variant, and a strain which is genetically close to HEV. Specifically, "HEV" in the present specification represents a strain which shows homology of approximately 50% or more, preferably 60% or more, and most preferably 65% or more, with respect to the sequence of approximately 2500 nucleotides on the 5' end side of JRA1 shown in Sequence No. 1.

### II. Strain of novel hepatitis E virus

The inventors of the present invention have assumed that an HEV strain which is indigenous to Japan does exist, on the basis of the fact that hepatitis E cases have been observed in Japan even among those who have never been abroad, although the number of these cases is relatively small. Under this assumption, the inventors of the present invention have attempted cloning of HEV genomes recovered from Japanese patients who developed acute hepatitis and, as a result, succeeded in isolating five HEV strains which appear to belong to a novel line.

These novel HEV strains are five strains named as follows by the inventors: HEV Japan JRA1 strain (Hepatitis E virus Japan JRA1, also referred to as "JRA1 strain" hereinafter, NCBI accession No. AP003430); HEV Japan JKN-Sap strain (Hepatitis E virus Japan JKN-Sap, also referred to as "JKN-Sap strain" hereinafter, NCBI accession No. AB074918); HEV Japan JMY-Haw strain (Hepatitis E virus Japan JMY-Haw, also referred to as "JMY-Haw strain" hereinafter, NCBI accession No. AB074920); HEV Japan JKK-Sap strain (Hepatitis E virus Japan JKK-Sap, also referred to as "JKK-Sap strain" hereinafter, NCBI accession No. AB074917); and HEV Japan JAK-Sai strain (Hepatitis E virus Japan JAK-Sai, also referred to as "JAK-Sai strain" hereinafter, NCBI accession No. AB074915). With regards to the origin of each virus strain, JKK-Sap, JKN-Sap and JMY-Haw were derived from acute hepatitis E patients from Hokkaido, JAK-Sai is derived from an acute hepatitis E patient from Saitama prefecture, and JRA1 is derived from a patient from Tokyo.

Further, the inventors of the present invention have cloned the HEV JSN-FH strain (Hepatitis E virus JSN-FH, which will occasionally be referred to as "JSN-FH strain") as a novel HEV strain from a fulminant hepatitis case.

Open reading frame 1 (which will be referred to as "ORF1" hereinafter) of the genome RNA sequence of JRA1 strain is shown at the sequence (SEQ) No. 1, and the whole length of this genome RNA sequence is shown at the SEQ No. 48.

The semi-whole length of the genome RNA sequence of JSN-FH strain, which does not include a portion of non-coding sequence at the 5' end side of the genome RNA sequence, is shown at the SEQ No. 11. The amino acid sequences which are coded in ORF1 and ORF 2 of the genome RNA sequence of JSN-FH strain are shown at the SEQ No. 12 and the SEQ No. 13, respectively.

A portion of ORF1 of the genome RNA sequence of each of JKN-Sap strain, JMY-Haw strain, JAK-Sai strain and JKK-Sap strain is shown at the SEQ No. 16, the SEQ No. 17, the SEQ No. 20 and the SEQ No. 21, respectively. Further, the whole length of the genome RNA sequence of each of JKN-Sap strain, JMY-Haw strain, JAK-Sai strain and JKK-Sap strain is shown at the SEQ No. 44, the SEQ No. 45 (the semi-whole length), the SEQ No. 46 (the semi-whole length) and the SEQ No. 47 (the semi-whole length), respectively.

### 2. HEV JRA1 strain

### (1) HEV JRA1 strain

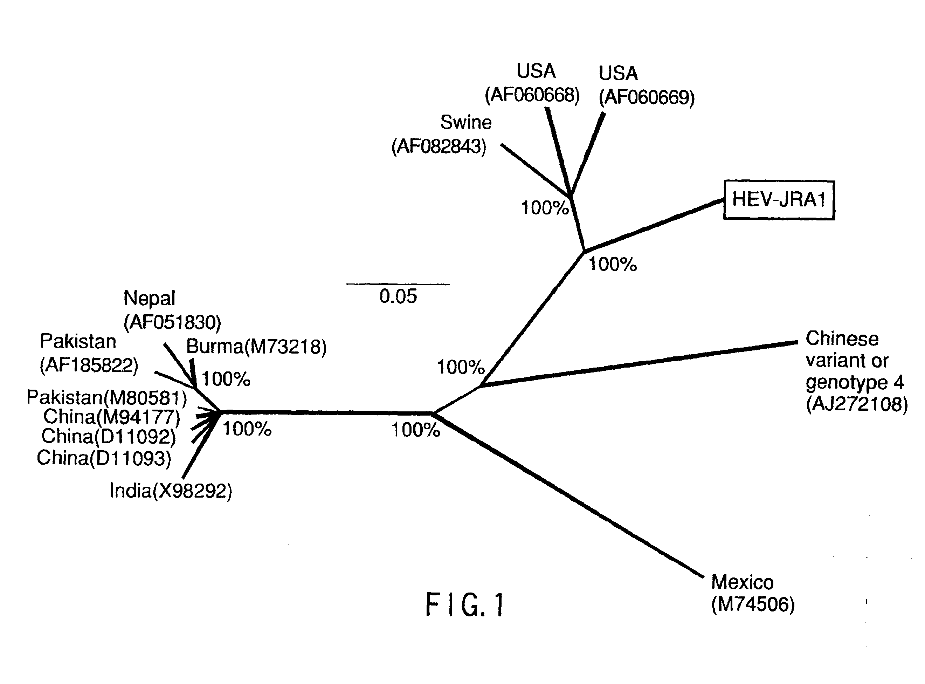

The inventors of the present invention have revealed that the nucleotide sequence of JRA1 strain disclosed at the SEQ No. 1 belongs to a novel genotype, as compared with the known strains of various types collected from various areas in the world, as shown in the phylogenetic tree of FIG. 1.

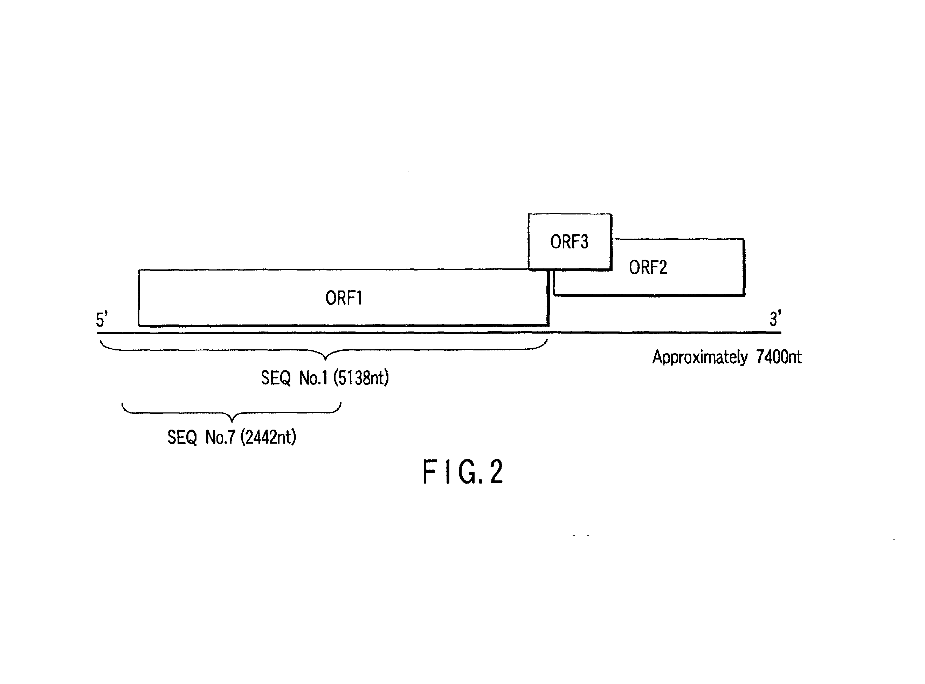

The difference in nucleotide sequence between the known strains and JRA1 strain is particularly obvious in the approximately 2500 nucleotides at the genome 5' end. As shown in FIG. 2, the hepatitis E virus genome, including HEV-JRA1, generally contains three open reading frames (which will be referred to as "ORF" hereinafter) of ORF1, ORF2 and ORF3. The approximately 5000 nucleotides at the 5' end is included to ORF1. The genome regions at the downstream side of ORF1, i.e., the other regions including the ORF2 region and the ORF3 region exhibit relatively high sequence-conservation between strains, as compared with the ORF1 region.

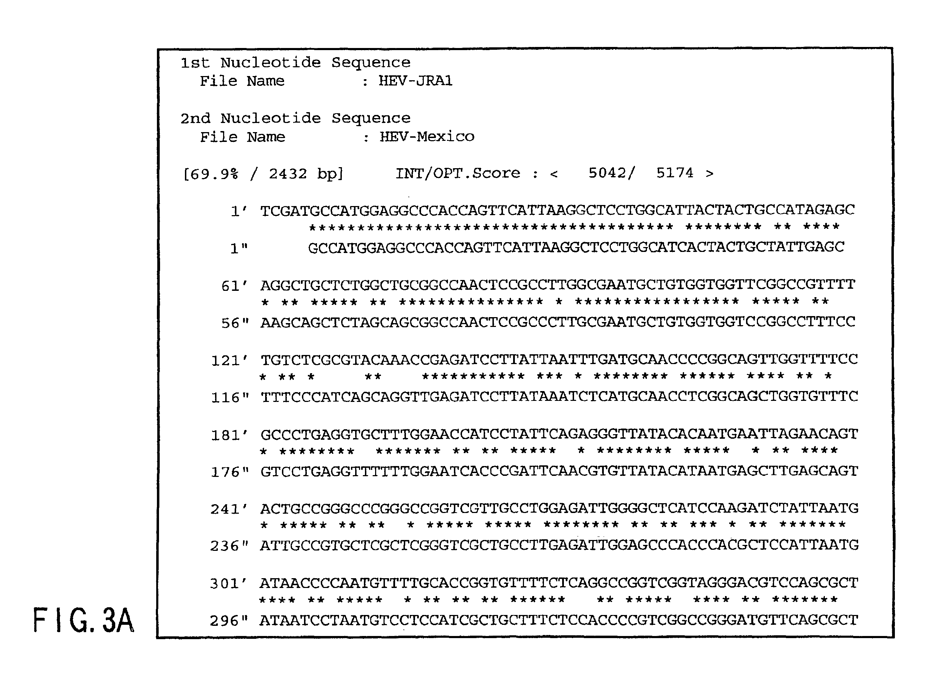

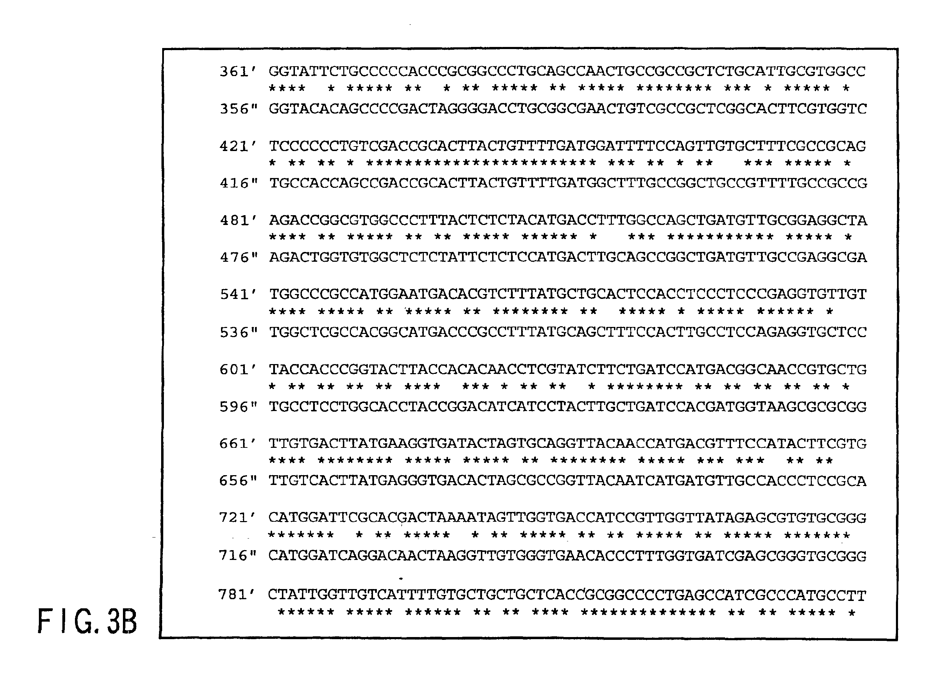

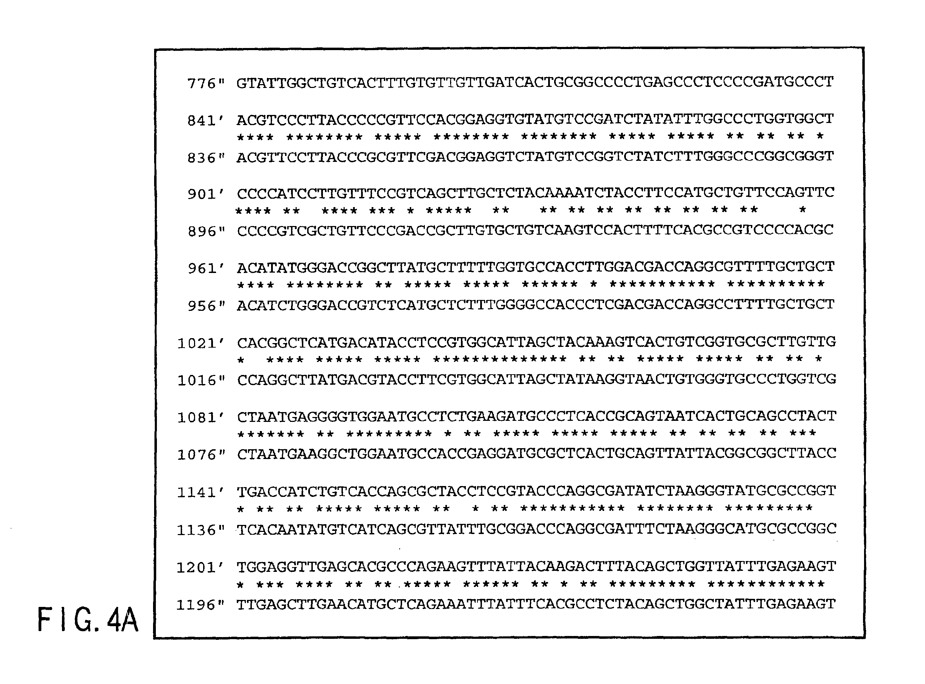

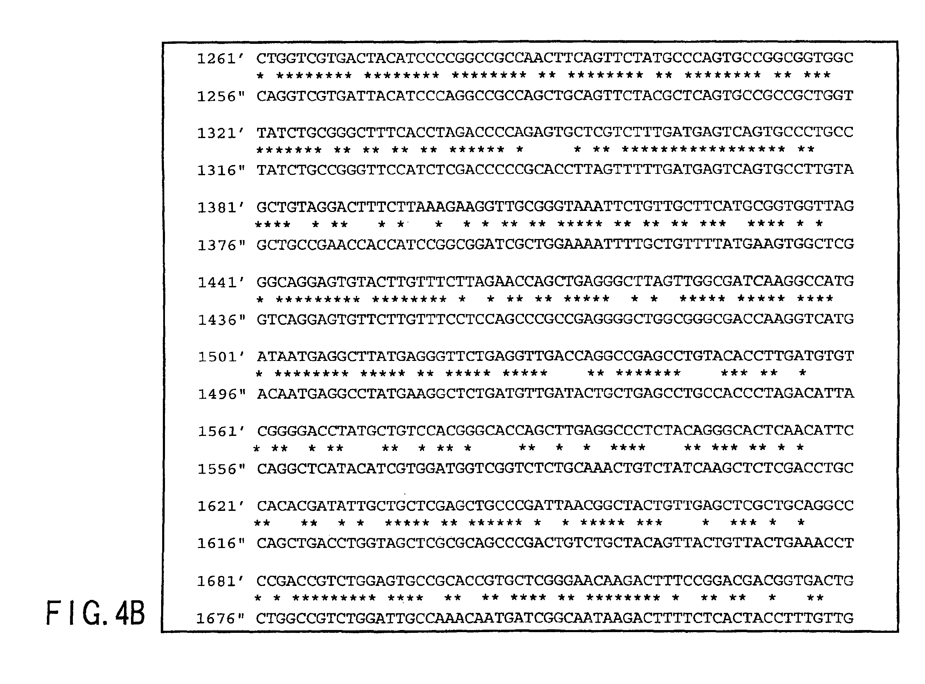

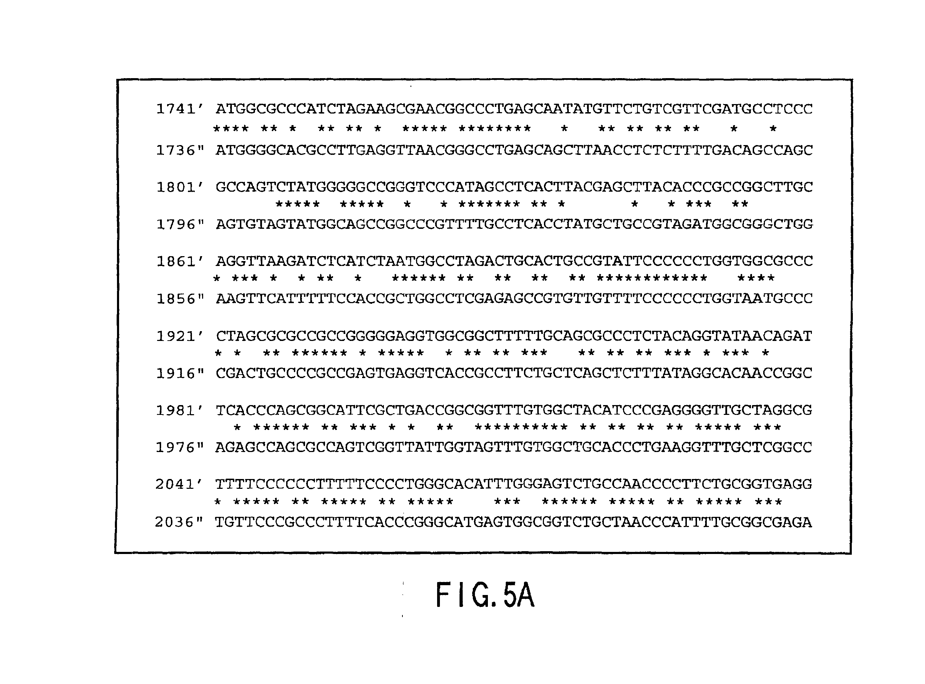

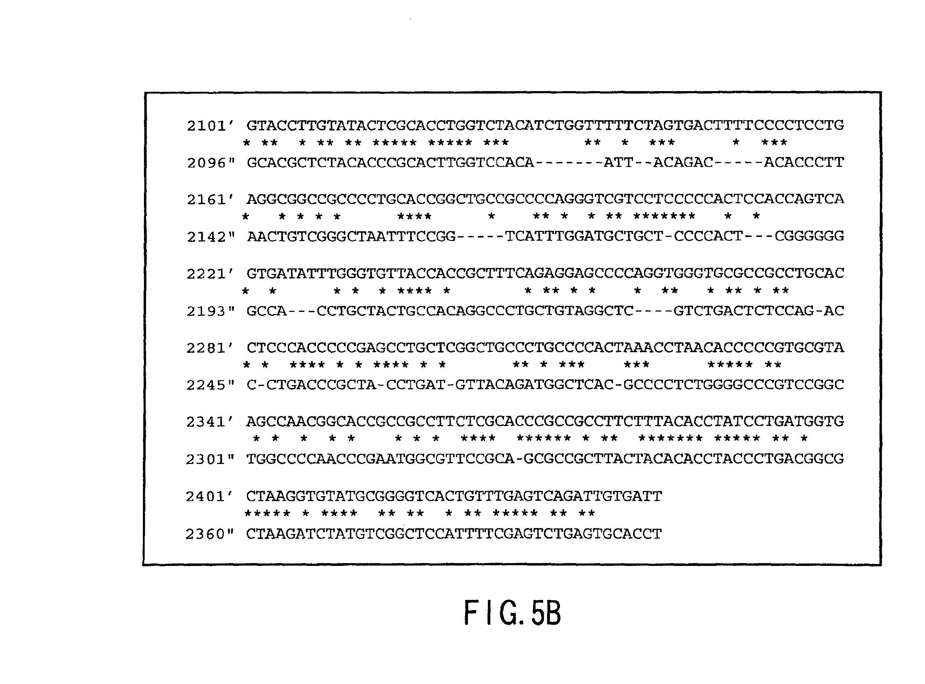

The sequence of the approximately 2500 nucleotides at the 5' end, in particular, in the ORF1 region exhibits, regarding the portion of the nucleotide sequence of JRA1 strain shown at the SEQ No. 7 of the sequence list described below, degree of homology (of sequence) of only 70% or less, as compared with the "Mexico" strain (SEQ ID NO-10 of USP 5,789,559, NCBI accession No. M74506, shown at the SEQ No. 49) which still shows the highest homology to JRA1 among the nucleotide sequence disclosed in the patents owned by Reyes. The nucleotide sequences of JRA1 strain and the "Mexico" strain are shown in FIGS. 3A, 3B, 4A, 4B, 5A and 5B in a manner that the former is compared with the latter. In FIGS. 3A, 3B, 4A, 4B, 5A and 5B, the sequence of HEV-JRA1 strain and a portion of the sequence of the "Mexico" strain are shown in a juxtaposed manner so that the former is shown as the upper sequence and the latter is shown as the lower sequence. The homology between the two sequences is 69.9% per 2432 nucleotides.

Accordingly, as such a specific novel sequence as described above of JRA1 strain has been revealed, JRA1 strain, which has never been subjected to detection and thus never been properly detected, can now be detected in an easy and accurate manner. Further, it is expected that the strains which are genetically close to JRA1 strain, which have hardly been detected by the conventional method, can also be detected by the present invention. Description will now be given of the embodiments of the present invention regarding the determination of the novel genome sequence of the novel hepatitis E virus strain described above.

### (2) Polynucleotide

In one embodiment of the present invention, a polynucleotide which is specific to JRA1 strain and has a novel sequence is provided. The polynucleotide of the present embodiment may be, for example, a polynucleotide shown as a nucleotide sequence of the SEQ No. 1 containing 5138 nucleotides. Alternatively, the polynucleotide may be a polynucleotide fragment, as a nucleotide sequence of any of the regions of the SEQ No. 1 nucleotide sequence. Specifically, the polynucleotide may be a polynucleotide shown as the nucleotide sequence of SEQ No. 7 containing 2442 nucleotides, or, a complementary strand of any one of the nucleotide sequences described above. Or, the polynucleotide of the present embodiment may be a polynucleotide fragment as a nucleotide sequence which constitutes a portion of any one of the aforementioned polynucleotides and the complementary strands thereof.

One or a few nucleotides of each polynucleotide described above may be subjected to modification such as deletion, substitution or addition.

In another embodiment of the present invention, a primer and a probe each constituted of any one of the nucleotide sequences of the SEQ Nos. 1 to 7 or the complementary strand thereof or a polynucleotide fragment having a fragment of any one of the nucleotide sequences of the SEQ Nos. 1 to 7 and the complementary strand thereof, are provided. The primer and probe may be used as a primer for amplification for detecting HEV in the detection object (for example, as a primer for PCR amplification or the like), or as a probe used in a "DNA chip". In this case, the number of nucleotides contained in the polynucleotide of the present embodiment is preferably in a range of 10 to 30 (inclusive of both 10 and 30). If the length of the polynucleotide fragment is too long, it is difficult to recognize the difference between the fragments, as the difference could be one nucleotide. If the length of the polynucleotide is too short, it is difficult to determine the nucleotide sequence of polynucleotide contained in the sample.

Examples of the primer and the probe of the present embodiment as described above include those for comprehensively detecting HEV and for selectively detecting each genotype of HEV.

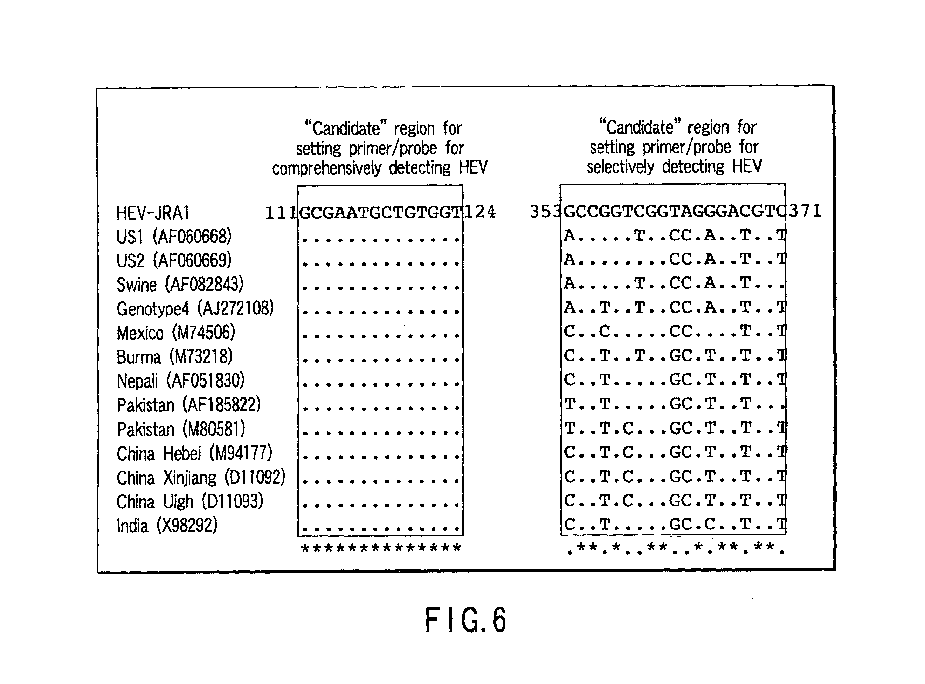

FIG. 6 shows a portion of the nucleotide sequence information of JRA1 strain and the nucleotide sequence information of known strains at the corresponding site, in a juxtaposed manner, so that the former is compared with the latter. By comparing the nucleotide sequence of JRA1 strain with the nucleotide sequence of known strains at the corresponding site, as shown in the example of FIG. 6, it is possible to clearly identify a highly-conserved region useful for setting a primer/probe for comprehensively detecting HEV and a highly-mutated region for selectively detecting each genotype of HEV.

The term "highly-conserved region" used in the present invention indicates a region which exhibits high homology, e.g., 90% or more, preferably 95% or more, and most preferably 100% of homology between the nucleotide sequence of HEV-JRA1 strain and the corresponding nucleotide sequence of a known strain. The term "highly-mutated region" used in the present specification indicates a region which is a nucleotide sequence specific to HEV-JRA1 strain and exhibits relatively low homology, e.g., 80% or less, preferably 75% or less, and most preferably 70% or less of homology between the nucleotide sequence of HEV-JRA1 strain and the corresponding nucleotide sequence of a known strain.

FIG. 6 shows the 111^{th} nucleotide to the 124^{th} nucleotide of the SEQ No. 1 of HEV-JRA1 strain as an example of a "candidate" region for setting a primer and a probe for comprehensively detecting HEV, and the region corresponding thereto of known strains. The polynucleotide shown as the nucleotide sequences of the SEQ Nos. 2 to 5 or the complementary strand of any one of the nucleotide sequences of the SEQ Nos. 2 to 5, for example, can be used as a primer and a probe for comprehensively detecting HEV. On the other hand, the region of the SEQ No. 1 which can be used as a primer and a probe for comprehensively detecting HEV is not limited thereto.

Similarly, FIG. 6 shows the 353^{rd} nucleotide to the 371^{st} nucleotide of the SEQ No. 1 of JRA1 strain as an example of a "candidate" region for setting a primer and a probe for selectively detecting HEV, and the region corresponding thereto of known strains. The polynucleotide shown as the nucleotide sequences of the SEQ Nos. 1 to 7 or the complementary strand of any one of the nucleotide sequences of the SEQ Nos. 1 and 7 (specifically, the polynucleotide including the nucleotide sequence of the SEQ No. 6 or the complementary strand of the nucleotide sequence of the SEQ No. 6), for example, can be used as a primer and a probe for selectively detecting HEV. On the other hand, the region of the SEQ Nos. 1 and 7 which can be used as a primer and a probe for selectively detecting HEV is not limited thereto.

In a further embodiment of the present invention, a polynucleotide including, as a portion thereof, the polynucleotide of the SEQ Nos. 1 to 7 or a fragment of the polynucleotide of the SEQ Nos. 1 to 7 is provided. The polynucleotide of the present embodiment may be a polynucleotide including a specific polynucleotide, which specific polynucleotide is obtained as a result of bonding of at least one type of a polynucleotide selected from the group consisting of: genes of a promoter, an enhancer, an upstream activation sequence, a silencer, an upstream repression sequence, an attenuator, poly(A) tail, nucleus transition signal, ISRE, a drug resistance factor, and a signal-peptide gene; gene of a membrane-penetration region; and gene of a marker protein including luciferin, green fluorescent protein, phycocyanin and horseradish peroxidase. Alternatively, the polynucleotide of the present embodiment may be a polynucleotide including any other suitable nucleotide sequence.

### (3) Polypeptide

In another embodiment of the present invention, a polypeptide as the amino acid sequence disclosed at the SEQ No. 8 is provided. The inventors of the present invention have demonstrated, by the clinical study, that the aforementioned polypeptide of the present embodiment is a polypeptide produced at an early stage in a HEV-JRA1 patient and that the antibody specific to the polypeptide is an antibody produced at an early stage in a HEV-JRA1 patient. Accordingly, the polypeptide and/or the antibody of the present embodiment can be used as a marker for HEV-JRA1 detection. The polypeptide and the antibody as described above can be obtained by the known methods. A polypeptide obtained as a result of modification such as deletion, substitution, addition of one or a few peptides, of a fragment of the aforementioned polypeptide, a polypeptide including the fragment, and an amino acid sequence disclosed at the SEQ No. 8, is also included within the scope of the present invention.

According to the present embodiment, if the polypeptide is a synthesized peptide, for example, and when the polypeptide is used as a polypeptide for antibody detection in which an antibody is detected as a result of bonding of the antibody to the polypeptide, the polypeptide of the present embodiment may include the polypeptide shown as the sequential 15 to 50 amino acid residue contained in the SEQ No. 8. If the polypeptide of the present embodiment is an expressed protein, the protein may include the polypeptide shown as the sequential 150 to 250 amino acid residue contained in the SEQ No. 8.

### 3. HEV Japan JSN-FH strain

### (1) HEV Japan JSN-FH strain

As described above, JSN-FH strain is a novel HEV strain isolated from a patient of a fulminant hepatitis case. The method of medical treatment is completely different between fulminant hepatitis and ordinary acute hepatitis. Accordingly, if the disease is diagnosed as fulminant hepatitis at an early stage, in other words, if the diagnosis as fulminant hepatitis can be made soon after the infection, the possibility of survival increases. However, the amount of HEV virus expression in a fulminant hepatitis patient is extremely small. Therefore, there were previously scant reports that HEV was successfully recovered from a fulminant hepatitis patient. Needless to say, determining the sequence over the whole length of HEV has been regarded virtually impossible. However, the inventors of the present invention have succeeded in molecular cloning of HEV from a fulminant hepatitis patient and also in determining the approximate whole length (or the approximate semi-whole length) of the genome of the HEV.

The genome of HEV recovered from a fulminant hepatitis patient had a unique sequence, as a whole. In one of the clones obtained by sub-cloning, a new point mutation was found. The mutation produces a premature stop codon in an open reading frame (ORF2) coding nucleocapsid protein and shortens the length of the translated product, from 660 amino acids which would be the length if ORF2 of the normal hepatitis E virus strain were to effect coding, to 211 amino acids. In short, the 212^{th} codon of ORF2 was replaced with any one of the stop codons TAA, TAG and TGA.

For hepatitis B, it has been found that, when a patient has been infected with a variant (strain) in which the translated product coded by the pre-core/core region cannot reach the full size, the patient is likely to develop fulminant hepatitis.

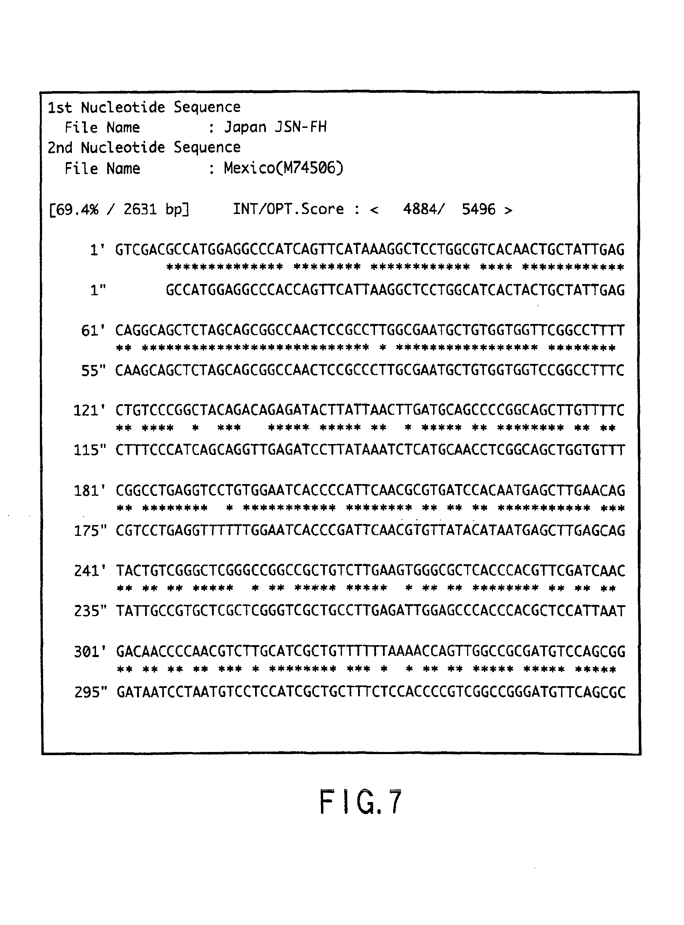

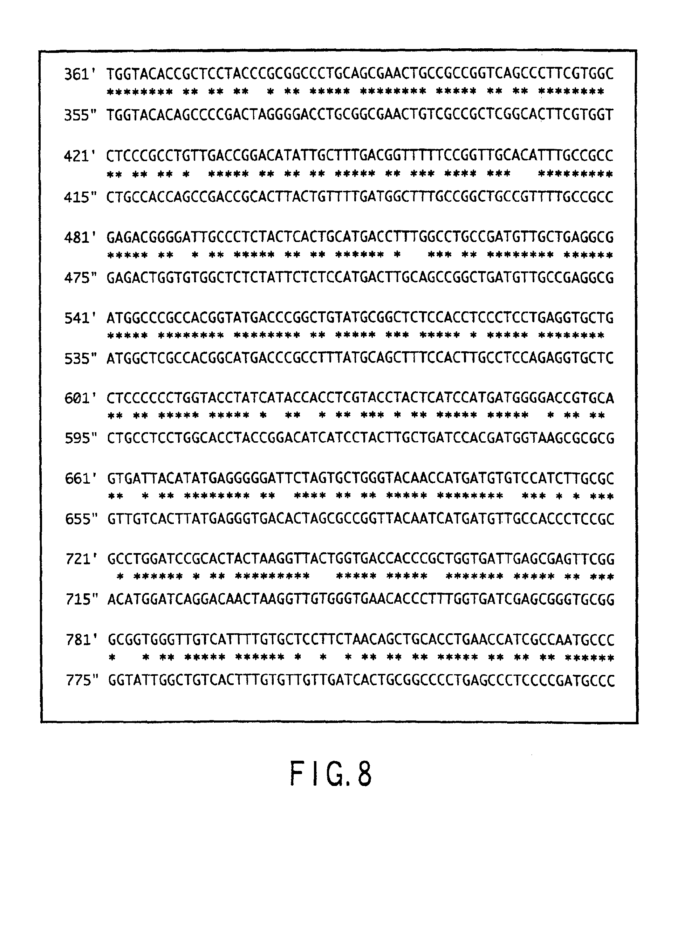

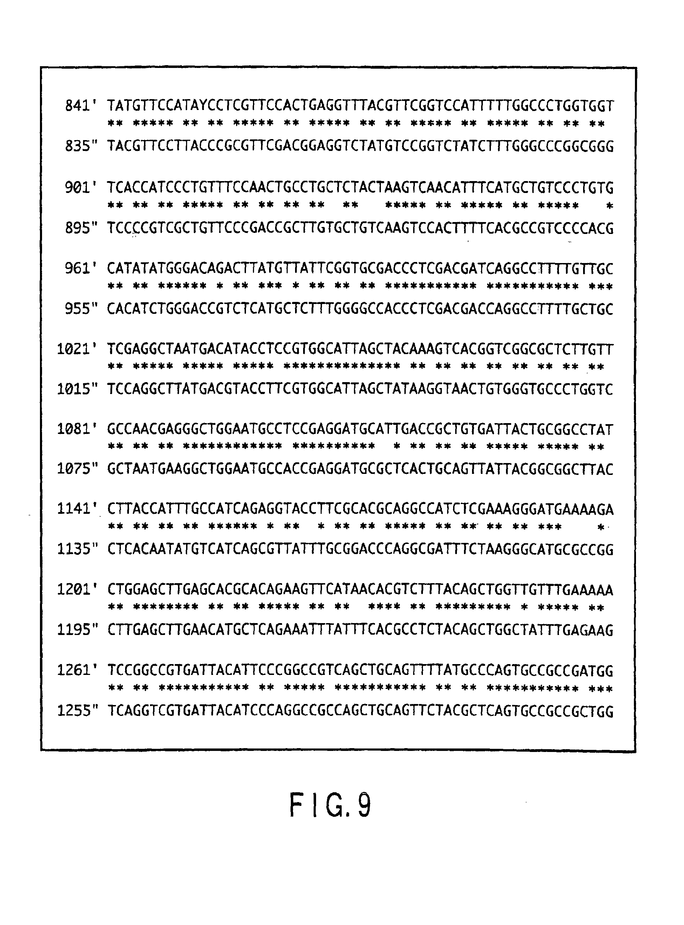

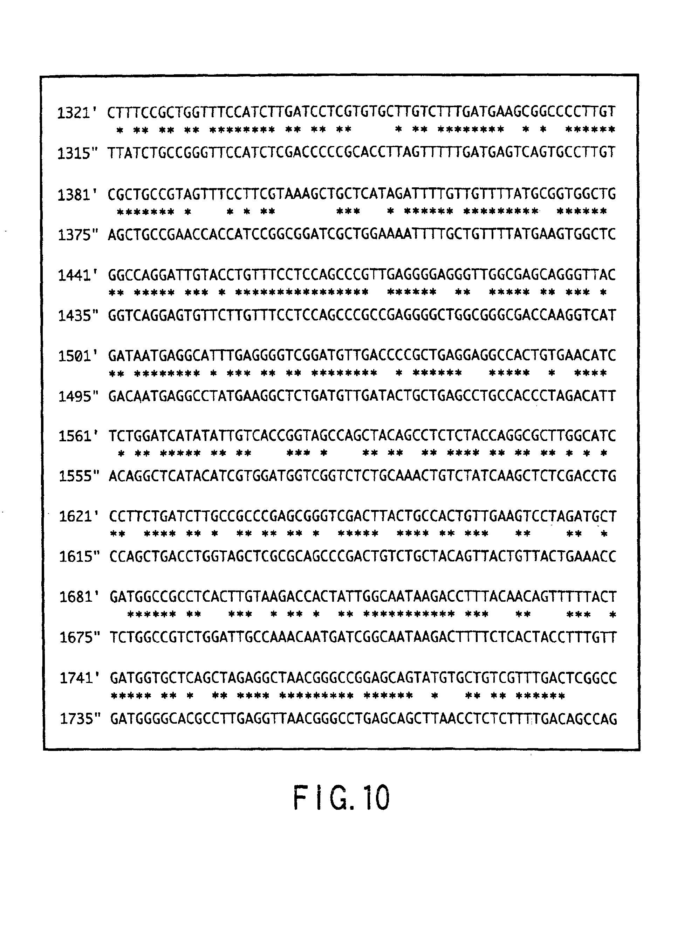

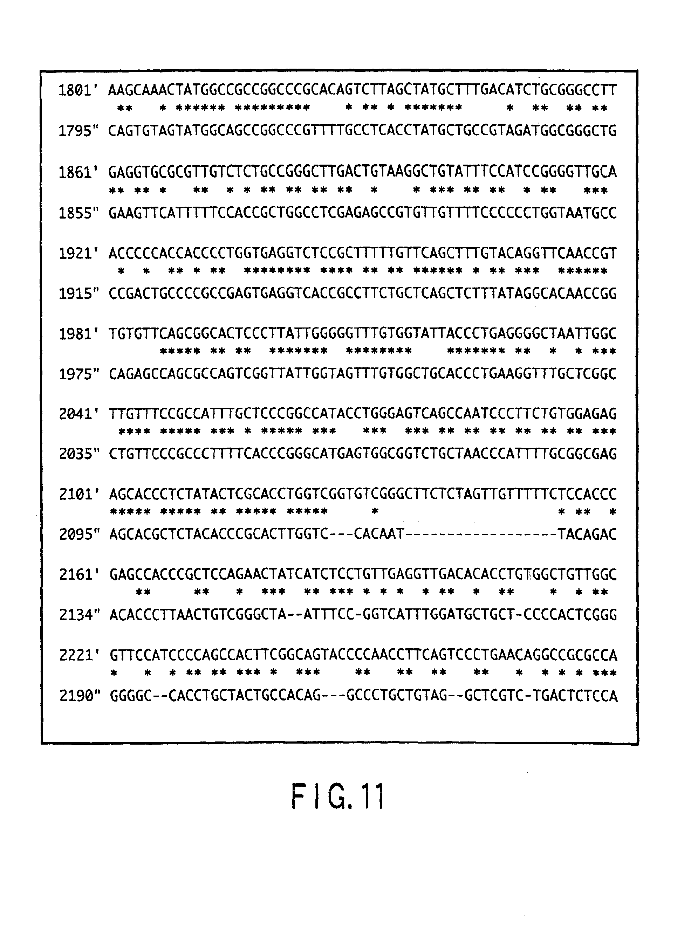

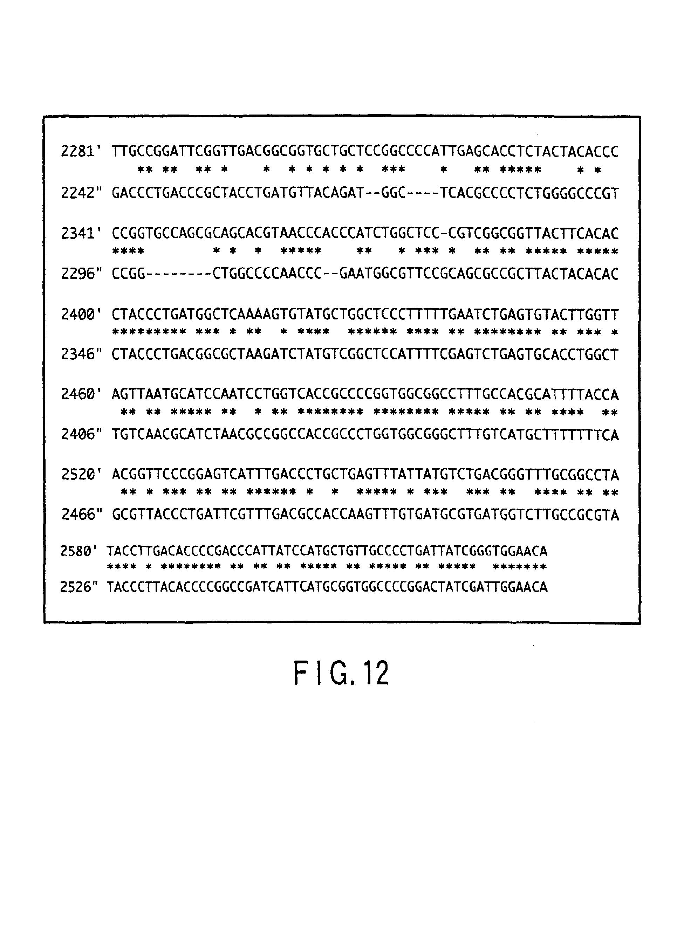

The nucleotide sequence of JSN-FH strain and the nucleotide sequence of "Mexico" strain (M74506) are shown in FIGS. 7 to 12 in a manner that the former is compared with the latter. The nucleotide sequence of HEV JSN-FH strain and the nucleotide sequence of "Mexico" strain (M74506) are shown in a juxtaposed manner, such that the nucleotide sequence of JSN-FH strain is located upper and the corresponding nucleotide sequence of "Mexico" strain (M74506) is located therebelow. In this case, the rate of homology is 69.4% per 2631 nucleotides.

Accordingly, as such a specific novel sequence as described above of JSN-FH strain has been revealed, JSN-FH strain, which has never been subjected to detection and thus never been properly detected, can now be detected in an easy and accurate manner. Further, it is expected that the strains which are genetically close to JSN-FH strain, which had not been detected by the conventional method, can also be detected by the present invention. If JSN-FH strain is detected in a patient, the result indicates that the patient is likely to develop fulminant hepatitis.

### (2) Polynucleotide

In a further embodiment of the present invention, a polynucleotide which is specific to JSN-FH strain and has a novel sequence is provided. The polynucleotide of the present embodiment may be, for example, a polynucleotide shown as a nucleotide sequence of SEQ No. 11 containing 7234 nucleotides. Alternatively, the polynucleotide may be a polynucleotide fragment, as a nucleotide sequence of any of the regions of the SEQ No. 11 nucleotide sequence. Specifically, the polynucleotide may be a polynucleotide shown as the nucleotide sequence of SEQ No. 22 containing 238 nucleotides. Or, the polynucleotide of the present embodiment may be a complementary strand of any one of the nucleotide sequences described above. Or, the polynucleotide of the present embodiment may be a polynucleotide fragment, as a nucleotide sequence which constitutes a portion of any one of the aforementioned polynucleotide and the complementary strand thereof.

Further, as the polynucleotide for determining whether or not the strain has a stop codon in ORF2, polynucleotides having the nucleotide sequences disclosed at the SEQ Nos. 9 and 10, and complementary strands of the polynucleotides having the nucleotide sequences disclosed at the SEQ Nos. 9 and 10, and a polynucleotide fragment having a fragment of any one of the aforementioned polynucleotides or the complementary strands thereof, may be provided. A primer and a probe may be provided using the polynucleotide described above. The nucleotide sequence disclosed at the SEQ Nos. 9 and 10 each represents a nucleotide sequence of a portion of ORF2 of JSN-FH strain. The nucleotide sequence disclosed at the SEQ No. 9 represents a nucleotide sequence which allows specifically detecting a strain not having a stop codon. The nucleotide sequence disclosed at the SEQ No. 10 represents a nucleotide sequence which allows specifically detecting a strain having a stop codon. A primer including a nucleotide sequence for amplifying a nucleotide portion including the aforementioned portion, also falls within the scope of the present invention. By detecting a nucleotide sequence which does not allow production of the full-size product of translation in ORF2 of the nucleic acid contained in the sample collected from the object, and detecting, on the basis of the aforementioned detection, that the object has been infected with a virus strain having such a stop codon in ORF2, it is possible to diagnose, at an early stage of infection, that the object or the patient has been infected with fulminant hepatitis virus.

One or a few nucleotides of each polynucleotide described above may be modified by, for example, deletion, substitution or addition.

In another embodiment of the present invention, a primer and a probe each constituted of any one of the nucleotide sequences disclosed at the SEQ Nos. 11 and 22 or the complementary strand thereof or a polynucleotide fragment having a fragment of any one of the nucleotide sequences of the SEQ Nos. 11 and 22 and the complementary strands thereof, are provided. The primer and probe may be used as a primer for various amplification for detecting HEV in the detection object (for example, as a primer for PCR amplification or the like), or as a probe used in a "DNA chip". In this case, the number of nucleotides contained in the polynucleotide of the present embodiment is preferably in a range of 10 to 30, inclusive. As previously mentioned, if the length of the polynucleotide fragment is too long, it will be difficult to recognize the difference between the fragments, and the difference could be one nucleotide. If the length of the polynucleotide is too short, it will generally be difficult to determine the nucleotide sequence of polynucleotide contained in the sample.

According to the present embodiment, polynucleotides of the SEQ Nos. 11, 22, 9 and 10 or a polynucleotide including, as a portion thereof, a fragment of any one of the polynucleotides of the SEQ Nos. 11, 22, 9 and 10 is provided. The polynucleotide of the present embodiment may be a polynucleotide including a specific polynucleotide, and this polynucleotide is obtained as a result of bonding of at least one type of polynucleotide selected from the group consisting of: genes of a promoter, an enhancer, an upstream activation sequence, a silencer, an upstream repression sequence, an attenuator, poly(A) tail, nucleus transition signal, ISRE, a drug resistance factor, and a signal-peptide gene; gene of a membrane-penetration region; and gene of a marker protein including luciferin, green fluorescent protein, phycocyanin and/or horseradish peroxidase. Alternatively, the polynucleotide of the present embodiment may be a polynucleotide including any other suitable nucleotide sequence.

### (3) Polypeptide

Further, in yet another embodiment of the present invention, polypeptides as the amino acid sequences disclosed at the SEQ Nos. 12 and 13 are provided. The amino acid sequence disclosed at the SEQ No. 12 is an amino acid sequence corresponding to the region of ORF1 of the genome sequence of JSN-FH strain. The amino acid sequence disclosed at the SEQ No. 13 is an amino acid sequence corresponding to the region of ORF2 of the genome sequence of JSN-FH strain.

The polypeptide and/or the antibody of the present embodiment can be used as a marker for JSN-FH detection. The polypeptide and the antibody as described above can be obtained by known methods. A polypeptide obtained as a result of modification such as deletion, substitution, addition of one or a few peptides, of a fragment of the aforementioned polypeptide, a polypeptide including the fragment, and an amino acid sequence disclosed at the SEQ No. 12 or No. 13, is also included within the scope of the present invention.

According to the present embodiment, if the polypeptide is a synthesized peptide, for example, and when the polypeptide is used as a polypeptide for antibody detection in which an antibody is detected as a result of bonding of the antibody to the polypeptide, the polypeptide of the present embodiment may include the polypeptide shown as the sequential 15 to 50 amino acid residue contained in the SEQ No. 12 or No. 13. If the polypeptide of the present embodiment is an expressed protein, the protein may include the polypeptide shown as the sequential 150 to 250 amino acid residue contained in the SEQ No. 12 or No. 13.

ORF1, which is represented by the SEQ No. 12, is a region coding various enzyme proteins necessary for replication of virion or other purposes. Accordingly, in addition to the data of the primary structure i.e., the SEQ No. 12, the data of the secondary structure and/or the tertiary structure of the protein(s) may be further obtained by known methods, such as X-ray diffraction, so that drug design can be carried out on the basis of the obtained data. Such drug design itself and a medicine obtained from the drug design are also included within the scope of the present invention.

An antibody specific to the shortened nucleocapsid protein, derived from ORF2 represented by the SEQ No. 13, may be produced by the known methods. By utilizing an antigen-antibody reaction in which the antibody as described above is used, HEV infection can be easily diagnosed, using a sample collected from the object.

An antibody for recognizing the antibody specific to the nucleocapside protein may be produced by a known method. By utilizing such an antibody, HEV infection can be easily diagnosed, using a sample collected from the object.

### 4. Comparison of the novel HEV strain with the conventional HEV strain

### (1) Comparison of the novel HEV strain with the conventional HEV strain

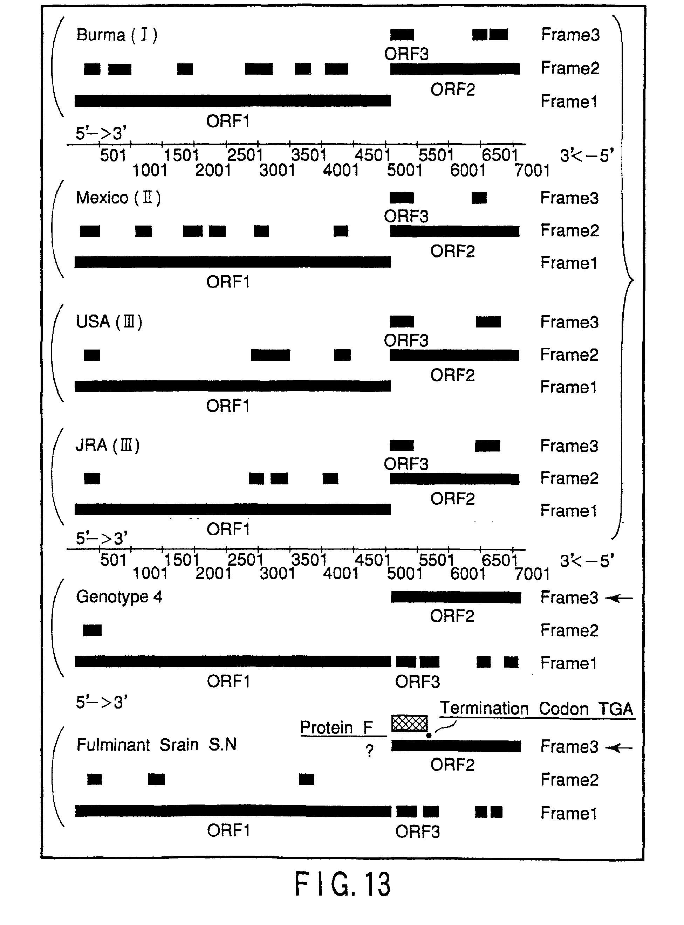

FIG. 13 shows the results of comparison in which HEV Burma B1 strain (M73218), HEV Mexico strain (M74506), HEV USA US-1 strain (AF060669), HEV Japan JRA1 (AP003430), Genotype 4 and HEV Japan JSN-FH are compared with each other, with regards to the location of ORF in each of the genome sequence thereof. The number in parenthesis added after the name of each strain is the NCBI accession number. Note that, in the present invention, "HEV" of each strain name may omitted (for example, HEV USA US-1 strain may read "USA US-1 strain").

The Roman numerals shown in parenthesis, next to each strain name, represent the genotype to which each strain belongs. As shown in FIG. 13, a termination codon TGA is present in OFR2 of JSN-FH strain.

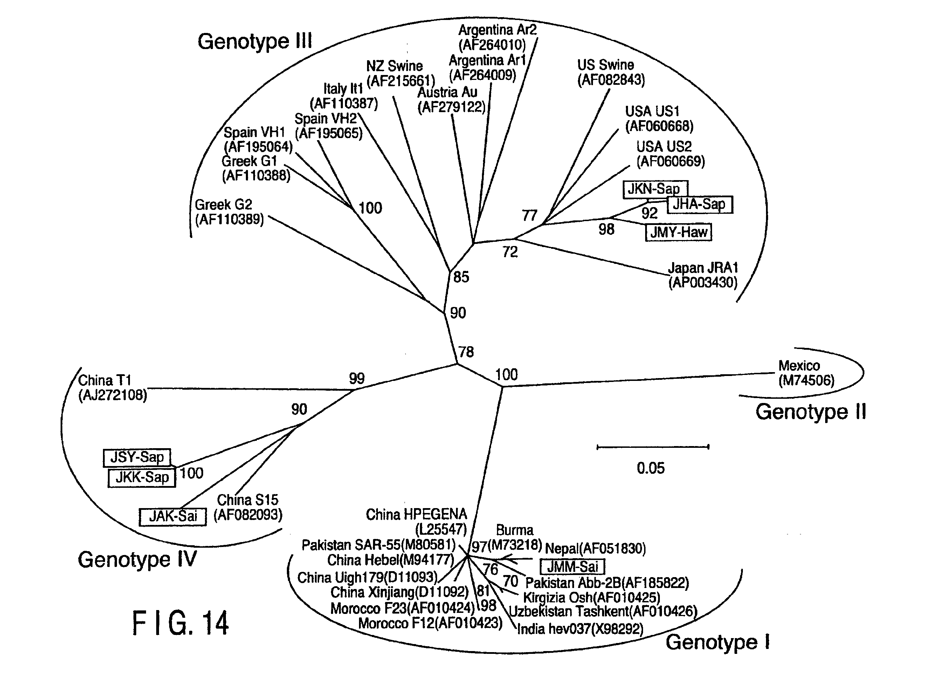

FIG. 14 shows a phylogenetic tree produced (according to the neighbor-joining method) on the basis of the 326nt region of ORF1, which represents genealogy of the strains of 7 types endemic to Japan, including the novel strains of 5 types of the present invention, and the HEV strains found in the countries other than Japan. As shown in FIG. 14, JMM-Sai was classified into type I. JKN-Sap, JHA-Sap and JMY-Haw were each classified into type III. JSY-Sap, JKK-Sap and JAK-Sai were each classified into type IV.

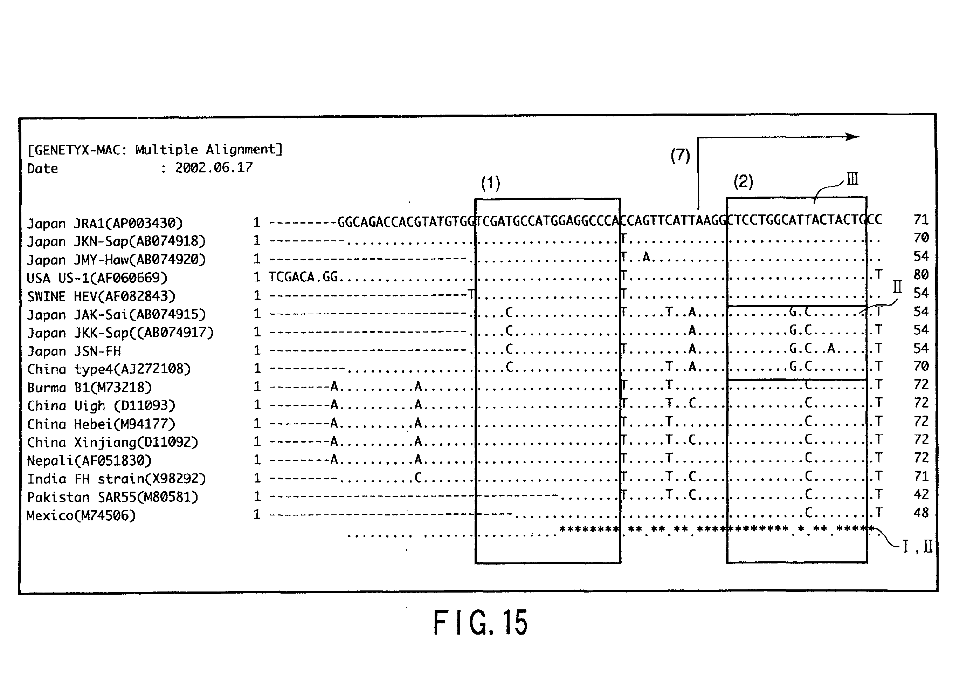

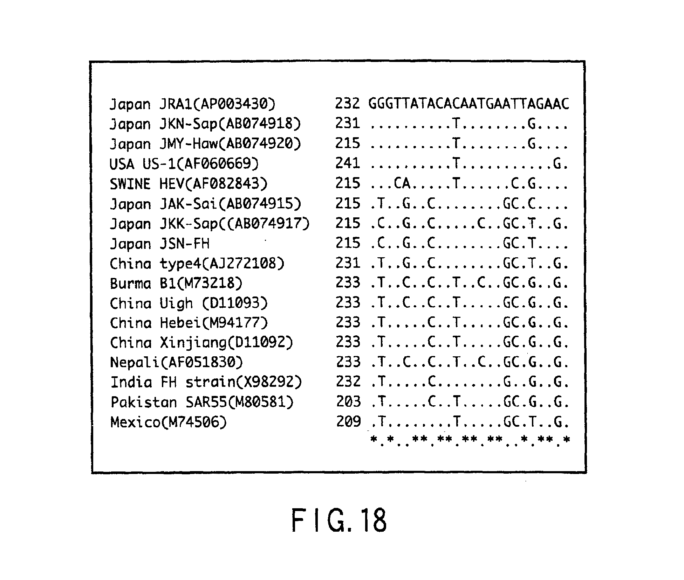

In FIGS. 15 to 18, the information of the nucleotide sequence of the ORF1 region of the following examples is shown, in a juxtaposed manner, for comparison. The examples include: the strains endemic to Japan of 5 types, as one embodiment of the present invention, i.e., {Japan JRA1 strain (SEQ No. 15), JKN-Sap strain (SEQ No. 16), JMY-Haw strain (SEQ No. 17), JKK-Sap strain (SEQ No. 21) and JAK-Sai strain (SEQ No. 20)}; the strain derived from a fulminant hepatitis patient (JSN-FH strain, SEQ No. 22); and the known foreign strains i.e., {USA US-1 (AF060669, SEQ No. 18), SWINE HEV (AF082843, SEQ No. 19), China type4 (AJ272108, SEQ No. 23), Burma B1 (M73218, SEQ No. 24), China Uigh (D11093, SEQ No. 25), China Hebei (M94177, SEQ No. 26), China Xinjiang (D11092, SEQ No. 27), Nepali (AF051830, SEQ No. 28), India FH strain (X98292, SEQ No. 29), Pakistan SAR55 (M80581, SEQ No. 30), and Mexico (M74506, SEQ No. 31)}.

In FIGS. 15 to 18, the strain names, the NCBI accession No. shown in parenthesis next to the strain names, and the information of nucleotide sequence next to the NCBI accession No. are shown in the aforementioned order. The numbers shown at both ends of the sequence each represent the number of nucleotide, counted from the transcript-initiation site whose number is "1". JRA 1 strain, shown as the uppermost sequence in FIGS. 15-18, is regarded as the reference. When the type of the nucleotide at a specific position of another strain coincides with the type of the nucleotide of JRA1 strain at the corresponding position, the nucleotide of the former is indicated by the symbol ".". When the type of the nucleotide at a specific position of another strain differs from the type of the nucleotide of JRA1 strain at the corresponding position, the type of the nucleotide of the former is identified as it is. At the lowermost sequence in FIGS. 15 to 18, the degree of conservation of sequence at the plurality of nucleotide sequences shown in the juxtaposed manner is indicated. Specifically, the symbol "*" at the lowermost sequence indicates that the type of the nucleotide at the specific position is the same throughout the plurality of strains. On the other hand, the symbol "." at the lowermost sequence indicates that type of the nucleotide at the specific position of the sequence is different between JRA1 strain and non-JRA1 strains, in at least one strain of the latter.

All of the novel strains and the nucleotide sequences thereof described above are included within the scope of the present invention.

### (2) Polynucleotide

According to a further embodiment of the present invention, polynucleotides which are specific to the strains JKN-Sap, JMY-Haw, JKK-Sap and JAK-Sai, respectively, and each have a novel sequence, are provided. The nucleotide sequence of the present embodiment may be, for example, a polynucleotide shown as a nucleotide sequence of any of the SEQ No. 44, the SEQ No. 45, the SEQ No. 46 and the SEQ No. 47, which nucleotide sequences represent the genome sequence of JKN-Sap, JMY-Haw, JKK-Sap and JAK-Sai, respectively. Alternatively, the polynucleotide may be the complementary strand of each of the nucleotide sequences described above. Yet alternatively, the polynucleotide may be a polynucleotide fragment, which constitutes a portion of any one of the aforementioned polynucleotides and the polynucleotides as the complementary strands thereof.

The polynucleotide of the present embodiment may be a polynucleotide fragment shown as the nucleotide sequence of any of the regions of the polynucleotide. The polynucleotide of the present invention may be a polynucleotide shown as a nucleotide sequence of any of the SEQ No. 16, the SEQ No. 17, the SEQ No. 20 and the SEQ No. 21 coding the ORF1 region. Or, the polynucleotide of the present embodiment may be a complementary strand of any one of the nucleotide sequences described above. Or, the polynucleotide of the present embodiment may be a polynucleotide fragment, which constitutes a portion of any one of the aforementioned polynucleotides and the complementary strands thereof.

Further, a primer including a nucleotide sequence for amplifying the nucleotide portion, including the nucleotide sequences of the SEQ No. 16, the SEQ No. 17, the SEQ No. 20 and the SEQ No. 21, is included within the scope of the present invention.

One or a few peptides of each polynucleotide described above may be subjected to modification such as deletion, substitution and addition.

In a further embodiment of the present invention, a primer and a probe each constituted of any one of the nucleotide sequences of the SEQ No. 44, the SEQ No. 45, the SEQ No. 46 and the SEQ No. 47 or the complementary strands thereof or a polynucleotide fragment having a fragment of any one of the nucleotide sequences of the SEQ Nos. 44 to 47 and the complementary strands thereof, are provided. The primer and probe may be used as a primer for various amplification for detecting HEV in the detection object (for example, as a primer for PCR amplification or the like), or as a probe used in a "DNA chip". As previously mentioned number of nucleotide contained in the polynucleotide of the present embodiment is preferably in a range of 10 to 30, inclusive. If the length of the polynucleotide fragment is too long, it will be difficult to recognize the difference between the fragments, and the difference could be one nucleotide. If the length of the polynucleotide is too short, it will be difficult to determine the nucleotide sequence of polynucleotide contained in the sample.

Further, another embodiment of the present invention, a polynucleotide including, as a portion thereof, the polynucleotide of any of the SEQ No. 44, the SEQ No. 45, the SEQ No. 46 and the SEQ No. 47, as well as the SEQ No. 16, the SEQ No. 17, the SEQ No. 20 and the SEQ No. 21 or a polynucleotide having, as a portion thereof, a fragment of these polynucleotides is provided. The polynucleotide of the present embodiment may be a polynucleotide including a specific polynucleotide, obtained as a result of bonding of at least one type of polynucleotide selected from the group consisting of: genes of a promoter, an enhancer, an upstream activation sequence, a silencer, an upstream repression sequence, an attenuator, poly(A) tail, nucleus transition signal, ISRE, a drug resistance factor, and a signal-peptide gene; gene of a membrane-penetration region; and gene of a marker protein including luciferin, green fluorescent protein, phycocyanin and horse radish peroxidase. Alternatively, the polynucleotide of the present embodiment may include any other suitable nucleotide sequence.

### (3) Polypeptide

In another embodiment of the present invention, polypeptides shown as the amino acid sequences coded by the polynucleotide sequence disclosed at the SEQ No. 44, the SEQ No. 45, the SEQ No. 46 and the SEQ No. 47, as well as the SEQ No. 16, the SEQ No. 17, the SEQ No. 20 and the SEQ No. 21, which are specific to the strains of JKN-Sap, JMY-Haw, JKK-Sap and JAK-Sai, respectively, are provided. Further, the present embodiment provides polypeptides and the fragments thereof, and the polypeptides are shown by the amino acid sequences of the SEQ No. 50, the SEQ No. 52, the SEQ No. 54 and the SEQ No. 56 representing the amino acid sequences of the ORF1 region and the SEQ No. 51, the SEQ No. 53, the SEQ No. 55 and the SEQ No. 57 representing the amino acid sequences of the ORF2 region, specific to the strains of JKN-Sap, JMY-Haw, JKK-Sap and JAK-Sai, respectively.

The polypeptide derived from the amino acid sequence coded by the ORF1 region can be utilized in carrying out drug design.

The polypeptide derived from the amino acid sequence coded by the ORF2 region can be used for producing an antibody for detecting the respective strains. Alternatively, the polypeptide derived from the amino acid sequence coded by the ORF2 region can, by itself, be used as an antibody for detection of diagnostic purpose. Or, vaccine may be produced by utilizing this polypeptide.

According to the present embodiment, if the polypeptide is a synthesized peptide, for example, and when the polypeptide is used as a polypeptide for antibody detection in which an antibody is detected as a result of bonding of the antibody to the polypeptide, the polypeptide of the present embodiment may include the amino acid sequence shown by the ORF2 region of the nucleotide sequence derived from the respective novel strains, e.g., the polypeptide shown as the sequential 15 to 50 amino acid residues contained in the SEQ No. 51, the SEQ No. 53, No. 55 or the SEQ No. 57. If the polypeptide of the present embodiment is an expressed protein, the protein may include the polypeptide shown as the sequential 150 to 250 amino acid residue contained in the SEQ No. 51, the SEQ No. 53, No. 55 or the SEQ No. 57.

The polypeptide and/or the antibody described above can also be used as a marker for detecting HEV JKN-Sap, JMY-Haw, JKK-Sap and JAK-Sai, respectively. The polypeptide and the antibody as described above can be obtained by the known methods. A polypeptide obtained as a result of modification such as deletion, substitution, addition of one or a few peptides, of a fragment of the aforementioned polypeptide, a polypeptide including the fragment, and an amino acid sequence disclosed at the SEQ Nos. 50 to 57, is also included within the scope of the present invention.

According to the present embodiment, if the polypeptide is a synthesized peptide, for example, and when the polypeptide is used as a polypeptide for antibody detection in which an antibody is detected as a result of bonding of the antibody to the polypeptide, the present polypeptide may include the polypeptide shown as the sequential 15 to 50 amino acid residues contained in the SEQ No. 8. If the polypeptide of the present embodiment is an expressed protein, the protein may include the polypeptide shown as the sequential 150 to 250 amino acid residues contained in the SEQ No. 8.

### 5. Primer and probe for comprehensively detecting HEV

As a result of the aforementioned discovery of the novel HEV strains and the determination of the nucleotide sequence thereof, a primer which can amplify the HEV genome of a variety of types (including unknown types) all at a time, that is, a universal primer (i.e., a primer for comprehensive HEV detection) can be provided. Further, a probe which can detect HEV of a variety of types (including unknown types) all at a time (i.e., a probe for comprehensive HEV detection) can be provided. Such a universal primer will be described hereinafter.

In FIGS. 15 to 18, the nucleotide sequences of ORF1 of the representative HEV strains, each of which belongs to Group I, II, III or IV, are listed. In the tables of FIGS. 15 to 18, the symbol shown inside the parenthesis next to the strain name is the NCBI accession No. The numbers shown at both ends of the sequence each represent the number of the nucleotide, counted from the transcript-initiation site whose number is "1".

The term "universal primer" or "universal probe" represents a polynucleotide fragment containing nucleic acid including a nucleotide sequence which allows comprehensive detection of HEV including unknown strains. For example, the nucleotide sequence of such a universal primer can be selected from the sequences shown in FIGS. 15 to 18. The sequence is, for example, preferably a highly conserved region.

The term "highly-conserved region" used in the present invention indicates a region which exhibits high homology, e.g., 90% or more, preferably 95% or more, and most preferably 100% of homology between the nucleotide sequence of HEV-JRA1 strain and the corresponding nucleotide sequence of a known strain.

FIGS. 15 to 18 show an example in which a portion of the information of nucleotide sequence of JSN-FH strain is compared with the information of nucleotide sequence of known strains at the corresponding site. By comparing the nucleotide sequence of JSN-FH strain with the nucleotide sequence of the known strains in such a manner, it is possible to clearly identify a highly-conserved region useful for setting a primer/probe for comprehensively detecting HEV and a highly-mutated region for selectively detecting each genotype of HEV.

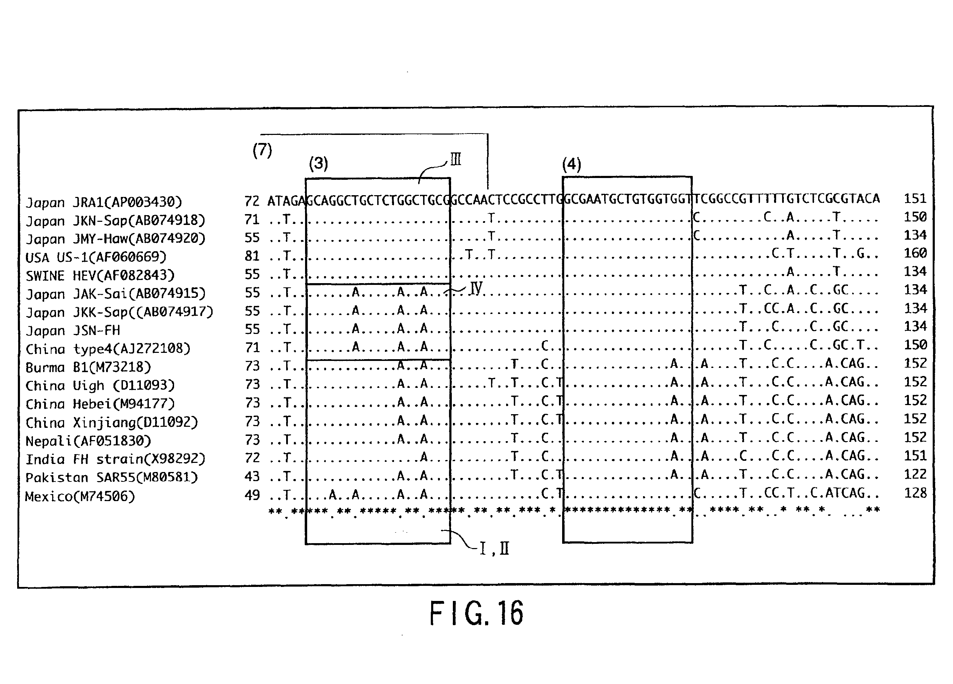

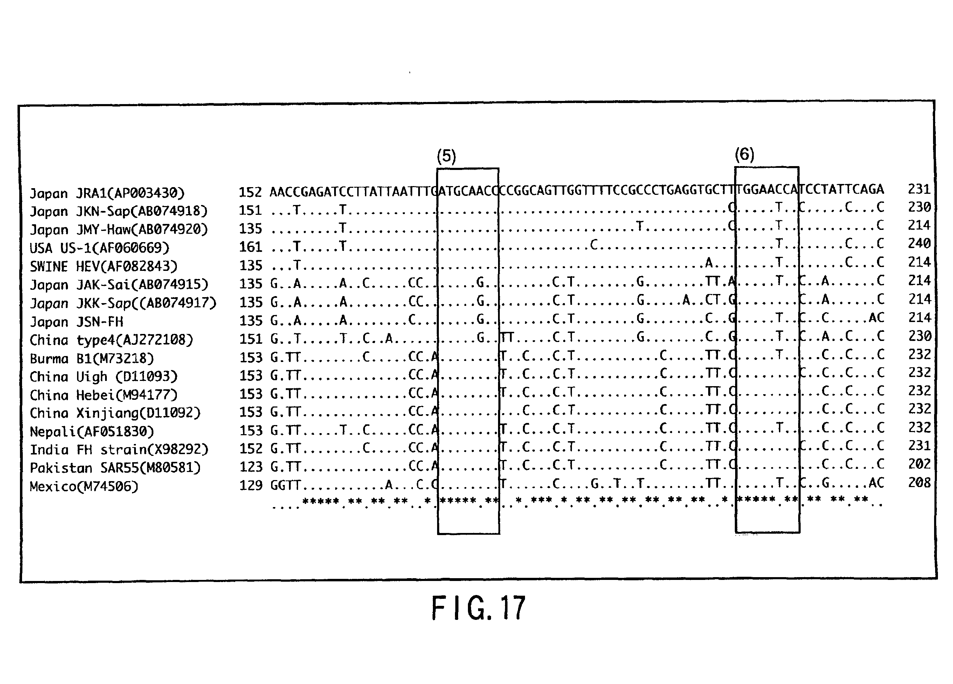

FIGS. 15 to 18 show an example of a "candidate" region for setting a primer and a probe for comprehensively detecting HEV. Preferable examples of the highly conserved region include: the region indicated by "(1)" in FIG. 15, i.e., the range from the 19^{th} (nucleotide) to the 37^{th} (nucleotide) of the SEQ No. 15 of HEV-JRA1 strain and the corresponding region of the known strains; the region indicated by "(4)" in FIG. 16, i.e., the range from the 111^{th} to the 127^{th} of the SEQ No. 15 of HEV-JRA1 strain and the corresponding region of the known strains; the region indicated by "(5)" in FIG. 17, i.e., the range from the 174^{th} to the 181^{st} of the SEQ No. 15 of HEV-JRA1 strain and the corresponding region of the known strains; and the region indicated by "(6)" in FIG. 17, i.e., the range from the 213^{th} to the 220^{th} of the SEQ No. 15 of HEV-JRA1 strain and the corresponding region of the known strains.

Regarding the primer for comprehensively detecting HEV, use of a polynucleotide of preferably 6 to 100 nucleotides, more preferably 15 to 25 nucleotides having a sequence as included in the (1) region of FIG. 15, as a sense primer, is preferable. Use of a polynucleotide of preferably 6 to 100 nucleotides, more preferably 15 to 25 nucleotides having a sequence as included in the (4), (5) and/or (6) regions of FIG. 15, as a sense primer, together with the aforementioned sense primer, is more preferable.

The region indicated by "(7)" in FIGS. 15 and 16, i.e., the range from the 48^{th} to the 100^{th} of the SEQ No. 15 of HEV-JRA1 strain and the corresponding region of the known strains can also be used as the primer groups for comprehensively detecting HEV. In this case, the sequence extending over the entire length of the (7) region is not necessary and any suitable sequence of preferably 6 to 25 nucleotides, more preferably 15 to 22 nucleotides selected from the (7) region can be used. Preferable examples of the primer for comprehensively detecting HEV include: 5'-gcagaccacrtatgtgktcg-3' (SEQ No. 32); 5'-ccacrtatgtggtcgaygcc-3' (SEQ No. 33); 5'-acmarctgscgrggytgcat-3' (SEQ No. 34); 5'-cgytgratwggrtgrttcca-3' (SEQ No. 35); 5'-tgktcgaygccatggaggc-3' (SEQ No. 36); 5'-tgktcgaygccatggaggc-3' (SEQ No. 37); 5'-aygccatggaggcccaycag-3' (SEQ No. 38); 5'-ckracyaccacagcattcgc-3' (SEQ No. 39); and

5'-ggcckracyaccacagcatt-3' (SEQ No. 40).

In the present specification, "a" or "A" represents adenine, "c" or "C" represents cytosine, "g" or "G" represents guanine, "t" or "T" represents thymine, "r" or "R" represents G or A. "y" or "Y" represents T or U or U. "w" or "W" represents A or T or U.

It is more preferable that the aforementioned primer groups for comprehensively detecting HEV is used, for example, for two-staged analysis as follows. In one example, the SEQ No. 32 and the SEQ No. 33 described above are used as sense primers and the SEQ No. 34 and the SEQ No. 35 described above are used as antisense primers, for the first amplification. Then, the SEQ No. 36, the SEQ No. 37 and the SEQ No. 38 described above are used as sense primers and the SEQ No. 39 and SEQ No. 40 described above are used as antisense primers, for the second amplification. As a result, polynucleotide fragments derived from unknown HEV variant strains of a variety of types can be obtained. The products obtained as a result of amplification may further be analyzed by known methods such as electrophoresis. The polynucleotide fragment which has been analyzed may then be classified into genotypes.

When the aforementioned sequences are used as probes, a polynucleotide of preferably 6 to 100 nucleotides, more preferably 12 to 25 nucleotides having a sequence including the sequences contained in the (1) region of FIG. 15 is preferably used as the probe for comprehensively detecting HEV. A polynucleotide of preferably 6 to 100 nucleotides, more preferably 12 to 25 nucleotides having a sequence including the sequences contained in the (4), (5) and/or (6) regions of FIG. 15 is more preferably used as the probe for this purpose.

It should be noted that the primer for comprehensively detecting HEV of the present invention is not restricted to the above-described preferable examples thereof.

### 6. Nucleotide sequence for genotype identification

A primer and probe for comprehensively detecting HEV can be obtained by selecting the sequence of the highly conserved region as described above, and comprehensive virus detection is possible by using the obtained primer and probe. On the other hand, a primer and probe for selectively detecting HEV can be obtained by selecting the sequence of the highly mutated region. Selective detection and/or identification of virus can be possible by using such a highly mutated sequence.

The term "highly-mutated region" used in the present specification indicates a region which is a nucleotide sequence specific to HEV-JRA1 strain and exhibits relatively low homology, e.g., 80% or less, preferably 75% or less, and most preferably 70% or less of homology between the nucleotide sequence of HEV-JRA1 strain and the corresponding nucleotide sequence of known strains.

FIGS. 15, 16, 17 and 18 show an example of a "candidate" region for setting a primer and a probe for selectively detecting HEV. Preferable examples of the highly mutated region include: the region indicated by "(2)" in FIG. 15, i.e., the range from the 52^{nd} (nucleotide) to the 69^{th} (nucleotide) of the SEQ No. 15 of HEV-JRA1 strain and the corresponding region of the known strains; and the region indicated by "(3)" in FIG. 16, i.e., the range from the 77^{th} to the 95^{th} of the SEQ No. 15 of HEV-JRA1 strain and the corresponding region of the known strains.

The (2) region of FIG. 15 is a region where the nucleotide sequence changes according to the genotype thereof. The (2) region of FIG. 15 has been demarcated to three portions according to the genotypes. Specifically, the strain included in the portion having the symbol "III" attached thereto in FIG. 15 belongs to genotype III. The strain included in the portion having the symbol "IV" attached thereto in FIG. 15 belongs to genotype IV. The portion having the symbol "I, II" attached thereto in FIG. 15 includes genotypes I or II. The (3) region of FIG. 16 can be understood in a manner similar to the (2) region of FIG. 15. In short, the HEV to be analyzed can be classified into the respective genotypes, by utilizing the difference in type of nucleotide at a specific position of the sequence between the respective genotypes.

When the aforementioned sequences are used as probes, a polynucleotide of preferably 6 to 100 nucleotides, more preferably 12 to 25 nucleotides having a sequence including the sequences contained in the (2) region of FIG. 15 is preferably used as the probe for selectively detecting HEV. A polynucleotide of preferably 6 to 100 nucleotides, more preferably 12 to 25 nucleotides having a sequence including the sequence(s) contained in the (3) region of FIG. 16 is more preferably used as the probe for this purpose.

When the aforementioned sequences are used as primers, a polynucleotide of preferably 6 to 100 nucleotides, more preferably 12 to 25 nucleotides having a sequence including the sequences contained in the (2) region of FIG. 15 is preferably used as a primer for selectively detecting HEV. A polynucleotide of preferably 6 to 100 nucleotides, more preferably 12 to 25 nucleotides having a sequence including the sequence contained in the (3) region of FIG. 16 is more preferably used as the probe for this purpose. Or, a primer which is capable of amplifying polynucleotide including at least the (2) region of FIG. 15 and/or the (3) region of FIG. 16 may be selected.

### 5. Method of detecting HEV virus

In a further embodiment of the present invention, a method of detecting HEV virus in a sample is provided.

The term "sample" used in the present embodiment represents; a biosample including blood, serum, lymph, tissues, and excrement such as feces, urine or the like collected from a biont as the object; a untreated sample as an environmental sample e.g., water and soil collected from the environment including rivers, sewage and the like; and a sample obtained by subjecting the aforementioned biosample or the environment sample to any necessary preparatory treatment such as homogenation, extraction or the like. Such a preparatory treatment will be easily selected by one skilled in the art, in accordance with the biosample or the environmental sample used as the object.

According to the present embodiment, the method of detecting HEV virus can be carried out, by using the primer described above, according to the known amplification method such as the polymerase chain reaction (which reaction is generally called as "PCR" and thus will be referred to as "PCR" hereinafter). Typical examples of PCR includes reverse transcription PCR, reverse transcription nested PCR, or modification thereof such as reverse PCR, 5' RACE and 3' RACE.

The detection method as described above can be conducted, for example, as follows. First, a desired primer is mixed with a sample containing hepatitis E virus genome. A PCR reaction is carried out under an appropriate PCR condition in which, for example, the temperature is changed such that the initial 95°C lasted for 4 minutes is followed by 30 cycles of {95°C for 30 seconds, 55°C for 30 seconds, 72°C for 45 seconds} and the final 72°C for 7 minutes. Thereafter, a genome fragment derived from hepatitis E virus can be detected by analyzing the product by means of electrophoresis, DNA chip or the like. As a result, the hepatitis E virus can be detected. For example, in the case in which the product is analyzed by electrophoresis, a genome fragment derived from hepatitis E virus can be detected by determining presence/absence of a band derived from HEV genome therein.

In another embodiment of the present invention, the method of detecting HEV virus can be carried out, by using the probe described above, according to a detection method in which the known hybridization method is utilized. In this case, the probe can be labeled with a desired marker substance.

In short, virus detection in the comprehensive manner is possible by using the aforementioned primer and probe for comprehensively detecting HEV. Alternatively, virus detection in the selective manner is possible by using the aforementioned primer and probe for selectively detecting HEV.

For example, by designing a detection system in which a highly conserved region is utilized, the precision of diagnosis of HEV infection can be enhanced. On the other hand, a system for detecting genotype specificity in which system a highly mutated region is utilized can make significant contribution to determination of the infection route and epidemiological study. The system for detecting genotype specificity can also be used for a virus-check test with respect to blood to be used for blood transfusion and a virus-check test of a sample derived from the environment.

According to the present embodiment, virus detection as described above can be achieved not only at the aforementioned nucleic acid level but also at the level of amino acid. For example, the amino acid sequence shown as the SEQ No. 8 and the like can be used as a marker which indicates the presence of HEV, as is the case with the aforementioned polynucleotide. Such an amino acid sequence can be used, for example, for comprehensively diagnosing infection of the virus in an organism including human and for specifically diagnosing the genotype of the virus. Similarly, any of the aforementioned amino acid sequences can be used for the purpose described above.

The gene and polynucleotide derived from HEV-JRA1 according to the present invention described above is a novel substance. The method of detecting HEV by using the polynucleotide or polypeptide and antibody produced from the polynucleotide excels the prior art, in usefulness or advantage thereof in detecting virus genome, virus antibody and the like in a sample. The advantage of the present invention will be described in detail hereinafter by examples.

In order to improve the HEV detection method and the diagnosis technique of HEV infection, it is preferable that the nucleotide sequence information of the gene of such a novel strain as described above is reflected on the detection and diagnosis systems.

### 8. Chip for detecting nucleotide sequence

According to one embodiment of the present invention, a chip for detecting nucleotide sequence, which chip includes the aforementioned polynucleotide, is provided. Examples of the nucleotide sequence-detection chip of the present embodiment include DNA chip for fluorescent detection, DNA chip of electric current-detection type and the like. However, the nucleotide sequence-detection chip of the present embodiment is not restricted to these examples. The detection method is simplified and made effective, by detecting virus by employing a chip for detecting nucleotide sequence in which chip the aforementioned polynucleotide or a complementary strand thereof is arranged as a probe. The chip for detecting nucleotide sequence can be produced according to the following procedure.

### (a) Production of a chip for detecting nucleotide sequence, to be used fluorescent detection

A polynucleotide according to the present invention or a polynucleotide having a sequence as a portion of the polynucleotide or a polynucleotide having a sequence complementary to any one of the sequences of these polynucleotides, are fixed on a substrate. As the substrate, any substrate of the conventional type e.g., a glass substrate or a silicon substrate can be used. Regarding the fixing means, any suitable means known to one skilled in the art, including a means utilizing a spotter and a means utilizing the general semiconductor technique, can be used.

### (b) Production of a nucleotide sequence detection chip of electric current detection type

A polynucleotide according to the present invention or a polynucleotide having a sequence as a portion of the polynucleotide or a polynucleotide having a sequence complementary to any one of the sequences of these polynucleotides, are fixed on a substrate, e.g., an electrode substrate, by means of covalent bond, ionic bond, physical adsorption or chemical adsorption. Examples of the DNA chip of electric current detection type include a gene detection device disclosed by JP-B No. 2573443 (issued on October 24, 1996) and the like. However, the chip for detecting nucleotide sequence, of electric current detection type, of the present invention is not restricted to these examples. JP-B No. 2573443 is herein incorporated to the present specification by reference.

According to the present embodiment, detection of virus can be carried out easily and effectively, by detecting virus by using a probe and a chip for detecting gene sequence including the polynucleotides as described above.

### 9. Protein chip

In one embodiment of the present invention, a protein chip which allows easy implementation of the detection method described above is provided. In the protein chip, an antibody which recognizes the aforementioned polypeptide is arranged as a probe. By using this protein chip, HEV in a sample can be detected in a simple, easy and efficient manner. Examples of the protein chip include a protein chip for fluorescent detection (which is generally called as "fluorescent colorant-type protein chip"), a protein chip of electric current detection type (which is generally called as "electric potential-type protein chip") and the like. However, the protein chip of the present embodiment is not restricted to these examples. Examples of the production procedure of the protein chip of the present embodiment will be described below.

### (a) Production of a protein chip for fluorescent detection

A monoclonal antibody of the aforementioned polypeptide is produced in advance. The obtained monoclonal antibody is fixed on a substrate. As the substrate, any substrate of the conventional type e.g., a glass substrate or a silicon substrate can be used. The fixing means may be selected from any suitable means known to one skilled in the art, such as a means utilizing a spotter and a means utilizing the general semiconductor technique. A fluorescent substance, a radioactive isotope, a colorant or the like may be used for labeling for detection.

### (b) Production of a protein chip of electric current detection type

A monoclonal antibody of the aforementioned polypeptide is produced in advance. The obtained monoclonal antibody is fixed on a substrate of a type generally used for the conventional electric current detection-type DNA chip, whereby a protein chip of electric current detection type of the present embodiment is produced.

According to the present embodiment, detection of a virus can be carried out easily and efficiently, by detecting virus by using a protein chip including the aforementioned antibody.

Alternatively, the aforementioned polypeptide, in stead of the antibody, may be fixed on a substrate, for producing a chip, so that this chip is used for detecting an antibody specific to the polypeptide.

### 10. Diagnosis system

By using the novel HEV strain, the polynucleotide and the polypeptide as described above according to the present invention, it is possible to diagnose: whether or not the object has been infected with HEV; and/or, if the object has been infected with HEV, to which genotype the HEV belongs; and/or whether or not the object has been infected with fulminating HEV.

The diagnosing method described above includes: for example, collecting a sample from the object by a known method; optionally carrying out purification of the sample and/or amplification of the nucleic acid; and obtaining a nucleic acid sample by purification. Thereafter, the targeted diagnosis can be carried out by using the polypeptide of the present invention, through detecting presence/absence of amplification of the targeted sequence or detecting presence/absence of hybridization. Or, the targeted diagnosis can be done by employing the aforementioned polypeptide of the present invention as a polypeptide for detecting antibody and utilizing the antigen-antibody reaction.

### 11. Vaccine for preventive purpose

In one embodiment of the present invention, a vaccine for HEV is provided. The vaccine can be prepared by using at least one type of polypeptide selected from the immunogenic polypeptides derived from HEV containing nucleic acid coded by (i.e., derived from) the aforementioned nucleotide sequence of the novel HEV virus of the present invention. For example, polypeptide of the ORF2 region of any of the aforementioned novel strains may be used. Production of vaccine containing the immunogenic polypeptide can be carried out by using any of the suitable known methods.

### 12. Drug design

The genetic information derived from the aforementioned novel HEV is useful for development and improvement of antiviral agents. Such development and improvement of antiviral agents can be carried out, for example, by following process, by using the known method of drug designing generally called "post-genome drug designing".
(1) The tertiary structure of enzyme proteins of a plurality of types, coded by ORF1 of the HEV genome, is actually measured by X-ray diffraction and/or NMR analysis of the expressed proteins. Alternatively, the tertiary structure of the enzyme proteins may be obtained by simulation on a computer, only on the basis of the information of the amino acid sequence. The "candidate" domain which can be the target of the drug (which domain will be referred to as "receptor domain" hereinafter), of the enzyme protein, is searched on the information obtained by the measurement or the simulation.
(2) With regards to a drug whose inhibitory action against the enzymic activity of HEV ORF1 protein has been confirmed, a compound which has already been synthesized, although the inhibitory effect thereof against the enzymic activity of HEV ORF1 protein has not been confirmed yet, a compound which has not been synthesized yet but whose structural formula has been known, and an imaginary compound having a novel structure (each of which drug or compound will be referred to as "ligand" hereinafter), the tertiary structures thereof are inputted into a computer, respectively.
(3) By using the data inputted into a computer in the aforementioned process (2), whether or not the receptor domain ("candidate" region) determined in the aforementioned process (1) can be three-dimensionally bound to any of the ligands of the aforementioned process (2) is determined through three-dimensional docking analysis. An effective combination of a ligand with the receptor domain is selected on the basis of the aforementioned determination. Alternatively, improvement of the ligand molecule necessary for causing more effective docking is simulated by the computer.
(4) A ligand obtained by the aforementioned process (3) (i.e., a ligand selected or improved by the process (3)) is likely to function as an effective antiviral agent which suppresses or inhibits proliferation of HEV.

HEV is a virus which includes a number of variants. Therefore, in order to develop and/or improve an antiviral agent being equally effective to all of the variant strains (or as many strains as possible) of HEV, it is necessary to obtain the information of nucleotide sequence of all the existing strains (or as many existing strains as possible) of HEV. According to the aforementioned aspects of the present invention, an advantageous effect is achieved in obtaining such information of nucleotide sequence.

Any of the ORF1 regions derived from the aforementioned novel HEV strains may be utilized in the manner described above. A drug obtained by the aforementioned method of designing drug is also included within the scope of the present invention.

### 13. Assay kit

In the present invention, the polynucleotide, polynucleotide fragment, polynucleotide probe, polynucleotide primer, polypeptide, antigen and antibody of any of the aforementioned embodiments may be provided as a kit in an appropriate manner, in accordance with the application purpose. For example, in order to produce a chip for detecting nucleotide sequence, a kit may be formed by combining the polynucleotide/polynucleotide's with a substrate and/or various reagents and/or a marker substance. Further, a kit may be formed by combining the polynucleotide probe with a reaction container and/or salts for buffer solution and/or other necessary reagents. For example, a kit may be formed by combining the polynucleotide primer with a reaction container and/or salts for buffer solution and/or other necessary reagents e.g., polymerase and/or a substrate. Note that the kit provided according to the present invention is not restricted to the aforementioned examples or combinations, but kits of other types may be provided according to necessity by combining the polynucleotide/polypeptide/antigen/antibody components of the present invention with various substances. The application purpose of the kit is not restricted to the aforementioned examples, either, and a kit for any of the application purposes or objects described above may flexibly be formed. Such a kit as described above is also included within the scope of the present invention.

Examples with regards to HEV genome detection according to the present invention will be described hereinafter. It should be noted that the following description is provided only for a demonstrational purpose and by no means restricts the scope of the present invention.

### Example 1. Detection of HEV genome RNA by RT-PCR method

### 1. Primer

In the RT-PCR of the present example, four types of oligonucleotide primers respectively having following nucleotide sequences, selected from the nucleotide sequences derived from HEV-JRA1 disclosed at the SEQ No. 1 or the SEQ No. 2 of the sequence list, were used.
#HE5-1 (5'-TCGATGCCATGGAGGCCCA-3') (sense primer, which corresponds to nt 19-37 of the sequence list 1) (The underlined portion corresponds to the SEQ No. 2 of the sequence list)
#HE5-2 (5'-GCCYTKGCGAATGCTGTGG-3') (sense primer, which corresponds to nt 105-123 of the sequence list 1) (Y = C or T; K = G or T) (The underlined portion corresponds to the SEQ No. 3 of the sequence list)
#HE5-3 (5'-TCRAARCAGTARGTGCGGTC-3') (antisense primer, which corresponds to nt 450-469 of the sequence list 1) (R = A or G) (The underlined portion corresponds to the SEQ No. 4 of the sequence list)
#HE5-4 (5'-CATAGCCTCSGCRACATCAG-3') (antisense primer, which corresponds to nt 541-560 of the sequence list 1) (S = G or C; R = A or G) (The underlined portion corresponds to the SEQ No. 5 of the sequence list)

### 2. Detection method

First, nucleic acid was extracted from 50 µL of the serum collected from the patient, by using SMITEST EX R & D (Genome Science Laboratories). #HE5-4 as an antisense primer was added to the extracted nucleic acid. The nucleic acid was reacted with the added antisense primer at 37°C for 30 minutes, under the presence of polymerase MMLV-RT (Stratagene), whereby the synthesis of cDNA (i.e., the reaction of reverse transcription from RNA to DNA) was carried out.

Next, the cDNA synthesized as described above was subjected to nested PCR by using the aforementioned four types of primers and Fast Start Taq DNA Polymerase (Roche Co., Ltd.). During the nested PCR, the temperature was changed such that the initial 95°C lasted for 4 minutes was followed by 30 cycles of {95°C for 30 seconds, 55°C for 30 seconds, 72°C for 45 seconds} and the final 72°C for 7 minutes. The product obtained as a result of the PCR reaction was subjected to electrophoresis by using agarose gel. Presence/absence of a band derived from HEV genome having length of 365 bp was checked.

### 3. Result and discussion

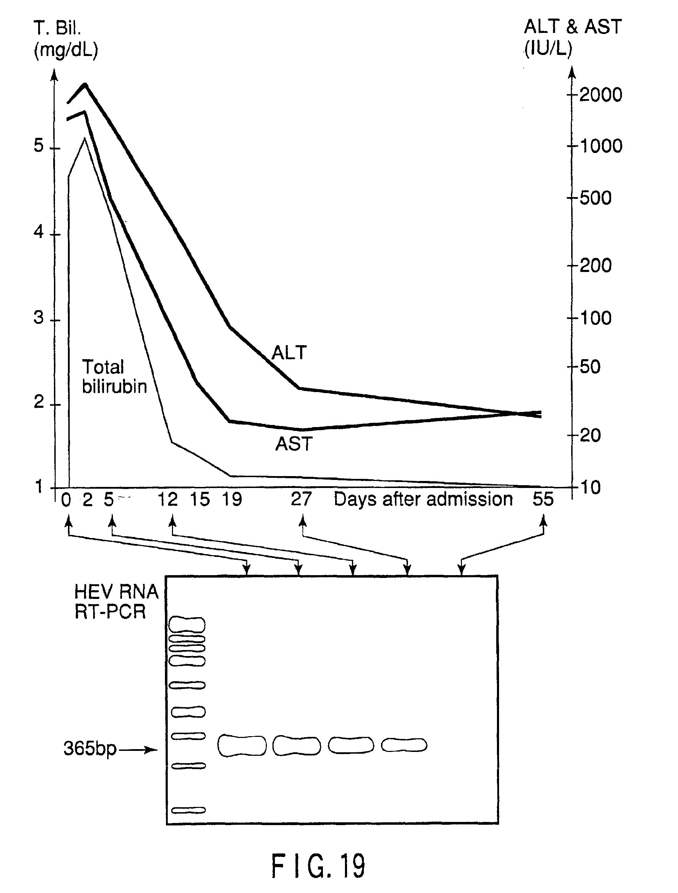

FIG. 19 shows the result of detecting HEV genome RNA by the aforementioned method, in a serum sample successively collected from a Japanese acute hepatitis patient. The graph of FIG. 7 shows changes in the values of liver function test which indicate the progress of hepatitis. The abbreviations of the terms used in FIG. 7 are as follows. "AST" represents aspartic aminotransferase. "ALT" represents alanine aminotransferase. "Total bilirubin" represents the concentration of bilirubin as a whole. The photograph of FIG. 7 shows the result of electrophoresis of the PCR product, derived from the serum collected from the same patient at the timing corresponding to the graph (i.e., by RT-PCR: the reverse transcription polymerase chain reaction method). The X-axis represents the days during which the patient was hospitalized.

In the case shown in FIG. 7, HEV RNA was continually detected in the serum of the patient, from the initial stage of the disease, throughout the hospitalized period exceeding 27 days. This is an epoch-making discovery which denies the conventional knowledge, because it has conventionally been considered, as common sense, that HEV RNA appears in blood only during a very short period until AST, ALT and Total bilirubin reach the peaks thereof (Purcell, R. H. In Fields Virology, eds. Fields, B. N., Knipe, D. M., & Howley, P. M. Lippincott-Raven, Philadelphia, 1996, 3^{rd} Ed., Vol. 2, pp. 2831-2843). The results shown in FIG. 7 indicate that the detection system of HEV genome RNA, which can be constructed according to the present invention, detects HEV genome RNA with very high sensitivity and that the clinical data obtained from the analysis using such a detection system effectively and thus usefully deepens the academic understanding of HEV infection.

### Example 2. Isolation of the novel HEV strain

Tests were conducted for seven acute hepatitis E patients of seven cases. Among the seven cases, the patients of five cases in which JHA-Sap, JKK-Sap, JKN-Sap, JMY-Haw and JSY-Sap were isolated had lived in Hokkaido. The patients of two cases in which JAK-Sai and JMM-Sai were isolated had lived in Saitama prefecture. Each patient developed the disease in an isolated manner i.e., with no contact with other patients regarding both time and place. Further, the case of each patient had nothing to do with any local epidemic of the disease. In six of the seven cases, patients had not been abroad recently. Only the patient from whom JMY-Haw was isolated had been to Hawaii as a tourist one month before developing the disease. The serum sample was collected from the patient at the acute state, frozen at a temperature of - 20°C or below and stored until the virological analysis was carried out.

The HEV sequence was determined, basically according to the method proposed by Takahashi (Takahashi K, Iwata K, Watanabe N, et al. Virology 2001; 287:9-12), with some improvements added thereto. The nucleic acid sample was extracted from 25 mL of serum by using a commercially available kit for nucleic acid extraction (SMITEST EX-R & D (Genome Science Laboratories)). The first strand cDNA was synthesized at 37°C for 30 minutes by using the reverse transcripase of Moloney mouse leukemia virus (produced by Stratagene Co., Ltd.). As the antisense primer, a mixture of HE5-4 (5'-CATAGCCTCSGCRACATCAG-3'; NT541-560) and HE5-5 (5'-CATYGCCTCSGCAACATCGG-3'; nt541-560, the positions of the respective nucleotides correspond to those of HEV-JRA1 strain) was used. Next, the obtained cDNA was subjected to the nested polymerase chain reaction (referred to as "PCR" hereinafter). For the first round PCR, a mixture of Fast Start Taq DNA Polymerase (produced by Roche Co., Ltd.), an outer-side sense primer HE5-1 (5'-TCGATGCCATGGAGGCCCA-3'; nt 19-37), and an outer-side antisense primer HE5-4/HE5-5 (with regards to the sequences thereof, refer to the aforementioned description), was used. Subsequently, for the second round PCR, a mixture of an inner-side sense primer HE5-2 (5'-GCCYTKGCGAATGCTGTGG-3'; nt 105-123), an inner-side antisense primer HE5-3 (5'-TCRAARCAGTARGTGCGGTC-3'; nt 450-569) and HE5-6 (5'-TYAAAACAGTAGGTTCGATC-3'; nt 450-469) was used. In the aforementioned PCR, the temperature was changed such that the initial 95°C lasted for 4 minutes was followed by 30 cycles of {95°C for 30 seconds, 55°C for 30 seconds, 72°C for 45 seconds} and the final 72°C for 7 minutes.

As a result, the 326-nt region was amplified from ORF1 of the HEV genome. Next, the sequencing process was carried out by using "Dye Terminator Cycle Sequencing FS Ready Reaction Kit" (produced by Perkin-Elmer Applied Biosystems Co., Ltd.) and "373A DNA sequencer" (produced by Applied Biosystem Co., Ltd.).

The sequences obtained from the strains isolated (i.e., derived) from the Japanese patients were compared with the sequences of the strains isolated from patients of various foreign nationalities. A computer software (GENETYX-MAC version 10.1 (Software Development Co., Ltd.) was used for comparison.

The result of the comparison described above is shown in FIG. 14. When the 326-nt ORF1 sequences of the seven strains endemic to Japan were compared with each other, the seven strains were classified into three main groups. JMM-Sai sequence exhibited homology of 73.0% to 75.7% with respect to each of the other six isolated strains. JKN-Sap, JHA-Sap and JMY-Haw exhibited relatively high homology of 95.4% to 98.8% with respect to the strains belonging to the same group, but exhibited relatively low homology of 73.0% to 79.1% with respect to the isolated strains belonging to other groups. JSY-Sap, JKK-Sap and JAK-Sai exhibited relatively high homology of 89.0% to 99.7% with respect to each other, but exhibited relatively low homology of 74.8% to 78.8% with respect to the other four strains. The entire-length genome of JRA1 strain as the prototype had homology of less than 90% with respect to these isolated strains.

### Example 3. Comprehensive detection of HEV

First, blood was collected from a plurality of patients. Nucleic acid was extracted from 50 µL of the serum collected from each patient, by using SMITEST EX R & D (Genome Science Laboratories). 5'-gcagaccacrtatgtgktcg-3' (SEQ No. 32) and 5'-ccacrtatgtggtcgaygcc-3' (SEQ No. 33) as the sense primers, as well as 5'-acmarctgscgrggytgcat-3' (SEQ No. 34) and 5'-cgytgratwggrtgrttcca-3' (SEQ No. 35) as the antisense primers were added to each of the extracted nucleic acid. Each nucleic acid was reacted with the added sense primer and antisense primer at 37°C for 30 minutes, under the presence of polymerase One-step RT-PCR (Stratagene), whereby the synthesis of cDNA (i.e., the reaction of reverse transcription from RNA to DNA) was carried out.

Next, each of the cDNA synthesized as described above was subjected to nested PCR by using: Fast Start Taq DNA Polymerase (Roche Co., Ltd.); 5'-tgktcgaygccatggaggc-3' (SEQ No. 36), 5'-tgktcgaygccatggaggc-3' (SEQ No. 37) and 5'-aygccatggaggcccaycag-3' (SEQ No. 38) as the sense primer; and 5'-ckracyaccacagcattcgc-3' (SEQ No. 39) and 5'-ggcckracyaccacagcatt-3' (SEQ No. 40) as the antisense primer. During the nested PCR, the temperature was changed such that the initial 95°C lasted for 4 minutes was followed by 30 cycles of {95°C for 30 seconds, 55°C for 30 seconds, 72°C for 45 seconds} and the final 72°C for 7 minutes. The PCR product-obtained as a result of the PCR reaction was subjected to electrophoresis by using agarose gel.

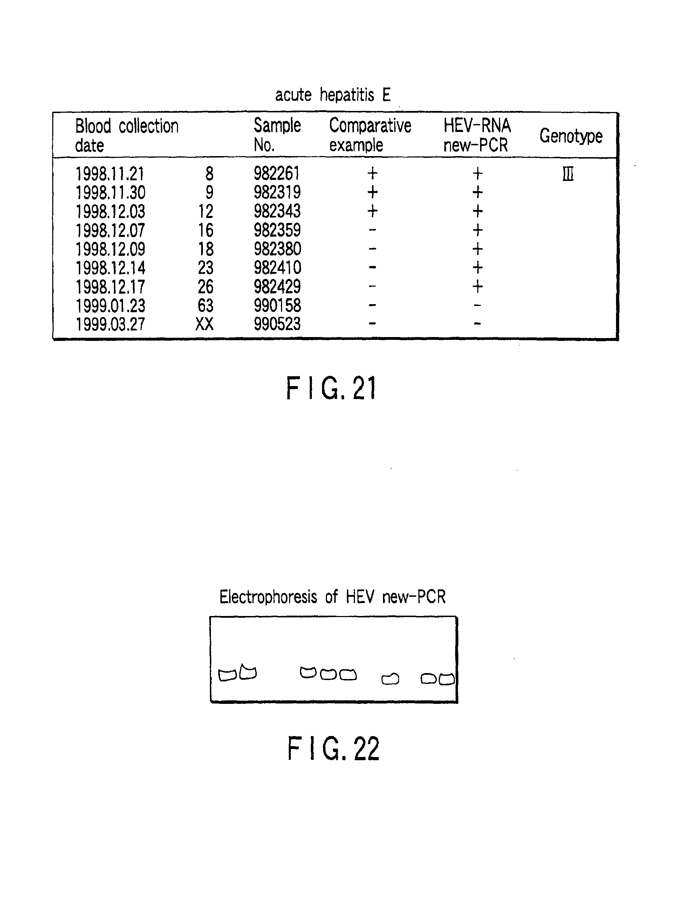

As a result, amplified products were obtained by the aforementioned method of the present example, for 8 cases of the total 37 cases. For the 8 cases in each of which a band was obtained as a result of electrophoresis, the obtained bands are schematically shown in FIG. 22. Each band obtained for each sample was observed at different positions, as shown in FIG. 22. This result indicates that, in the above-described 8 cases, the plural primers used therein acted, as some different combinations of the primers, on the HEV genome in the sample and effected amplification. In other words, the result indicates that the genotypes of HEV detected in the HEV-positive patients are different from each other.

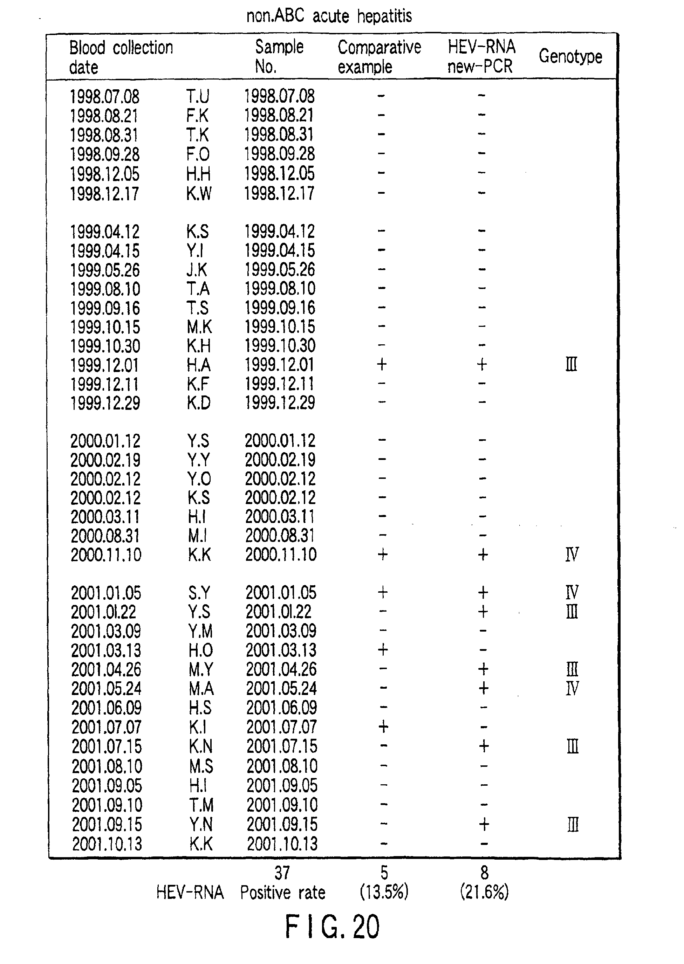

The amplification reaction was carried out for each of the 37 samples of the non-A,B,C type acute hepatitis patients. The result of the amplification is summarized in FIG. 20, in a manner that the result obtained by the conventional method is compared with the result obtained by the method of the present example. The column "Blood collection date" of FIG. 20 represents the date when blood was collected from each patient. The alphabet letters right next to "Blood collection date" represent the initials of the patient. "Sample No." represents the serial sample number used in the hospital. The column "Comparative example" of FIG. 20 indicates presence/absence of HEV detection when amplification was effected by using the conventional primer. The column "new-PCR " indicates presence/absence of HEV detection by the method of the present example. In each column of "Comparative example" and "new-PCR", "+" indicates that HEV was detected and "-" indicates that HEV was not detected. As shown in FIG. 20, the method of the present example was capable of detecting the virus strains which the conventional primer failed to detect, although the same amplification and electrophoresis processes were conducted in the two cases.

### Example 4. Follow-up check of a patient

For a patient who developed acute hepatitis E, presence/absence of the virus in blood of the patient was successively checked. As the measuring method, the method according to the present invention i.e., the method described in example 3 and the conventional method described in example 3 were employed. The result is shown in FIG. 21. The leftmost column of FIG. 21 represents the date when blood was collected. The number right next to "Blood collection date" indicates the period (days) counted from the day when the patient was hospitalized. "Sample No." of FIG. 21 represents the serial sample number used in the hospital. The column "Comparative example" of FIG. 21 indicates presence/absence of HEV detection when amplification was effected by using the conventional primer. The column "new-PCR" indicates presence/ absence of HEV detection by the method of the present example. In each column of "Comparative example" and "new-PCR", "+" indicates that HEV was detected and "-" indicates that HEV was not detected.

From the result of FIG. 21, it is understood that the method using the primer of the present invention enables detection of virus, even when the virus in blood has been reduced to such a degree that the conventional method can no longer detect the virus.

### Example 5. Genotype identification of HEV

Next, for each of the products obtained as a result of amplification in example 3, the genotype thereof was identified by using a probe-fixed plate as described below.

### (1) Probe-fixed plate

A probe of 53 mer (5'-aaggctcctggcrtyactactgcyatwgagcaggcwgctctrgcwgcggccaa-3'), a probe for HEV-I, II (5'-ctcctggcatcactactgc-3'), a probe for HEV-III (5'-ctcctggcattactactgc-3') and a prove for HEV-IV (5'-ctcctggcgtcactactg-3') were solid-phase fixed, respectively, on a well of a commercial microtiter platewell.

### (2) Genotype identification by hybridization assay

Each of the products obtained as a result of amplification in example 3 was added to the probe-fixed plate prepared in the aforementioned (1), whereby hybridization was effected. Thereafter, optical density (O. D.) of each well was measured.

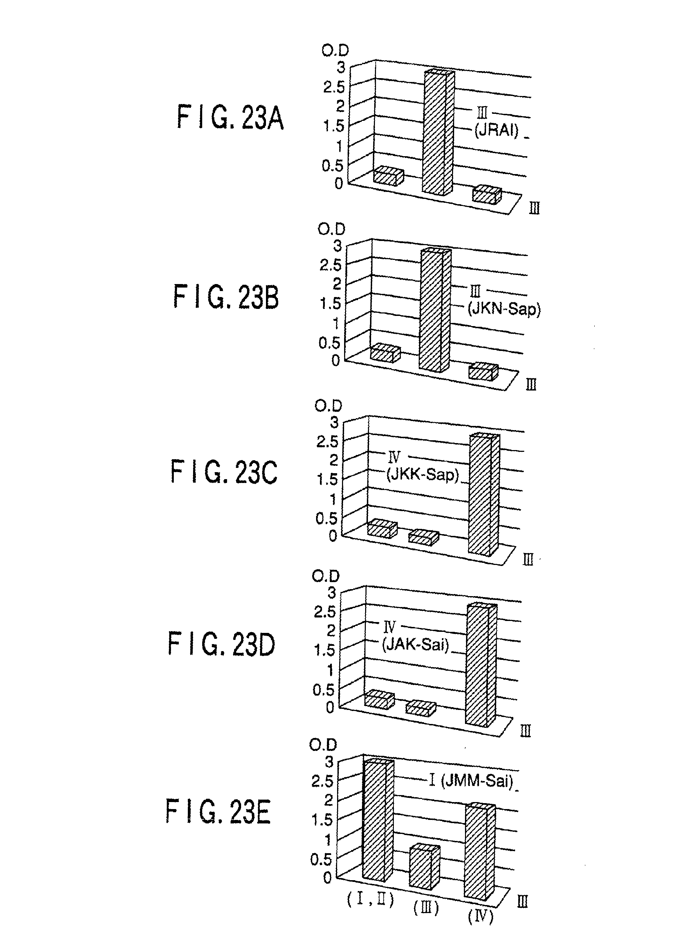

The results are shown in FIGS. 23A to 23E. The graphs of FIG. 23A, FIG. 23B, FIG. 23C, FIG. 23D and FIG. 23E show the result of genotype identification conducted for the HEV having different genotypes, i.e., JRA1 strain, JKN-Sap strain, JKK-Sap strain, JAK-Sai strain and JMM-Sai strain, respectively. In all of the graphs, the X-axis represents the HEV-I, II solid phase well, the HEV-III solid phase well, and the HEV-IV (54 mer primer) solid phase well, from left to right in this order. In the case of genotype III, only the O. D. of the well in which the probe for HEV-III had been solid-phase fixed was distinctly high. In the case of genotype IV, only the O. D. of the well in which the probe for HEV-IV had been solid-phase fixed was distinctly high. In the case of genotype I, the well in which the probe for HEV-I, II had been solid-phase fixed exhibited relatively high O. D., as compared with the other wells. On the basis of the indexes described above, the genotype of the virus in the serum of each patient was identified by using the corresponding probe. The result of the genotype identification was shown in the column "genotype" of FIG. 20. As is obvious from the result shown in FIG. 20, it has been demonstrated that genotype identification of HEV collected from a patient can be easily carried out by using the probe according to the present invention.

The total contents of all the references cited in the present specification are incorporated in the present specification, by reference.

## Claims

1. A polynucleotide probe including a sequence comprising at least eight nucleotides, the polynucleotide probe being used for detecting polynucleotides of hepatitis E virus, **characterized in that**:
(1) the sequence comprising at least eight nucleotides is hybridized with the polynucleotides of the hepatitis E virus, thereby, due to the hybridization, detecting the hepatitis E virus; and
(2) the sequence comprising at least eight nucleotides is obtained from a sequence selected from the group consisting of nucleotide sequences disclosed at SEQ No. 11, SEQ No. 44, SEQ No. 45, SEQ No. 46, SEQ No. 47 and SEQ No. 48, and complementary strands thereof.

2. A polynucleotide probe according to claim 1, wherein the sequence comprising at least eight nucleotides is obtained from a nucleotide sequence coding a non-structural protein and nucleocapsid protein of HEV, or a complementary strand thereof.

3. A probe assay kit, including the polynucleotide probe according to claim 1.

4. A polynucleotide probe according to claim 1, wherein the sequence comprising at least eight nucleotides is selected from the group consisting of sequences represented by the ranges: the 19^{th} nucleotide to the 37th nucleotide of SEQ No. 15;the 52nd nucleotide to the 69th nucleotide of SEQ No. 15;the 77th nucleotide to the 95th nucleotide of SEQ No. 15;the 111th nucleotide to the 127th nucleotide of SEQ No. 15; the 174th nucleotide to the 181st nucleotide of SEQ No. 15; the 213th nucleotide to the 220th nucleotide of SEQ No. 15; and the 48th nucleotide to the 100th nucleotide of the SEQ No. 15; and complementary strands thereof.

5. A polynucleotide probe according to claim 1, wherein the hepatitis E virus causes fulminant hepatitis E and the sequence comprising at least eight nucleotides, selected according to (2) of claim 1, is obtained from a nucleotide sequence disclosed at SEQ No. 11.

6. A polynucleotide probe according to claim 1, wherein the hepatitis E virus is fulminant hepatitis E virus and the sequence comprising at least eight nucleotides, selected according to (2) of claim 1, is selected from the group consisting of nucleotide sequences disclosed at SEQ No. 9 and SEQ No. 10.

7. A method of detecting the presence of hepatitis E virus in a sample, comprising:
(1) obtaining a sample from an object;
(2) reacting the sample obtained in (1) with the polynucleotide probe according to claim 1;
(3) detecting a double strand produced as a result of the reaction (2);
(4) determining whether or not hepatitis E virus is present in the sample, on the basis of the detection result of (3).

8. A method of detecting presence of fulminant hepatitis E virus in a sample, comprising:
(1) obtaining a sample from an object;
(2) reacting the sample obtained in the obtaining of (1) with the polynucleotide probe according to claim 6;
(3) detecting a double strand produced as a result of the reaction of (2);
(4) determining whether or not hepatitis E virus is present in the sample, on the basis of the detection result of (3).

9. A method of determining the genotype of hepatitis E virus in a sample, comprising:
(1) reacting a sample with the polynucleotide probe according to claim 1;
(2) detecting a double strand produced as a result of the reaction of (1);
(3) determining genotype of hepatitis E virus present in the sample, on the basis of the detection result of (2).

10. A chip for detecting a nucleotide sequence, on which the polynucleotide probe according to claim 1 has been solid-phase fixed.

11. A chip for detecting a nucleotide sequence, on which the polynucleotide probe according to claim 6 has been solid-phase fixed.

12. A pair or a plurality of pairs of primers for PCR for amplifying polynucleotide of hepatitis E virus, the at least a pair of primer for PCR each independently having a sequence comprising at least eight nucleotides, **characterized in that**:
(1) the sequence comprising at least eight nucleotides is hybridized with the polynucleotides of the hepatitis E virus, thereby, due to the hybridization, amplifying a portion of the polynucleotides of the hepatitis E virus; and
(2) the sequence comprising at least eight nucleotides is obtained from a sequence selected from the group consisting of nucleotide sequences disclosed at SEQ No. 11, SEQ No. 44, SEQ No. 45, SEQ No. 46, SEQ No. 47 and SEQ No. 48, and complementary strands thereof.

13. A pair a plurality of pairs of primers for PCR according to claim 12, wherein the sequence comprising at least eight nucleotides is obtained from a nucleotide sequence coding a nucleocapsid protein of HCV, or a complementary strand thereof.

14. A PCR assay kit, comprising the pair or the a plurality of pairs of primers for PCR according to claim 12.

15. A pair or a plurality of pairs of primers for PCR according to claim 12, wherein the sequence comprising at least eight nucleotides is selected from the group consisting of sequences represented by the ranges: the 19th nucleotide to the 37th nucleotide of SEQ No. 15; the 52nd nucleotide to the 69th nucleotide of SEQ No. 15; the 77th nucleotide to the 95th nucleotide of SEQ No. 15; the 111th nucleotide to the 127th nucleotide of SEQ No. 15; the 174th nucleotide to the 181st nucleotide of the SEQ No. 15; the 213th nucleotide to the 220th nucleotide of the SEQ No. 15; and the 48th nucleotide to the 100th nucleotide of SEQ No. 15; and complementary strands thereof.

16. A pair or a plurality of pairs of primers for PCR according to claim 12, wherein the hepatitis E virus causes fulminant hepatitis E and the sequence comprising at least eight nucleotides selected according to (2) of claim 12 is obtained from a nucleotide sequence disclosed at SEQ No. 11.

17. A pair or a plurality of pairs of primers for PCR according to claim 12, wherein the hepatitis E virus is fulminant hepatitis E virus and the sequence comprising at least eight nucleotides selected according to (2) of claim 12 is selected from the group consisting of nucleotide sequences disclosed at SEQ No. 9 and SEQ No. 10.

18. A method of detecting presence of hepatitis E virus in a sample, comprising:
(1) obtaining a sample from an object;
(2) reacting the sample obtained in the obtaining of (1) with a pair of primers for PCR according to claim 12 and a polymerase, under conditions suitable for amplification ;
(3) detecting presence of a product obtained as a result of amplification by the reaction of (2);
(4) determining whether or not hepatitis E virus is present in the sample, on the basis of the detection result of (3).

19. A method of detecting presence of fulminant hepatitis E virus in a sample, comprising:
(1) obtaining a sample from an object;
(2) reacting the sample obtained in (1) with the at least one pair of primers for PCR according to claim 17 and a polymerase, under conditions suitable for amplification ;
(3) detecting the presence of a product obtained as a result of amplification by the reaction of (2);
(4) determining whether or not hepatitis E virus is present in the sample, on the basis of the detection result of (3).

20. A method of determining the genotype of hepatitis E virus in a sample, comprising:
(1) reacting a sample with a pair of primers for PCR according to claim 12 and a polymerase, under conditions suitable for amplification ;
(2) determining the length of a product obtained as a result of amplification by the reaction of (1);
(3) determining the genotype of hepatitis E virus present in the sample, on the basis of the detection result of (2).


SEQUENCE LISTING

<110> KABUSHIKI KAISHA TOSHIBA

<120> Polynucleotide probe and primer derived from hepatitis E virus
collected from Japanese, chip including the same, kit including the
same, and method of detecting hepatitis E virus using the same

<130> EPP86647

<140> PCT/JP02/06365
<141> 2002-6-25

<150> JP2001-191837
<151>2001-6-25

<160> 57

<210> 1
<211> 5138
<212> DNA
<213> Hepatitis E virus JRA1

<400> 1
ggcagaccac gtatgtggtc gatgccatgg aggcccacca gttcattaag gctcctggca 60
ttactactgc catagagcag gctgctctgg ctgcggccaa ctccgccttg gcgaatgctg 120
tggtggttcg gccgtttttg tctcgcgtac aaaccgagat ccttattaat ttgatgcaac 180
cccggcagtt ggttttccgc cctgaggtgc tttggaacca tcctattcag agggttatac 240
acaatgaatt agaacagtac tgccgggccc gggccggtcg ttgcctggag attggggctc 300

atccaagatc tattaatgat aaccccaatg ttttgcaccg gtgttttctc aggccggtcg 360
gtagggacgt ccagcgctgg tattctgccc ccacccgcgg ccctgcagcc aactgccgcc 420
gctctgcatt gcgtggcctc ccccctgtcg accgcactta ctgttttgat ggattttcca 480
gttgtgcttt cgccgcagag accggcgtgg ccctttactc tctacatgac ctttggccag 540
ctgatgttgc ggaggctatg gcccgccatg gaatgacacg tctttatgct gcactccacc 600
tccctcccga ggtgttgtta ccacccggta cttaccacac aacctcgtat cttctgatcc 660
atgacggcaa ccgtgctgtt gtgacttatg aaggtgatac tagtgcaggt tacaaccatg 720
acgtttccat acttcgtgca tggattcgca cgactaaaat agttggtgac catccgttgg 780
ttatagagcg tgtgcgggct attggttgtc attttgtgct gctgctcacc gcggcccctg 840
agccatcgcc catgccttac gtcccttacc cccgttccac ggaggtgtat gtccgatcta 900
tatttggccc tggtggctcc ccatccttgt ttccgtcagc ttgctctaca aaatctacct 960
tccatgctgt tccagttcac atatgggacc ggcttatgct ttttggtgcc accttggacg 1020
accaggcgtt ttgctgctca cggctcatga catacctccg tggcattagc tacaaagtca 1080
ctgtcggtgc gcttgttgct aatgaggggt ggaatgcctc tgaagatgcc ctcaccgcag 1140
taatcactgc agcctacttg accatctgtc accagcgcta cctccgtacc caggcgatat 1200
ctaagggtat gcgccggttg gaggttgagc acgcccagaa gtttattaca agactttaca 1260
gctggttatt tgagaagtct ggtcgtgact acatccccgg ccgccaactt cagttctatg 1320
cccagtgccg gcggtggcta tctgcgggct ttcacctaga ccccagagtg ctcgtctttg 1380
atgagtcagt gccctgccgc tgtaggactt tcttaaagaa ggttgcgggt aaattctgtt 1440
gcttcatgcg gtggttaggg caggagtgta cttgtttctt agaaccagct gagggcttag 1500
ttggcgatca aggccatgat aatgaggctt atgagggttc tgaggttgac caggccgagc 1560
ctgtacacct tgatgtgtcg gggacctatg ctgtccacgg gcaccagctt gaggccctct 1620
acagggcact caacattcca cacgatattg ctgctcgagc tgcccgatta acggctactg 1680
ttgagctcgc tgcaggcccc gaccgtctgg agtgccgcac cgtgctcggg aacaagactt 1740
tccggacgac ggtgactgat ggcgcccatc tagaagcgaa cggccctgag caatatgttc 1800
tgtcgttcga tgcctcccgc cagtctatgg gggccgggtc ccatagcctc acttacgagc 1860
ttacacccgc cggcttgcag gttaagatct catctaatgg cctagactgc actgccgtat 1920
tcccccctgg tggcgcccct agcgcgccgc cgggggaggt ggcggctttt tgcagcgccc 1980
tctacaggta taacagattc acccagcggc attcgctgac cggcggtttg tggctacatc 2040

ccgaggggtt gctaggcgtt ttcccccctt tttcccctgg gcacatttgg gagtctgcca 2100
accccttctg cggtgagggt accttgtata ctcgcacctg gtctacatct ggtttttcta 2160
gtgacttttc ccctcctgag gcggccgccc ctgcaccggc tgccgcccca gggtcgtcct 2220
cccccactcc accagtcagt gatatttggg tgttaccacc gctttcagag gagccccagg 2280
tgggtgcgcc gcctgcacct cccacccccg agcctgctcg gctgccctgc cccactaaac 2340
ctaacacccc cgtgcgtaag ccaacggcac cgccgccttc tcgcacccgc cgccttcttt 2400
acacctatcc tgatggtgct aaggtgtatg cggggtcact gtttgagtca gattgtgatt 2460
ggctggtgaa tgcctctaac ccgggccatc gtcctggggg tggcctctgt cacgcctttt 2520
accaacgttt tcccgaggcg ttctatccaa ctgaatttat tatgcgtgag ggtcttgcag 2580
catacacctt gaccccgcgc cctattatcc atgcagtggc ccccgactat agggttgagc 2640
agaatccaaa gaggcttgag gcagcgtacc gagaaacttg ctcccgccgc ggcaccgctg 2700
cctatccact tcttggctca ggtatatacc aggtccctgc tggtcttagt tttgatgcct 2760
gggagcgtaa ccatcgccct ggcgatgagc tttacctgac tgaacccgct gcagcctggt 2820
ttgaagctaa taagccaacg cagccagcac ttacgataac ggaagataca gctcgcacgg 2880
ccaacctagc attagagatc gatgctgcca cagacgttgg ccgtgcttgt gccggctgca 2940
ctattagccc cgggattgtg cattatcagt tcactgccgg ggtcccaggc tcaggcaaat 3000
caaggtccat acagcagggg gatgtcgatg ttgtggttgt ccccacccgg gaacttcgca 3060
atagctggcg ccgtcggggc tttgcggcct ttacacccca cacggcggcc cgtgttacta 3120
taggccgccg tgttgttatt gacgaggccc catcccttcc accgcacttg ttgctgttac 3180
acatgcagcg ggcctcctca gtccatctcc tcggtgaccc aaatcagatc cctgccatcg 3240
acttcgagca tgccggcctg gtccccgcaa ttcgccctga gcttgcacca acgagctggt 3300
ggcatgttac gcatcgctgt ccggccgatg tatgtgagct catacgcgga gcctacccta 3360
aaatccagac cacgagccgt gtgctacggt ctctgttctg gaatgagccg gctattggcc 3420
agaagttggt cttcacgcag gccgctaaag ctgccaaccc tggtgcaatt acggtccatg 3480
aagcccaggg tgctaccttc acagagacca caattatagc cacggctgat gccaggggtc 3540
ttattcagtc atcccgggcc catgctatag ttgcacttac ccgccacaca gagaagtgcg 3600
ttattttgga tgcccctggt ttgttgcgcg aggtcggcat atcagatgta attgttaaca 3660
actttttcct tgctggcgga gaggtgggcc atcaccgccc ctctgtgata cctcgtggca 3720
atcctgaccg gaaccttgac accctacagg ccttcccgcc atcctgccaa attagtgctt 3780

accatcagtt ggctgaggag ttaggccatc gcccggctcc tgttgctgcc gttttgcccc 3840
cctgccccga gctcgagcag ggcttattgt atatgccaca ggagcttaca gtgtccgata 3900
gtgtgttggt ttttgagctc actgacatag tccattgccg catggctgcc ccaagtcagc 3960
ggaaggccgt cctctcaaca cttgtgggga ggtatggccg caagacgaaa ttgtatgagg 4020
cagcccattc agatgttcga gagtctctag ctaggttcat tcccactatc ggacctgtcc 4080
aggccaccac gtgtgagtta tatgaactgg ttgaggccat ggtggaaaag ggccaggacg 4140
gctctgccgt cctggagctt gatctatgta atcgtgatgt ttcgcgcatt acattctttc 4200
agaaagactg taataagttt acaactggtg agaccattgc ccatggtaag gttggccagg 4260
gcatatcggc ctggagtaag accttttgcg ccctgtttgg tccgtggttt cgtgccattg 4320
aaaaagaaat actagccctg ctcccgccta atatcttcta cggcgacgcc tacgaggagt 4380
cggtgtttgc cgcggccgtg tccggggcgg ggtcttgcat ggtatttgaa aatgactttt 4440
cggaatttga tagtactcag aacaatttct cccttggcct tgagtgtgtg gttatggaag 4500
agtgtggtat gccccaatgg ctgatcagat tgtatcacct ggtccggtca gcctggattc 4560
tgcaggcgcc aaaggagtct cttaaaggtt tctggaagaa gcattctggt gagcctggta 4620
cccttctctg gaacaccatc tggaacatgg cgatcatagc ccattgctat gagtttcgcg 4680
atttccgggt cgccgctttt aagggtgatg actcggtagt cctctgtagt gattaccgac 4740
agagtcgtaa cgcggcagcc ttaatcgcag gttgtgggct caagttgaag gttgactatc 4800
gccctattgg gctgtacgct ggtgtggtgg tggcccctgg cttggggaca ctgcctgatg 4860
tagtgcgatt tgctggccgg ctgtccgaaa agaattgggg ccctggccca gagcgtgctg 4920
agcagctgcg tcttgctgtc tgtgacttcc ttcgagggtt aacgaatgtt gcgcaggttt 4980
gtgttgatgt tgtgtcccgt gtttatggag tcagccccgg gctggtacat aaccttattg 5040
gcatgctgca gaccattgct gatggcaagg cccactttac agagtcaatt aaacctgtgc 5100
ttgaccttac aaattctatt atacagcggg tggaatga 5138

<210> 2
<211> 15
<212> DNA
<213> Hepatitis E virus

<400> 2

tgccatggag gccca                                                    15

<210> 3

<211> 15

<212> DNA

<213> Hepatitis E virus

<400> 3

tkgcgaatgc tgtgg                                                    15

<210> 4

<211> 15

<212> DNA

<213> Hepatitis E virus

<400> 4

rcagtargtg cggtc                                                    15

<210> 5

<211> 15

<212> DNA

<213> Hepatitis E virus

<400> 5

cctcsgcrac atcag                                                    15

<210> 6

<211> 15

<212> DNA

<213> Hepatitis E virus

<400> 6

gtcggtaggg acgtc 15

<210> 7

<211> 2442

<212> DNA

<213> Hepatitis E virus

<400> 7

tcgatgccat ggaggcccac cagttcatta aggctcctgg cattactact gccatagagc 60

aggctgctct ggctgcggcc aactccgcct tggcgaatgc tgtggtggtt cggccgtttt 120

tgtctcgcgt acaaaccgag atccttatta atttgatgca accccggcag ttggttttcc 180

gccctgaggt gctttggaac catcctattc agagggttat acacaatgaa ttagaacagt 240

actgccgggc ccgggccggt cgttgcctgg agattggggc tcatccaaga tctattaatg 300

ataaccccaa tgttttgcac cggtgttttc tcaggccggt cggtagggac gtccagcgct 360

ggtattctgc ccccacccgc ggccctgcag ccaactgccg ccgctctgca ttgcgtggcc 420

tcccccctgt cgaccgcact tactgttttg atggattttc cagttgtgct ttcgccgcag 480

agaccggcgt ggccctttac tctctacatg acctttggcc agctgatgtt gcggaggcta 540

tggcccgcca tggaatgaca cgtctttatg ctgcactcca cctccctccc gaggtgttgt 600

taccacccgg tacttaccac acaacctcgt atcttctgat ccatgacggc aaccgtgctg 660

ttgtgactta tgaaggtgat actagtgcag gttacaacca tgacgtttcc atacttcgtg 720

catggattcg cacgactaaa atagttggtg accatccgtt ggttatagag cgtgtgcggg 780
ctattggttg tcattttgtg ctgctgctca ccgcggcccc tgagccatcg cccatgcctt 840
acgtccctta cccccgttcc acggaggtgt atgtccgatc tatatttggc cctggtggct 900
ccccatcctt gtttccgtca gcttgctcta caaaatctac cttccatgct gttccagttc 960
acatatggga ccggcttatg ctttttggtg ccaccttgga cgaccaggcg ttttgctgct 1020
cacggctcat gacatacctc cgtggcatta gctacaaagt cactgtcggt gcgcttgttg 1080
ctaatgaggg gtggaatgcc tctgaagatg ccctcaccgc agtaatcact gcagcctact 1140
tgaccatctg tcaccagcgc tacctccgta cccaggcgat atctaagggt atgcgccggt 1200
tggaggttga gcacgcccag aagtttatta caagacttta cagctggtta tttgagaagt 1260
ctggtcgtga ctacatcccc ggccgccaac ttcagttcta tgcccagtgc cggcggtggc 1320
tatctgcggg ctttcaccta gaccccagag tgctcgtctt tgatgagtca gtgccctgcc 1380
gctgtaggac tttcttaaag aaggttgcgg gtaaattctg ttgcttcatg cggtggttag 1440
ggcaggagtg tacttgtttc ttagaaccag ctgagggctt agttggcgat caaggccatg 1500
ataatgaggc ttatgagggt tctgaggttg accaggccga gcctgtacac cttgatgtgt 1560
cggggaccta tgctgtccac gggcaccagc ttgaggccct ctacagggca ctcaacattc 1620
cacacgatat tgctgctcga gctgcccgat taacggctac tgttgagctc gctgcaggcc 1680
ccgaccgtct ggagtgccgc accgtgctcg ggaacaagac tttccggacg acggtgactg 1740
atggcgccca tctagaagcg aacggccctg agcaatatgt tctgtcgttc gatgcctccc 1800
gccagtctat gggggccggg tcccatagcc tcacttacga gcttacaccc gccggcttgc 1860
aggttaagat ctcatctaat ggcctagact gcactgccgt attcccccct ggtggcgccc 1920
ctagcgcgcc gccgggggag gtggcggctt tttgcagcgc cctctacagg tataacagat 1980
tcacccagcg gcattcgctg accggcggtt tgtggctaca tcccgagggg ttgctaggcg 2040
ttttcccccc tttttcccct gggcacattt gggagtctgc caaccccttc tgcggtgagg 2100
gtaccttgta tactcgcacc tggtctacat ctggtttttc tagtgacttt tcccctcctg 2160
aggcggccgc ccctgcaccg gctgccgccc cagggtcgtc ctcccccact ccaccagtca 2220
gtgatatttg ggtgttacca ccgctttcag aggagcccca ggtgggtgcg ccgcctgcac 2280
ctcccacccc cgagcctgct cggctgccct gccccactaa acctaacacc cccgtgcgta 2340
agccaacggc accgccgcct tctcgcaccc gccgccttct ttacacctat cctgatggtg 2400
ctaaggtgta tgcggggtca ctgtttgagt cagattgtga tt 2442

<210> 8

<211> 1703

<212> PRT

<213> Hepatitis E virus

<400> 8

Met Glu Ala His Gln Phe Ile Lys Ala Pro Gly Ile Thr Thr Ala Ile
1               5                   10                  15

Glu Gln Ala Ala Leu Ala Ala Ala Asn Ser Ala Leu Ala Asn Ala Val
            20                  25                  30

Val Val Arg Pro Phe Leu Ser Arg Val Gln Thr Glu Ile Leu Ile Asn
        35                  40                  45

Leu Met Gln Pro Arg Gln Leu Val Phe Arg Pro Glu Val Leu Trp Asn
    50                  55                  60

His Pro Ile Gln Arg Val Ile His Asn Glu Leu Glu Gln Tyr Cys Arg
65                  70                  75                  80

Ala Arg Ala Gly Arg Cys Leu Glu Ile Gly Ala His Pro Arg Ser Ile
                85                  90                  95

Asn Asp Asn Pro Asn Val Leu His Arg Cys Phe Leu Arg Pro Val Gly
            100                 105                 110

Arg Asp Val Gln Arg Trp Tyr Ser Ala Pro Thr Arg Gly Pro Ala Ala
        115                 120                 125

Asn Cys Arg Arg Ser Ala Leu Arg Gly Leu Pro Pro Val Asp Arg Thr
    130                 135                 140

Tyr Cys Phe Asp Gly Phe Ser Ser Cys Ala Phe Ala Ala Glu Thr Gly
145                 150                 155                 160

Val Ala Leu Tyr Ser Leu His Asp Leu Trp Pro Ala Asp Val Ala Glu
                165                 170                 175

Ala Met Ala Arg His Gly Met Thr Arg Leu Tyr Ala Ala Leu His Leu
            180                 185                 190

Pro Pro Glu Val Leu Leu Pro Pro Gly Thr Tyr His Thr Thr Ser Tyr
        195                 200                 205

Leu Leu Ile His Asp Gly Asn Arg Ala Val Val Thr Tyr Glu Gly Asp
    210                 215                 220

Thr Ser Ala Gly Tyr Asn His Asp Val Ser Ile Leu Arg Ala Trp Ile
225                 230                 235                 240

Arg Thr Thr Lys Ile Val Gly Asp His Pro Leu Val Ile Glu Arg Val
                245                 250                 255

Arg Ala Ile Gly Cys His Phe Val Leu Leu Leu Thr Ala Ala Pro Glu
            260                 265                 270

Pro Ser Pro Met Pro Tyr Val Pro Tyr Pro Arg Ser Thr Glu Val Tyr
        275                 280                 285

Val Arg Ser Ile Phe Gly Pro Gly Gly Ser Pro Ser Leu Phe Pro Ser
    290                 295                 300

Ala Cys Ser Thr Lys Ser Thr Phe His Ala Val Pro Val His Ile Trp
305                 310                 315                 320

Asp Arg Leu Met Leu Phe Gly Ala Thr Leu Asp Asp Gln Ala Phe Cys
                325                 330                 335

Cys Ser Arg Leu Met Thr Tyr Leu Arg Gly Ile Ser Tyr Lys Val Thr
            340                 345                 350

Val Gly Ala Leu Val Ala Asn Glu Gly Trp Asn Ala Ser Glu Asp Ala
        355                 360                 365

Leu Thr Ala Val Ile Thr Ala Ala Tyr Leu Thr Ile Cys His Gln Arg
    370                 375                 380

Tyr Leu Arg Thr Gln Ala Ile Ser Lys Gly Met Arg Arg Leu Glu Val
385                 390                 395                 400

Glu His Ala Gln Lys Phe Ile Thr Arg Leu Tyr Ser Trp Leu Phe Glu
                405                 410                 415

Lys Ser Gly Arg Asp Tyr Ile Pro Gly Arg Gln Leu Gln Phe Tyr Ala

            420                 425                 430

Gln Cys Arg Arg Trp Leu Ser Ala Gly Phe His Leu Asp Pro Arg Val
        435                 440                 445

Leu Val Phe Asp Glu Ser Val Pro Cys Arg Cys Arg Thr Phe Leu Lys
    450                 455                 460

Lys Val Ala Gly Lys Phe Cys Cys Phe Met Arg Trp Leu Gly Gln Glu
465                 470                 475                 480

Cys Thr Cys Phe Leu Glu Pro Ala Glu Gly Leu Val Gly Asp Gln Gly
                485                 490                 495

His Asp Asn Glu Ala Tyr Glu Gly Ser Glu Val Asp Gln Ala Glu Pro
            500                 505                 510

Val His Leu Asp Val Ser Gly Thr Tyr Ala Val His Gly His Gln Leu
        515                 520                 525

Glu Ala Leu Tyr Arg Ala Leu Asn Ile Pro His Asp Ile Ala Ala Arg
    530                 535                 540

Ala Ala Arg Leu Thr Ala Thr Val Glu Leu Ala Ala Gly Pro Asp Arg
545                 550                 555                 560

Leu Glu Cys Arg Thr Val Leu Gly Asn Lys Thr Phe Arg Thr Thr Val
                565                 570                 575

Thr Asp Gly Ala His Leu Glu Ala Asn Gly Pro Glu Gln Tyr Val Leu
            580                 585                 590

Ser Phe Asp Ala Ser Arg Gln Ser Met Gly Ala Gly Ser His Ser Leu
        595                 600                 605

Thr Tyr Glu Leu Thr Pro Ala Gly Leu Gln Val Lys Ile Ser Ser Asn
    610                 615                 620

Gly Leu Asp Cys Thr Ala Val Phe Pro Pro Gly Gly Ala Pro Ser Ala
625                 630                 635                 640

Pro Pro Gly Glu Val Ala Ala Phe Cys Ser Ala Leu Tyr Arg Tyr Asn
                645                 650                 655

Arg Phe Thr Gln Arg His Ser Leu Thr Gly Gly Leu Trp Leu His Pro
            660                 665                 670

Glu Gly Leu Leu Gly Val Phe Pro Pro Phe Ser Pro Gly His Ile Trp
        675                 680                 685

Glu Ser Ala Asn Pro Phe Cys Gly Glu Gly Thr Leu Tyr Thr Arg Thr
    690                 695                 700

Trp Ser Thr Ser Gly Phe Ser Ser Asp Phe Ser Pro Pro Glu Ala Ala
705                 710                 715                 720

Ala Pro Ala Pro Ala Ala Ala Pro Gly Ser Ser Ser Pro Thr Pro Pro
                725                 730                 735

Val Ser Asp Ile Trp Val Leu Pro Pro Leu Ser Glu Glu Pro Gln Val
            740                 745                 750

Gly Ala Pro Pro Ala Pro Pro Thr Pro Glu Pro Ala Arg Leu Pro Cys
        755                 760                 765

Pro Thr Lys Pro Asn Thr Pro Val Arg Lys Pro Thr Ala Pro Pro Pro
    770                 775                 780

Ser Arg Thr Arg Arg Leu Leu Tyr Thr Tyr Pro Asp Gly Ala Lys Val
785                 790                 795                 800

Tyr Ala Gly Ser Leu Phe Glu Ser Asp Cys Asp Trp Leu Val Asn Ala
                805                 810                 815

Ser Asn Pro Gly His Arg Pro Gly Gly Gly Leu Cys His Ala Phe Tyr
            820                 825                 830

Gln Arg Phe Pro Glu Ala Phe Tyr Pro Thr Glu Phe Ile Met Arg Glu
        835                 840                 845

Gly Leu Ala Ala Tyr Thr Leu Thr Pro Arg Pro Ile Ile His Ala Val
    850                 855                 860

Ala Pro Asp Tyr Arg Val Glu Gln Asn Pro Lys Arg Leu Glu Ala Ala
865                 870                 875                 880

Tyr Arg Glu Thr Cys Ser Arg Arg Gly Thr Ala Ala Tyr Pro Leu Leu

                885                 890                 895

Gly Ser Gly Ile Tyr Gln Val Pro Ala Gly Leu Ser Phe Asp Ala Trp
            900                 905                 910

Glu Arg Asn His Arg Pro Gly Asp Glu Leu Tyr Leu Thr Glu Pro Ala
        915                 920                 925

Ala Ala Trp Phe Glu Ala Asn Lys Pro Thr Gln Pro Ala Leu Thr Ile
    930                 935                 940

Thr Glu Asp Thr Ala Arg Thr Ala Asn Leu Ala Leu Glu Ile Asp Ala
945                 950                 955                 960

Ala Thr Asp Val Gly Arg Ala Cys Ala Gly Cys Thr Ile Ser Pro Gly
                965                 970                 975

Ile Val His Tyr Gln Phe Thr Ala Gly Val Pro Gly Ser Gly Lys Ser
            980                 985                 990

Arg Ser Ile Gln Gln Gly Asp Val Asp Val Val Val Val Pro Thr Arg
        995                 1000                1005

Glu Leu Arg Asn Ser Trp Arg Arg Arg Gly Phe Ala Ala Phe Thr Pro
    1010                1015                1020

His Thr Ala Ala Arg Val Thr Ile Gly Arg Arg Val Val Ile Asp Glu
1025                1030                1035                1040

Ala Pro Ser Leu Pro Pro His Leu Leu Leu Leu His Met Gln Arg Ala
                1045                1050                1055
Ser Ser Val His Leu Leu Gly Asp Pro Asn Gln Ile Pro Ala Ile Asp
            1060                1065                1070
Phe Glu His Ala Gly Leu Val Pro Ala Ile Arg Pro Glu Leu Ala Pro
        1075                1080                1085
Thr Ser Trp Trp His Val Thr His Arg Cys Pro Ala Asp Val Cys Glu
    1090                1095                1100
Leu Ile Arg Gly Ala Tyr Pro Lys Ile Gln Thr Thr Ser Arg Val Leu
1105                1110                1115                1120
Arg Ser Leu Phe Trp Asn Glu Pro Ala Ile Gly Gln Lys Leu Val Phe
                1125                1130                1135
Thr Gln Ala Ala Lys Ala Ala Asn Pro Gly Ala Ile Thr Val His Glu
            1140                1145                1150
Ala Gln Gly Ala Thr Phe Thr Glu Thr Thr Ile Ile Ala Thr Ala Asp
        1155                1160                1165
Ala Arg Gly Leu Ile Gln Ser Ser Arg Ala His Ala Ile Val Ala Leu
    1170                1175                1180
Thr Arg His Thr Glu Lys Cys Val Ile Leu Asp Ala Pro Gly Leu Leu
1185                1190                1195                1200

Arg Glu Val Gly Ile Ser Asp Val Ile Val Asn Asn Phe Phe Leu Ala
                1205                1210                1215

Gly Gly Glu Val Gly His His Arg Pro Ser Val Ile Pro Arg Gly Asn
            1220                1225                1230

Pro Asp Arg Asn Leu Asp Thr Leu Gln Ala Phe Pro Pro Ser Cys Gln
        1235                1240                1245

Ile Ser Ala Tyr His Gln Leu Ala Glu Glu Leu Gly His Arg Pro Ala
    1250                1255                1260

Pro Val Ala Ala Val Leu Pro Pro Cys Pro Glu Leu Glu Gln Gly Leu
1265                1270                1275                1280

Leu Tyr Met Pro Gln Glu Leu Thr Val Ser Asp Ser Val Leu Val Phe
                1285                1290                1295

Glu Leu Thr Asp Ile Val His Cys Arg Met Ala Ala Pro Ser Gln Arg
            1300                1305                1310

Lys Ala Val Leu Ser Thr Leu Val Gly Arg Tyr Gly Arg Lys Thr Lys
        1315                1320                1325

Leu Tyr Glu Ala Ala His Ser Asp Val Arg Glu Ser Leu Ala Arg Phe
    1330                1335                1340

Ile Pro Thr Ile Gly Pro Val Gln Ala Thr Thr Cys Glu Leu Tyr Glu

1345                1350                1355                1360

Leu Val Glu Ala Met Val Glu Lys Gly Gln Asp Gly Ser Ala Val Leu
                1365                1370                1375

Glu Leu Asp Leu Cys Asn Arg Asp Val Ser Arg Ile Thr Phe Phe Gln
            1380                1385                1390

Lys Asp Cys Asn Lys Phe Thr Thr Gly Glu Thr Ile Ala His Gly Lys
        1395                1400                1405

Val Gly Gln Gly Ile Ser Ala Trp Ser Lys Thr Phe Cys Ala Leu Phe
    1410                1415                1420

Gly Pro Trp Phe Arg Ala Ile Glu Lys Glu Ile Leu Ala Leu Leu Pro
1425                1430                1435                1440

Pro Asn Ile Phe Tyr Gly Asp Ala Tyr Glu Glu Ser Val Phe Ala Ala
                1445                1450                1455

Ala Val Ser Gly Ala Gly Ser Cys Met Val Phe Glu Asn Asp Phe Ser
            1460                1465                1470

Glu Phe Asp Ser Thr Gln Asn Asn Phe Ser Leu Gly Leu Glu Cys Val
        1475                1480                1485

Val Met Glu Glu Cys Gly Met Pro Gln Trp Leu Ile Arg Leu Tyr His
    1490                1495                1500

Leu Val Arg Ser Ala Trp Ile Leu Gln Ala Pro Lys Glu Ser Leu Lys
1505                1510                1515                1520

Gly Phe Trp Lys Lys His Ser Gly Glu Pro Gly Thr Leu Leu Trp Asn
                1525                1530                1535

Thr Ile Trp Asn Met Ala Ile Ile Ala His Cys Tyr Glu Phe Arg Asp
            1540                1545                1550

Phe Arg Val Ala Ala Phe Lys Gly Asp Asp Ser Val Val Leu Cys Ser
        1555                1560                1565

Asp Tyr Arg Gln Ser Arg Asn Ala Ala Ala Leu Ile Ala Gly Cys Gly
    1570                1575                1580

Leu Lys Leu Lys Val Asp Tyr Arg Pro Ile Gly Leu Tyr Ala Gly Val
1585                1590                1595                1600

Val Val Ala Pro Gly Leu Gly Thr Leu Pro Asp Val Val Arg Phe Ala
                1605                1610                1615

Gly Arg Leu Ser Glu Lys Asn Trp Gly Pro Gly Pro Glu Arg Ala Glu
            1620                1625                1630

Gln Leu Arg Leu Ala Val Cys Asp Phe Leu Arg Gly Leu Thr Asn Val
        1635                1640                1645

Ala Gln Val Cys Val Asp Val Val Ser Arg Val Tyr Gly Val Ser Pro
    1650                1655                1660

Gly Leu Val His Asn Leu Ile Gly Met Leu Gln Thr Ile Ala Asp Gly
1665                1670                1675                1680

Lys Ala His Phe Thr Glu Ser Ile Lys Pro Val Leu Asp Leu Thr Asn
                1685                1690                1695

Ser Ile Ile Gln Arg Val Glu
            1700

<210> 9
<211> 19
<212> DNA
<213> Artificial Sequence

<220>
<223> Description of Artificial Sequence: Hepatitis E virus

<400> 9
ttctttctgg ccccagact                                                19

<210> 10
<211> 19
<212> DNA
<213> Artificial Sequence

<220>
<223> Description of Artificial Sequence: Hepatitis E virus

<400> 10

ttctttctga ccccagact                                                19

<210> 11

<211> 7234

<212> RNA

<213> Hepatitis E virus JSN-FH

<400> 11

gtcgacgcca tggaggccca tcagttcata aaggctcctg gcgtcacaac tgctattgag 60

caggcagctc tagcagcggc caactccgcc ttggcgaatg ctgtggtggt tcggcctttt 120

ctgtcccggc tacagacaga gatacttatt aacttgatgc agccccggca gcttgttttc 180

cggcctgagg tcctgtggaa tcaccccatt caacgcgtga tccacaatga gcttgaacag 240

tactgtcggg ctcgggccgg ccgctgtctt gaagtgggcg ctcacccacg ttcgatcaac 300

gacaacccca acgtcttgca tcgctgtttt ttaaaaccag ttggccgcga tgtccagcgg 360

tggtacaccg ctcctacccg cggccctgca gcgaactgcc gccggtcagc ccttcgtggc 420

ctcccgcctg ttgaccggac atattgcttt gacggttttt ccggttgcac atttgccgcc 480

gagacgggga ttgccctcta ctcactgcat gacctttggc ctgccgatgt tgctgaggcg 540

atggcccgcc acggtatgac ccggctgtat gcggctctcc acctccctcc tgaggtgctg 600

ctcccccctg gtacctatca taccacctcg tacctactca tccatgatgg ggaccgtgca 660

gtgattacat atgaggggga ttctagtgct gggtacaacc atgatgtgtc catcttgcgc 720

gcctggatcc gcactactaa ggttactggt gaccacccgc tggtgattga gcgagttcgg 780

gcggtgggtt gtcattttgt gctccttcta acagctgcac ctgaaccatc gccaatgccc 840

tatgttccat aycctcgttc cactgaggtt tacgttcggt ccatttttgg ccctggtggt 900

tcaccatccc tgtttccaac tgcctgctct actaagtcaa catttcatgc tgtccctgtg 960

catatatggg acagacttat gttattcggt gcgaccctcg acgatcaggc cttttgttgc 1020

tcgaggctaa tgacatacct ccgtggcatt agctacaaag tcacggtcgg cgctcttgtt 1080

gccaacgagg gctggaatgc ctccgaggat gcattgaccg ctgtgattac tgcggcctat 1140

cttaccattt gccatcagag gtaccttcgc acgcaggcca tctcgaaagg gatgaaaaga 1200
ctggagcttg agcacgcaca gaagttcata acacgtcttt acagctggtt gtttgaaaaa 1260
tccggccgtg attacattcc cggccgtcag ctgcagtttt atgcccagtg ccgccgatgg 1320
ctttccgctg gtttccatct tgatcctcgt gtgcttgtct ttgatgaagc ggccccttgt 1380
cgctgccgta gtttccttcg taaagctgct catagatttt gttgttttat gcggtggctg 1440
ggccaggatt gtacctgttt cctccagccc gttgagggga gggttggcga gcagggttac 1500
gataatgagg catttgaggg gtcggatgtt gaccccgctg aggaggccac tgtgaacatc 1560
tctggatcat atattgtcac cggtagccag ctacagcctc tctaccaggc gcttggcatc 1620
ccttctgatc ttgccgcccg agcgggtcga cttactgcca ctgttgaagt cctagatgct 1680
gatggccgcc tcacttgtaa gaccactatt ggcaataaga cctttacaac agtttttact 1740
gatggtgctc agctagaggc taacgggccg gagcagtatg tgctgtcgtt tgactcggcc 1800
aagcaaacta tggccgccgg cccgcacagt cttagctatg ctttgacatc tgcgggcctt 1860
gaggtgcgcg ttgtctctgc cgggcttgac tgtaaggctg tatttccatc cggggttgca 1920
acccccacca cccctggtga ggtctccgct ttttgttcag ctttgtacag gttcaaccgt 1980
tgtgttcagc ggcactccct tattgggggt ttgtggtatt accctgaggg gctaattggc 2040
ttgtttccgc catttgctcc cggccatacc tgggagtcag ccaatccctt ctgtggagag 2100
agcaccctct atactcgcac ctggtcggtg tcgggcttct ctagttgttt ttctccaccc 2160
gagccacccg ctccagaact atcatctcct gttgaggttg acacacctgt ggctgttggc 2220
gttccatccc cagccacttc ggcagtaccc caaccttcag tccctgaaca ggccgcgcca 2280
ttgccggatt cggttgacgg cggtgctgct ccggccccat tgagcacctc tactacaccc 2340
ccggtgccag cgcagcacgt aacccaccca tctggctccc gtcggcggtt acttcacacc 2400
taccctgatg gctcaaaagt gtatgctggc tccctttttg aatctgagtg tacttggtta 2460
gttaatgcat ccaatcctgg tcaccgcccc ggtggcggcc tttgccacgc attttaccaa 2520
cggttcccgg agtcatttga ccctgctgag tttattatgt ctgacgggtt tgcggcctat 2580
accttgacac cccgacccat tatccatgct gttgcccctg attatcgggt ggaacataac 2640
cctaagaggc tcgaggctgc ctatcgagag acgtgttccc gtcgtgggac ggctgcttat 2700
cccttgcttg gcgccggtat atacagggtg cccgttgggc tgagctttga tgcttgggag 2760
cgtaaccacc gacccgggga tgagttgtac ctgactgagc cggctatagc ttggttcgag 2820
accaaccgac ctactctccc cgcgcttact attactgagg acacagcacg aacagcgaac 2880

ctggctcttg agttagattc agccactgag gtcggcaggg cgtgtgccgg ctgtcgtgtc 2940
gagccgggtg ttgtccatta ccagttcaca gcaggtgtcc ctggctccgg caagtcacgg 3000
tcaattcagc agggcgaggt agacgtcgta gtggtgccaa cccgtgagtt gcgtaactcg 3060
tggcggcgcc gcgggtttgc agcttataca ccacacaccg cggcccgtgt tacctgtggc 3120
cgtagggttg ttattgacga ggccccgtcg cttccaccac atttactttt gctgcatatg 3180
cagcgagcct cgtcggttca tctccttggc gaccccaacc aaatccctgc tatcgatttt 3240
gagcatgctg gcctcgttcc ggcgatccgg cctgagcttg ttccaacaaa gtggtggcac 3300
cttactcata ggtgtcctgc ggatgtttgt gagctaatcc gcggcgcgta cccaaagatc 3360
cagacggcaa gccgagttct ccgctccctg ttctgggggg agccccccgt tggccaaaaa 3420
ctagtgttta ctcaggcggc gaaggccgcc aaccccggtg caattacagt ccacgaggcc 3480
cagggtgcta catttactga gactactatt attgcaacgg ctgatgcccg cgggctgatt 3540
cagtcctcca gggcccacgc tattgtggcc ctgactcgcc acacagagaa atgtgtggtc 3600
gttgacgccc cggggcttct tcgtgaggtt ggcatttcag atgccattgt taataacttt 3660
tttctttctg gcggccaggt tggtcagcac cgcccatcag tcatacctcg tggcactgtt 3720
gatagcaatg ttgccacgct tgatgcattt ccaccttctt gtcaatttag cgcctatcac 3780
cagcttgcag aggagcttgg tcatcggccg gccccgattg ccgccgttct gcccccttgt 3840
ccggagctcg agcaaggctt actctatatg ccccaggaac tgaccacgtc cgatagtgtg 3900
ctcacattcg aacttacaga cattgtacac tgtcgtatgg cggcacctag tcagcgcaaa 3960
gcagtcctgt cgacccttgt cggcaggtac ggccgccgta cgaagttgta tgaagctgct 4020
cacacggatg ttcgtgggtc cctctgtcat tttctccccg agcttggccc catcagcgtt 4080
actacctgcg agctttatga gcttgttgag gctatggtgg agaagggtca ggatggctct 4140
gcagtcttgg aactcgatct atgcagtcgt gatgtgtcac gtataacatt cttccagaag 4200
gactgtaata aattcacaac aggcgagaca atagcgcacg gtaaagttgg gcaggggata 4260
tctgcgtgga gcaagacctt ttgtgccctg tttggcccat ggttccgcgc tattgagaag 4320
gagattcttg cggcgcttgc ccctaatgta ttttatggtg atgcatatga agatacagtt 4380
ttggccgccg ctgtcgccgg ggccccaggc tgtaaggttt ttgagaatga tttctctgag 4440
tttgacagca cccaaaataa cttttcactc ggactggagt gcataatcat ggaggaatgt 4500
ggcatgccgc agtggatgat ccgactttat catcttgttc gctccgcctg ggtcctgcag 4560
gctcccaagg agtccctgcg agggttctgg aagaagcact cgggcgagcc tggcactctg 4620

ctctggaaca ctgtctggaa catggcggtt attgcgcatt gttatgaatt ccgtgaccta 4680
aaggtcgcgg catttaaagg agatgactca gttgtgctct gtagtgacta ccggcagagc 4740
cgcaacgctg ctgctctaat cgccggttgc gggttgaaac ttaaggtgga ttttaggcct 4800
attgggctat atgctggtgt tgttgtggcc ccgggcctcg ggaccctccc tgatgtcgtt 4860
aggtttgctg ggcggctctc agagaaaaac tgggggccgg gttcagagag ggcggaacag 4920
ctacggctgg ccgtttgtga ttttctgcga aagttaacga atgtggctca ggtctgtgtg 4980
gatgttgttt cgcaggttta tggtgtcagc cccggtttag tacataacct gattggaatg 5040
ctccagacca ttgctgatgg taaggcgcat tttactgaaa cagtcaaacc cgtccttgat 5100
cttactagct ctattatata ccgggtggaa tgaataacat gttcttttgc tctgtgcatg 5160
gagatgccac catgcgctct cgggctcttc tgtttctgct cttcgtgctt ctgcctatgc 5220
tgcccgcgcc accggccggt cagccgtctg gccgtcgccg cgggcggcgc agcggcggtg 5280
ccggcggtgg tttctggggt gaccgggttg attctcagcc cttcgccctc ccctatattc 5340
atccaaccaa ccccttcgca tctgacatac caaccgcagc cgggtctgga gctcgccctc 5400
ggcagccggc ccgtccactc ggctccgctt ggcgtgacca gtcccagcgc cccgccgctc 5460
ccgcccgccg tcgatctgcc ccagctgggg cttcgccgct gactgctgtg gccccggccc 5520
ctgataccgc ccctgtgccc gacgttgact cccgcggcgc tatactacgc cgccagtata 5580
atctatcaac atccccgctt acgtctacca tcgctactgg caccaatctt gttttatatg 5640
ctgctcccct tagccctctg cttccgcttc aagatgggac taacactcat attatggcca 5700
ctgaagcatc aaattacgcc cagtaccgcg ttgtccgtgc caccatccgg tatcgcccac 5760
ttgtgccgaa tgctgttggc gggtacgcta tatctatttc tttctggccc cagactacaa 5820
ctaccccaac atctgtcgat atgaattcta ttacctctac tgatgttcgg attcttgttc 5880
agcctggtat cgcctccgag cttgtgattc ccagtgagcg cctgcactac cgcaatcagg 5940
gctggcgctc ggttgagacc tctggtgtcg cggaggagga ggcgacctcc ggccttgtta 6000
tgctctgcat ccatggatca cctgtgaact cttacactaa tacaccttac actggtgctc 6060
ttggcttact cgatttcgca cttgaacttg agttccgtaa tttgacaccc ggtaacacga 6120
atacgcgcgt ttcccgttat tcgagtagcg cgcgccacaa gctacgtcgt gggcccgacg 6180
gtactgctga gctaaccacc actgctgcta cacggtttat gaaagatctt cacttcacag 6240
ggaccaatgg tgttggtgag gtcggtcgcg gtatagcgct aactctgttt aatcttgctg 6300
acacgcttct cggcgggctc ccgacagaat tgatttcgtc ggctggtggc cagctatttt 6360

actctcgccc cgtcgtctca gccaatggcg agccgacagt gaagctttac acttcagtcg 6420
agaacgccca gcaggataag ggtatagcta ttccacatga tatcgacctt ggtgagtctc 6480
gagttgtcat ccaggattac gataaccagc acgagcagga ccgtcccacc ccgtctccag 6540
ccccttcccg ccctttttct gtcctccgcg ctaatgatgt gctttggctc tcacttacag 6600
ctgctgagta tgatcagact acttatggtt cttccactaa tcctatgtat gtctctgata 6660
ctgtgacatt tgtcaatgtg gcgactggtg ctcagggggt ctctcgctct ctggactggt 6720
ctaaagtcac ccttgatggg cgtccactta ctactatcca gcagtactct aagaccttct 6780
ttgttctgcc cctccgtggc aagctctctt tctgggaggc tggcaccact aaggccggct 6840
acccttataa ttataatacc actgccagtg accagatctt gattgaaaat gcagccggtc 6900
accgtgtttg tatctcaacc tacactacta atcttggctc tggccctgtt tctatttctt 6960
ctgtcggtgt ccttgcacct cattctgcgc tggccgcttt agaggacact gttgattatc 7020
ctgctcgcgc ccacaccttt gatgatttct gccctgagtg ccgtacgctc ggccttcagg 7080
gctgtgcttt tcaatcaact gttgctgagc tgcagcgtct taaaatgaag gtgggtaaaa 7140
cccgggagta ttgatttatt gtgcttgtac cttccttctg atttgtttct tttatttcct 7200
tcttctgcgt ttcgcgctcc ctggaaaaaa aaaa 7234

<210> 12
<211> 1707
<212> PRT
<213> Hepatitis E virus JSN-FH ORF1

<400> 12
Met Glu Ala His Gln Phe Ile Lys Ala Pro Gly Val Thr Thr Ala Ile
1               5                   10                  15

Glu Gln Ala Ala Leu Ala Ala Ala Asn Ser Ala Leu Ala Asn Ala Val
            20                  25                  30

Val Val Arg Pro Phe Leu Ser Arg Leu Gln Thr Glu Ile Leu Ile Asn

        35                  40                  45

Leu Met Gln Pro Arg Gln Leu Val Phe Arg Pro Glu Val Leu Trp Asn
    50                  55                  60

His Pro Ile Gln Arg Val Ile His Asn Glu Leu Glu Gln Tyr Cys Arg
65                  70                  75                  80

Ala Arg Ala Gly Arg Cys Leu Glu Val Gly Ala His Pro Arg Ser Ile
                85                  90                  95

Asn Asp Asn Pro Asn Val Leu His Arg Cys Phe Leu Lys Pro Val Gly
            100                 105                 110

Arg Asp Val Gln Arg Trp Tyr Thr Ala Pro Thr Arg Gly Pro Ala Ala
        115                 120                 125

Asn Cys Arg Arg Ser Ala Leu Arg Gly Leu Pro Pro Val Asp Arg Thr
    130                 135                 140

Tyr Cys Phe Asp Gly Phe Ser Gly Cys Thr Phe Ala Ala Glu Thr Gly
145                 150                 155                 160

Ile Ala Leu Tyr Ser Leu His Asp Leu Trp Pro Ala Asp Val Ala Glu
                165                 170                 175

Ala Met Ala Arg His Gly Met Thr Arg Leu Tyr Ala Ala Leu His Leu
            180                 185                 190

Pro Pro Glu Val Leu Leu Pro Pro Gly Thr Tyr His Thr Thr Ser Tyr
        195                 200                 205
Leu Leu Ile His Asp Gly Asp Arg Ala Val Ile Thr Tyr Glu Gly Asp
    210                 215                 220
Ser Ser Ala Gly Tyr Asn His Asp Val Ser Ile Leu Arg Ala Trp Ile
225                 230                 235                 240
Arg Thr Thr Lys Val Thr Gly Asp His Pro Leu Val Ile Glu Arg Val
                245                 250                 255
Arg Ala Val Gly Cys His Phe Val Leu Leu Leu Thr Ala Ala Pro Glu
            260                 265                 270
Pro Ser Pro Met Pro Tyr Val Pro Xaa Pro Arg Ser Thr Glu Val Tyr
        275                 280                 285
Val Arg Ser Ile Phe Gly Pro Gly Gly Ser Pro Ser Leu Phe Pro Thr
    290                 295                 300
Ala Cys Ser Thr Lys Ser Thr Phe His Ala Val Pro Val His Ile Trp
305                 310                 315                 320
Asp Arg Leu Met Leu Phe Gly Ala Thr Leu Asp Asp Gln Ala Phe Cys
                325                 330                 335
Cys Ser Arg Leu Met Thr Tyr Leu Arg Gly Ile Ser Tyr Lys Val Thr
            340                 345                 350

Val Gly Ala Leu Val Ala Asn Glu Gly Trp Asn Ala Ser Glu Asp Ala
        355                 360                 365

Leu Thr Ala Val Ile Thr Ala Ala Tyr Leu Thr Ile Cys His Gln Arg
    370                 375                 380

Tyr Leu Arg Thr Gln Ala Ile Ser Lys Gly Met Lys Arg Leu Glu Leu
385                 390                 395                 400

Glu His Ala Gln Lys Phe Ile Thr Arg Leu Tyr Ser Trp Leu Phe Glu
                405                 410                 415

Lys Ser Gly Arg Asp Tyr Ile Pro Gly Arg Gln Leu Gln Phe Tyr Ala
            420                 425                 430

Gln Cys Arg Arg Trp Leu Ser Ala Gly Phe His Leu Asp Pro Arg Val
        435                 440                 445

Leu Val Phe Asp Glu Ala Ala Pro Cys Arg Cys Arg Ser Phe Leu Arg
    450                 455                 460

Lys Ala Ala His Arg Phe Cys Cys Phe Met Arg Trp Leu Gly Gln Asp
465                 470                 475                 480

Cys Thr Cys Phe Leu Gln Pro Val Glu Gly Arg Val Gly Glu Gln Gly
                485                 490                 495

Tyr Asp Asn Glu Ala Phe Glu Gly Ser Asp Val Asp Pro Ala Glu Glu

            500                 505                 510

Ala Thr Val Asn Ile Ser Gly Ser Tyr Ile Val Thr Gly Ser Gln Leu
        515                 520                 525

Gln Pro Leu Tyr Gln Ala Leu Gly Ile Pro Ser Asp Leu Ala Ala Arg
    530                 535                 540

Ala Gly Arg Leu Thr Ala Thr Val Glu Val Leu Asp Ala Asp Gly Arg
545                 550                 555                 560

Leu Thr Cys Lys Thr Thr Ile Gly Asn Lys Thr Phe Thr Thr Val Phe
                565                 570                 575

Thr Asp Gly Ala Gln Leu Glu Ala Asn Gly Pro Glu Gln Tyr Val Leu
            580                 585                 590

Ser Phe Asp Ser Ala Lys Gln Thr Met Ala Ala Gly Pro His Ser Leu
        595                 600                 605

Ser Tyr Ala Leu Thr Ser Ala Gly Leu Glu Val Arg Val Val Ser Ala
    610                 615                 620

Gly Leu Asp Cys Lys Ala Val Phe Pro Ser Gly Val Ala Thr Pro Thr
625                 630                 635                 640

Thr Pro Gly Glu Val Ser Ala Phe Cys Ser Ala Leu Tyr Arg Phe Asn
                645                 650                 655

Arg Cys Val Gln Arg His Ser Leu Ile Gly Gly Leu Trp Tyr Tyr Pro
            660                 665                 670
Glu Gly Leu Ile Gly Leu Phe Pro Pro Phe Ala Pro Gly His Thr Trp
        675                 680                 685
Glu Ser Ala Asn Pro Phe Cys Gly Glu Ser Thr Leu Tyr Thr Arg Thr
    690                 695                 700
Trp Ser Val Ser Gly Phe Ser Ser Cys Phe Ser Pro Pro Glu Pro Pro
705                 710                 715                 720
Ala Pro Glu Leu Ser Ser Pro Val Glu Val Asp Thr Pro Val Ala Val
                725                 730                 735
Gly Val Pro Ser Pro Ala Thr Ser Ala Val Pro Gln Pro Ser Val Pro
            740                 745                 750
Glu Gln Ala Ala Pro Leu Pro Asp Ser Val Asp Gly Gly Ala Ala Pro
        755                 760                 765
Ala Pro Leu Ser Thr Ser Thr Thr Pro Pro Val Pro Ala Gln His Val
    770                 775                 780
Thr His Pro Ser Gly Ser Arg Arg Arg Leu Leu His Thr Tyr Pro Asp
785                 790                 795                 800
Gly Ser Lys Val Tyr Ala Gly Ser Leu Phe Glu Ser Glu Cys Thr Trp
                805                 810                 815

Leu Val Asn Ala Ser Asn Pro Gly His Arg Pro Gly Gly Gly Leu Cys
            820                 825                 830
His Ala Phe Tyr Gln Arg Phe Pro Glu Ser Phe Asp Pro Ala Glu Phe
        835                 840                 845
Ile Met Ser Asp Gly Phe Ala Ala Tyr Thr Leu Thr Pro Arg Pro Ile
    850                 855                 860
Ile His Ala Val Ala Pro Asp Tyr Arg Val Glu His Asn Pro Lys Arg
865                 870                 875                 880
Leu Glu Ala Ala Tyr Arg Glu Thr Cys Ser Arg Arg Gly Thr Ala Ala
                885                 890                 895
Tyr Pro Leu Leu Gly Ala Gly Ile Tyr Arg Val Pro Val Gly Leu Ser
            900                 905                 910
Phe Asp Ala Trp Glu Arg Asn His Arg Pro Gly Asp Glu Leu Tyr Leu
        915                 920                 925
Thr Glu Pro Ala Ile Ala Trp Phe Glu Thr Asn Arg Pro Thr Leu Pro
    930                 935                 940
Ala Leu Thr Ile Thr Glu Asp Thr Ala Arg Thr Ala Asn Leu Ala Leu
945                 950                 955                 960
Glu Leu Asp Ser Ala Thr Glu Val Gly Arg Ala Cys Ala Gly Cys Arg

                965                 970                 975

Val Glu Pro Gly Val Val His Tyr Gln Phe Thr Ala Gly Val Pro Gly
            980                 985                 990

Ser Gly Lys Ser Arg Ser Ile Gln Gln Gly Glu Val Asp Val Val Val
        995                 1000                1005

Val Pro Thr Arg Glu Leu Arg Asn Ser Trp Arg Arg Arg Gly Phe Ala
    1010                1015                1020

Ala Tyr Thr Pro His Thr Ala Ala Arg Val Thr Cys Gly Arg Arg Val
1025                1030                1035                1040

Val Ile Asp Glu Ala Pro Ser Leu Pro Pro His Leu Leu Leu Leu His
                1045                1050                1055

Met Gln Arg Ala Ser Ser Val His Leu Leu Gly Asp Pro Asn Gln Ile
            1060                1065                1070

Pro Ala Ile Asp Phe Glu His Ala Gly Leu Val Pro Ala Ile Arg Pro
        1075                1080                1085

Glu Leu Val Pro Thr Lys Trp Trp His Leu Thr His Arg Cys Pro Ala
    1090                1095                1100

Asp Val Cys Glu Leu Ile Arg Gly Ala Tyr Pro Lys Ile Gln Thr Ala
1105                1110                1115                1120

Ser Arg Val Leu Arg Ser Leu Phe Trp Gly Glu Pro Pro Val Gly Gln
                1125                1130                1135

Lys Leu Val Phe Thr Gln Ala Ala Lys Ala Ala Asn Pro Gly Ala Ile
            1140                1145                1150

Thr Val His Glu Ala Gln Gly Ala Thr Phe Thr Glu Thr Thr Ile Ile
        1155                1160                1165

Ala Thr Ala Asp Ala Arg Gly Leu Ile Gln Ser Ser Arg Ala His Ala
    1170                1175                1180

Ile Val Ala Leu Thr Arg His Thr Glu Lys Cys Val Val Val Asp Ala
1185                1190                1195                1200

Pro Gly Leu Leu Arg Glu Val Gly Ile Ser Asp Ala Ile Val Asn Asn
                1205                1210                1215

Phe Phe Leu Ser Gly Gly Gln Val Gly Gln His Arg Pro Ser Val Ile
            1220                1225                1230

Pro Arg Gly Thr Val Asp Ser Asn Val Ala Thr Leu Asp Ala Phe Pro
        1235                1240                1245

Pro Ser Cys Gln Phe Ser Ala Tyr His Gln Leu Ala Glu Glu Leu Gly
    1250                1255                1260

His Arg Pro Ala Pro Ile Ala Ala Val Leu Pro Pro Cys Pro Glu Leu
1265                1270                1275                1280

Glu Gln Gly Leu Leu Tyr Met Pro Gln Glu Leu Thr Thr Ser Asp Ser
                1285                1290                1295
Val Leu Thr Phe Glu Leu Thr Asp Ile Val His Cys Arg Met Ala Ala
            1300                1305                1310
Pro Ser Gln Arg Lys Ala Val Leu Ser Thr Leu Val Gly Arg Tyr Gly
        1315                1320                1325
Arg Arg Thr Lys Leu Tyr Glu Ala Ala His Thr Asp Val Arg Gly Ser
    1330                1335                1340
Leu Cys His Phe Leu Pro Glu Leu Gly Pro Ile Ser Val Thr Thr Cys
1345                1350                1355                1360
Glu Leu Tyr Glu Leu Val Glu Ala Met Val Glu Lys Gly Gln Asp Gly
                1365                1370                1375
Ser Ala Val Leu Glu Leu Asp Leu Cys Ser Arg Asp Val Ser Arg Ile
            1380                1385                1390
Thr Phe Phe Gln Lys Asp Cys Asn Lys Phe Thr Thr Gly Glu Thr Ile
        1395                1400                1405
Ala His Gly Lys Val Gly Gln Gly Ile Ser Ala Trp Ser Lys Thr Phe
    1410                1415                1420
Cys Ala Leu Phe Gly Pro Trp Phe Arg Ala Ile Glu Lys Glu Ile Leu

1425                1430                1435                1440

Ala Ala Leu Ala Pro Asn Val Phe Tyr Gly Asp Ala Tyr Glu Asp Thr
                1445                1450                1455

Val Leu Ala Ala Ala Val Ala Gly Ala Pro Gly Cys Lys Val Phe Glu
            1460                1465                1470

Asn Asp Phe Ser Glu Phe Asp Ser Thr Gln Asn Asn Phe Ser Leu Gly
        1475                1480                1485

Leu Glu Cys Ile Ile Met Glu Glu Cys Gly Met Pro Gln Trp Met Ile
    1490                1495                1500

Arg Leu Tyr His Leu Val Arg Ser Ala Trp Val Leu Gln Ala Pro Lys
1505                1510                1515                1520

Glu Ser Leu Arg Gly Phe Trp Lys Lys His Ser Gly Glu Pro Gly Thr
                1525                1530                1535

Leu Leu Trp Asn Thr Val Trp Asn Met Ala Val Ile Ala His Cys Tyr
            1540                1545                1550

Glu Phe Arg Asp Leu Lys Val Ala Ala Phe Lys Gly Asp Asp Ser Val
        1555                1560                1565

Val Leu Cys Ser Asp Tyr Arg Gln Ser Arg Asn Ala Ala Ala Leu Ile
    1570                1575                1580

Ala Gly Cys Gly Leu Lys Leu Lys Val Asp Phe Arg Pro Ile Gly Leu
1585                1590                1595                1600
Tyr Ala Gly Val Val Val Ala Pro Gly Leu Gly Thr Leu Pro Asp Val
                1605                1610                1615
Val Arg Phe Ala Gly Arg Leu Ser Glu Lys Asn Trp Gly Pro Gly Ser
            1620                1625                1630
Glu Arg Ala Glu Gln Leu Arg Leu Ala Val Cys Asp Phe Leu Arg Lys
        1635                1640                1645
Leu Thr Asn Val Ala Gln Val Cys Val Asp Val Val Ser Gln Val Tyr
    1650                1655                1660
Gly Val Ser Pro Gly Leu Val His Asn Leu Ile Gly Met Leu Gln Thr
1665                1670                1675                1680
Ile Ala Asp Gly Lys Ala His Phe Thr Glu Thr Val Lys Pro Val Leu
                1685                1690                1695
Asp Leu Thr Ser Ser Ile Ile Tyr Arg Val Glu
            1700                1705
<210> 13
<211> 211
<212> PRT
<213> Hepatitis E virus JSN-FH ORF2(1-211)

<400> 13

Met Arg Ser Arg Ala Leu Leu Phe Leu Leu Phe Val Leu Leu Pro Met
1               5                   10                  15

Leu Pro Ala Pro Pro Ala Gly Gln Pro Ser Gly Arg Arg Arg Gly Arg
            20                  25                  30

Arg Ser Gly Gly Ala Gly Gly Gly Phe Trp Gly Asp Arg Val Asp Ser
        35                  40                  45

Gln Pro Phe Ala Leu Pro Tyr Ile His Pro Thr Asn Pro Phe Ala Ser
    50                  55                  60

Asp Ile Pro Thr Ala Ala Gly Ser Gly Ala Arg Pro Arg Gln Pro Ala
65                  70                  75                  80

Arg Pro Leu Gly Ser Ala Trp Arg Asp Gln Ser Gln Arg Pro Ala Ala
                85                  90                  95

Pro Ala Arg Arg Arg Ser Ala Pro Ala Gly Ala Ser Pro Leu Thr Ala
            100                 105                 110

Val Ala Pro Ala Pro Asp Thr Ala Pro Val Pro Asp Val Asp Ser Arg
        115                 120                 125

Gly Ala Ile Leu Arg Arg Gln Tyr Asn Leu Ser Thr Ser Pro Leu Thr
    130                 135                 140

Ser Thr Ile Ala Thr Gly Thr Asn Leu Val Leu Tyr Ala Ala Pro Leu
145                 150                 155                 160

Ser Pro Leu Leu Pro Leu Gln Asp Gly Thr Asn Thr His Ile Met Ala
                165                 170                 175

Thr Glu Ala Ser Asn Tyr Ala Gln Tyr Arg Val Val Arg Ala Thr Ile
            180                 185                 190

Arg Tyr Arg Pro Leu Val Pro Asn Ala Val Gly Gly Tyr Ala Ile Ser
        195                 200                 205

Ile Ser Phe
    210

<210> 14
<211> 2582
<212> RNA
<213> Hepatitis E virus Mexico (M74506)

<400> 14
gccatggagg cccaccagtt cattaaggct cctggcatca ctactgctat tgagcaagca 60
gctctagcag cggccaactc cgcccttgcg aatgctgtgg tggtccggcc tttcctttcc 120
catcagcagg ttgagatcct tataaatctc atgcaacctc ggcagctggt gtttcgtcct 180
gaggtttttt ggaatcaccc gattcaacgt gttatacata atgagcttga gcagtattgc 240
cgtgctcgct cgggtcgctg ccttgagatt ggagcccacc cacgctccat taatgataat 300
cctaatgtcc tccatcgctg ctttctccac cccgtcggcc gggatgttca gcgctggtac 360
acagccccga ctaggggacc tgcggcgaac tgtcgccgct cggcacttcg tggtctgcca 420

ccagccgacc gcacttactg ttttgatggc tttgccggct gccgttttgc cgccgagact 480
ggtgtggctc tctattctct ccatgacttg cagccggctg atgttgccga ggcgatggct 540
cgccacggca tgacccgcct ttatgcagct ttccacttgc ctccagaggt gctcctgcct 600
cctggcacct accggacatc atcctacttg ctgatccacg atggtaagcg cgcggttgtc 660
acttatgagg gtgacactag cgccggttac aatcatgatg ttgccaccct ccgcacatgg 720
atcaggacaa ctaaggttgt gggtgaacac cctttggtga tcgagcgggt gcggggtatt 780
ggctgtcact ttgtgttgtt gatcactgcg gcccctgagc cctccccgat gccctacgtt 840
ccttacccgc gttcgacgga ggtctatgtc cggtctatct ttgggcccgg cgggtccccg 900
tcgctgttcc cgaccgcttg tgctgtcaag tccacttttc acgccgtccc cacgcacatc 960
tgggaccgtc tcatgctctt tggggccacc ctcgacgacc aggccttttg ctgctccagg 1020
cttatgacgt accttcgtgg cattagctat aaggtaactg tgggtgccct ggtcgctaat 1080
gaaggctgga atgccaccga ggatgcgctc actgcagtta ttacggcggc ttacctcaca 1140
atatgtcatc agcgttattt gcggacccag gcgatttcta agggcatgcg ccggcttgag 1200
cttgaacatg ctcagaaatt tatttcacgc ctctacagct ggctatttga gaagtcaggt 1260
cgtgattaca tcccaggccg ccagctgcag ttctacgctc agtgccgccg ctggttatct 1320
gccgggttcc atctcgaccc ccgcacctta gtttttgatg agtcagtgcc ttgtagctgc 1380
cgaaccacca tccggcggat cgctggaaaa ttttgctgtt ttatgaagtg gctcggtcag 1440
gagtgttctt gtttcctcca gcccgccgag gggctggcgg gcgaccaagg tcatgacaat 1500
gaggcctatg aaggctctga tgttgatact gctgagcctg ccaccctaga cattacaggc 1560
tcatacatcg tggatggtcg gtctctgcaa actgtctatc aagctctcga cctgccagct 1620
gacctggtag ctcgcgcagc ccgactgtct gctacagtta ctgttactga aacctctggc 1680
cgtctggatt gccaaacaat gatcggcaat aagacttttc tcactacctt tgttgatggg 1740
gcacgccttg aggttaacgg gcctgagcag cttaacctct cttttgacag ccagcagtgt 1800
agtatggcag ccggcccgtt ttgcctcacc tatgctgccg tagatggcgg gctggaagtt 1860
catttttcca ccgctggcct cgagagccgt gttgttttcc cccctggtaa tgccccgact 1920
gccccgccga gtgaggtcac cgccttctgc tcagctcttt ataggcacaa ccggcagagc 1980
cagcgccagt cggttattgg tagtttgtgg ctgcaccctg aaggtttgct cggcctgttc 2040
ccgccctttt cacccgggca tgagtggcgg tctgctaacc cattttgcgg cgagagcacg 2100
ctctacaccc gcacttggtc cacaattaca gacacaccct taactgtcgg gctaatttcc 2160

ggtcatttgg atgctgctcc ccactcgggg gggccacctg ctactgccac aggccctgct 2220

gtaggctcgt ctgactctcc agaccctgac ccgctacctg atgttacaga tggctcacgc 2280

ccctctgggg cccgtccggc tggccccaac ccgaatggcg ttccgcagcg ccgcttacta 2340

cacacctacc ctgacggcgc taagatctat gtcggctcca ttttcgagtc tgagtgcacc 2400

tggcttgtca acgcatctaa cgccggccac cgccctggtg gcgggctttg tcatgctttt 2460

tttcagcgtt accctgattc gtttgacgcc accaagtttg tgatgcgtga tggtcttgcc 2520

gcgtataccc ttacaccccg gccgatcatt catgcggtgg ccccggacta tcgattggaa 2580

ca 2582

<210> 15

<211> 255

<212> RNA

<213> Hepatitis E virus Japan JRA1(AP003430)

<400> 15

ggcagaccac gtatgtggtc gatgccatgg aggcccacca gttcattaag gctcctggca 60

ttactactgc catagagcag gctgctctgg ctgcggccaa ctccgccttg gcgaatgctg 120

tggtggttcg gccgtttttg tctcgcgtac aaaccgagat ccttattaat ttgatgcaac 180

cccggcagtt ggttttccgc cctgaggtgc tttggaacca tcctattcag agggttatac 240

acaatgaatt agaac 255

<210> 16

<211> 254

<212> RNA

<213> Hepatitis E virus Japan JKN-Sap(AB074918)

<400> 16

gcagaccacg tatgtggtcg atgccatgga ggcccatcag ttcattaagg ctcctggcat 60

tactactgcc attgagcagg ctgctctggc tgcggccaat tccgccttgg cgaatgctgt 120

ggtggtccgg ccgttcttat ctcgtgtaca aactgagatt cttattaatt tgatgcaacc 180
ccggcagttg gttttccgcc ctgaggtgct ctggaatcac cctatccagc gggttataca 240
taatgaattg gaac 254

<210> 17
<211> 238
<212> RNA
<213> Hepatitis E virus JMY-Haw(AB074920)

<400> 17
gtcgatgcca tggaggccca tcaattcatt aaggctcctg gcattactac tgccattgag 60
caggctgctc tggctgcggc caattccgcc ttggcgaatg ctgtggtggt ccggccgttt 120
ttatctcgtg tacaaaccga gattcttatt aatttgatgc aaccccggca gttggttttc 180
cgtcctgagg tgctctggaa tcatcctatt cagcgggtta tacataatga attggaac 238

<210> 18
<211> 264
<212> RNA
<213> Hepatitis E virus USA US-1(AF060669)

<400> 18
tcgacagggg gcagaccacg tatgtggtcg atgccatgga ggcccatcag ttcattaagg 60
ctcctggcat tactactgct attgagcagg ctgctctggc tgcggctaat tccgccttgg 120
cgaatgctgt ggtggttcgg ccgtttcttt ctcgtgtgca aactgagatt cttattaatt 180
tgatgcaacc ccggcagttg gtcttccgcc ctgaggtgct ttggaatcat cctatccagc 240
gggttataca taatgaatta gagc 264

<210> 19
<211> 238

<212> RNA
<213> Hepatitis E virus SWINE HEV(AF082843)

<400> 19
ttcgatgcca tggaggccca tcagttcatt aaggctcctg gcattactac tgccattgag 60
caggctgctc tggctgcggc caactccgcc ttggcgaatg ctgtggtggt tcggccgttt 120
ttatctcgtg tacaaactga gatccttatt aatttgatgc aaccccggca gttggttttc 180
cgccctgagg tactttggaa tcatcctatc cagcgggcaa tacataatga actggaac 238

<210> 20
<211> 238
<212> RNA
<213> Hepatitis E virus Japan JAK-Sai(AB074915)

<400> 20
gtcgacgcca tggaggccca tcagtttata aaggctcctg gcgtcactac tgctattgag 60
caggcagctc tagcagcggc caactccgcc ttggcgaatg ctgtggtggt tcggcctttc 120
ttatcccggc tacagacaga gatactcatt aacctgatgc agccccggca gcttgttttc 180
cggcctgagg ttttatggaa tcacccaatt cagcgtgtga tccacaatga gctcgaac 238

<210> 21
<211> 238
<212> RNA
<213> Hepatitis E virus Japan JKK-Sap(AB074917)

<400> 21
gtcgacgcca tggaggccca ccagttcata aaggctcctg gcgtcactac tgctattgag 60
caggcagctc tagcagcggc caactccgcc ttggcgaatg ctgtggtggt tcggcctttc 120
ctatcccggc tacagacaga gatacttatt aacctgatgc agccccggca gcttgttttc 180

cggcctgaag tcttgtggaa ccacccaatt cagcgcgtga tccacaacga gcttgagc 238

<210> 22

<211> 238

<212> RNA

<213> Hepatitis E virus Japan JSN-FH

<400> 22

gtcgacgcca tggaggccca tcagttcata aaggctcctg gcgtcacaac tgctattgag 60

caggcagctc tagcagcggc caactccgcc ttggcgaatg ctgtggtggt tcggcctttt 120

ctgtcccggc tacagacaga gatacttatt aacttgatgc agccccggca gcttgttttc 180

cggcctgagg tcctgtggaa tcaccccatt caacgcgtga tccacaatga gcttgaac 238

<210> 23

<211> 254

<212> RNA

<213> Hepatitis E virus China type4(AJ272108)

<400> 23

gcagaccacg tatgtggtcg acgccatgga ggcccaccag tttataaagg ctcctggcgt 60

cactactgct attgagcagg cagctctagc agcggccaac tccgccctgg cgaatgctgt 120

ggtggttcgg cctttcttgt cccggcttca gactgagatt ctcataaatt tgatgcagcc 180

ttggcagctt gttttccggc ctgaggtcct gtggaatcac ccaatccagc gtgtgatcca 240

caatgagctt gagc 254

<210> 24

<211> 256

<212> RNA

<213> Hepatitis E virus Burma B1(M73218)

<400> 24

aggcagacca catatgtggt cgatgccatg gaggcccatc agtttattaa ggctcctggc 60

atcactactg ctattgagca ggctgctcta gcagcggcca actctgccct ggcgaatgct 120

gtggtagtta ggccttttct ctctcaccag cagattgaga tcctcattaa cctaatgcaa 180

cctcgccagc ttgttttccg ccccgaggtt ttctggaatc atcccatcca gcgtgtcatc 240

cataacgagc tggagc 256

<210> 25

<211> 256

<212> RNA

<213> Hepatitis E virus China Uigh(D11093)

<400> 25

aggcagacca catatgtggt cgatgccatg gaggcccatc agtttatcaa ggctcctggc 60

atcactactg ctattgagca ggctgctcta gcagcggcca attctgccct tgcgaatgct 120

gtggtagtta ggccttttct ctctcaccag cagattgaga tccttattaa cctaatgcaa 180

cctcgccagc ttgttttccg ccccgaggtt ttctggaacc accccatcca gcgtgtcatc 240

cataatgagc tggagc 256

<210> 26

<211> 256

<212> RNA

<213> Hepatitis E virus China Hebei(M94177)

<400> 26

aggcagacca catatgtggt cgatgccatg gaggcccatc agtttattaa ggctcctggc 60

atcactactg ctattgagca ggctgctcta gcagcggcca actctgccct tgcgaatgct 120

gtggtagtta ggccttttct ctctcaccag cagattgaga tccttattaa cctaatgcaa 180

cctcgccagc ttgttttccg ccccgaggtt ttctggaacc atcccatcca gcgtgttatc 240
cataatgagc tggagc 256

<210> 27
<211> 256
<212> RNA
<213> Hepatitis E virus China Xinjiang(D11092)

<400> 27
aggcagacca catatgtggt cgatgccatg gaggcccatc agtttatcaa ggctcctggc 60
atcactactg ctattgagca ggctgctcta gcagcggcca actctgccct tgcgaatgct 120
gtggtagtta ggccttttct ctctcaccag cagattgaga tccttattaa cctaatgcaa 180
ccccgccagc ttgttttccg ccccgaggtt ttctggaacc atcccatcca gcgtgttatc 240
cataatgagc tggagc 256

<210> 28
<211> 256
<212> RNA
<213> Hepatitis E virus Nepali(AF051830)

<400> 28
aggcagacca catatgtggt cgatgccatg gaggcccatc agtttattaa ggctcctggc 60
atcactactg ctattgagca ggctgctcta gcagcggcca actctgccct ggcgaatgct 120
gtggtagtta ggccttttct ctctcaccag cagattgaga ttctcattaa cctaatgcaa 180
cctcgccagc ttgttttccg ccccgaggtt ttctggaatc atcccatcca gcgtgtcatc 240
cataacgagc tggagc 256

<210> 29
<211> 255

<212> RNA
<213> Hepatitis E virus India FH strain(X98292)

<400> 29
ggcagaccac ctatgtggtc gatgccatgg aggcccatca gtttatcaag gctcctggca 60
tcactactgc tattgagcag gctgctctgg cagcggccaa ctctgccctg gcgaatgctg 120
tggtagttag gccctttctc tctcaccagc agattgagat cctcattaac ctaatgcaac 180
ctcgccagct tgttttccgc cccgaggttt tctggaacca ccccatccag cgtgttatcc 240
acaatgagtt ggagc 255

<210> 30
<211> 226
<212> RNA
<213> Hepatitis E virus Pakistan SAR55(M80581)

<400> 30
gaggcccatc agtttatcaa ggctcctggc atcactactg ctattgagca ggctgctcta 60
gcagcggcca actctgccct tgcgaatgct gtggtagtta ggccttttct ctctcaccag 120
cagattgaga tccttattaa cctaatgcaa cctcgccagc ttgttttccg ccccgaggtt 180
ttctggaacc atcccatcca gcgtgttatc cataatgagc tggagc 226

<210> 31
<211> 232
<212> RNA
<213> Hepatitis E virus Mexico(M74506)

<400> 31
gccatggagg cccaccagtt cattaaggct cctggcatca ctactgctat tgagcaagca 60
gctctagcag cggccaactc cgcccttgcg aatgctgtgg tggtccggcc tttcctttcc 120

catcagcagg ttgagatcct tataaatctc atgcaacctc ggcagctggt gtttcgtcct 180

gaggtttttt ggaatcaccc gattcaacgt gttatacata atgagcttga gc 232

<210> 32

<211> 20

<212> RNA

<213> Hepatitis E virus

<400> 32

gcagaccacr tatgtgktcg 20

<210> 33

<211> 20

<212> RNA

<213> Hepatitis E virus

<400> 33

ccacrtatgt ggtcgaygcc 20

<210> 34

<211> 20

<212> RNA

<213> Hepatitis E virus

<400> 34

acmarctgsc grggytgcat 20

<210> 35

<211> 20

<212> RNA
<213> Hepatitis E virus

<400> 35
cgytgratwg grtgrttcca                                                    20

<210> 36
<211> 19
<212> RNA
<213> Hepatitis E virus

<400> 36
tgktcgaygc catggaggc                                                     19

<210> 37
<211> 19
<212> RNA
<213> Hepatitis E virus

<400> 37
tgktcgaygc catggaggc                                                     19

<210> 38
<211> 20
<212> RNA
<213> Hepatitis E virus

<400> 38
aygccatgga ggcccaycag                                                    20

<210> 39

<211> 20

<212> RNA

<213> Hepatitis E virus

<400> 39

ckracyacca cagcattcgc 20

<210> 40

<211> 20

<212> RNA

<213> Hepatitis E virus

<400> 40

ggcckracya ccacagcatt 20

<210> 41

<211> 53

<212> RNA

<213> Hepatitis E virus

<400> 41

aaggctcctg gcrtyactac tgcyatwgag caggcwgctc trgcwgcggc caa 53

<210> 42

<211> 19

<212> RNA

<213> Hepatitis E virus

<400> 42

ctcctggcat cactactgc                                                    19

<210> 43

<211> 19

<212> RNA

<213> Hepatitis E virus

<400> 43

ctcctggcat tactactgc                                                    19

<210> 44

<211> 7256

<212> RNA

<213> Hepatitis E Virus Japan JKN-Sap(AB074918)

<400> 44

gcagaccacg tatgtggtcg atgccatgga ggcccatcag ttcattaagg ctcctggcat 60

tactactgcc attgagcagg ctgctctggc tgcggccaat tccgccttgg cgaatgctgt 120

ggtggtccgg ccgttcttat ctcgtgtaca aactgagatt cttattaatt tgatgcaacc 180

ccggcagttg gttttccgcc ctgaggtgct ctggaatcac cctatccagc gggttataca 240

taatgaattg gaacagtact gccgggcccg ggccggccgt tgcctggagg ttggggctca 300

cccgaggtcc attaatgaca atcccaatgt cctgcacagg tgctttctta gaccggttgg 360

ccgagacgtc cagcgctggt actctgcccc cacccgtggc cctgcggcca actgccgccg 420

ctccgcgttg cgtggtctcc ctcccgctga ccgcacttat tgctttgatg gattctcccg 480

ctgtgctttt gctgcagaga ccggcgtggc cctttactct ctgcatgacc tttggccagc 540

tgatgtcgca gaggctatgg cccgccacgg gatgacacgc ctatatgctg cactacacct 600

ccctcctgag gtgctgttgc cacccggcac ttaccacaca acctcgtatc tcctgatcca 660

cgacggcgac cgtgccgtcg taacttatga gggcgatact agtgcaggct acaatcacga 720
tgtttccata cttcgtgcgt ggatccgtac tactaaaata gttggtgacc acccgttggt 780
tatagagcgt gtgcgggcca ttggatgtca ttttgtgctg ctgctcaccg cagcccctga 840
gccgtcacct atgccttatg tcccctaccc tcgttcaact gaggtgtatg tacgatctat 900
atttggccct ggtggctccc catctttgtt cccgtcagcc tgctctacta aatctacttt 960
tcatgctgtc ccggttcata tctgggaccg gcttatgctt tttggtgcca ccctggacga 1020
tcaggcgttt tgttgttcac ggctcatgac ttacctccgt ggtattagct acaaggtcac 1080
tgtcggtgcg cttgttgcta atgagggatg gaatgcctct gaggacgccc ttactgcagt 1140
gatcactgcg gcttacctga ctatttgcca ccaacgctac cttcgaaccc aggcgatatc 1200
caagggtatg cgccggttgg aggttgagca tgcccagaaa ttcatcacaa ggctctacag 1260
ctggctattt gagaaatctg gtcgtgatta tatccccggc cgccagcttc agttctatgc 1320
acaatgtcgg cggtggttat ctgcaggctt ccacctcgac cccagggtgc ttgtcttcga 1380
tgaagcagtg ccatgccgct gtaggacgtt tttgaagaag gtcgcgggta aattctgctg 1440
ttttatgcgg tggctagggc aggagtgcac ctgtttcttg gagccagctg agggcctaat 1500
tggagaccaa ggccatgata atgaggccta tgagggttct gaggtcgacc cggctgaacc 1560
tgcacatctt gatgtttcgg ggacctatgc tgtccatggg catcagcttg aggcccttta 1620
tagggcactc aatgtcccac atgatattgc cgctcgagcc tcccggctaa cggctactgt 1680
cgagcttgtt gcaagtccag accgcttaga gtgccgtact gtgcttggta ataagacctt 1740
tcggacaacg gtggttgatg gtgcccatct tgaagcaaat ggccctgagg agtatgttct 1800
atcattcgac gcctctcgtc agtctatggg ggccggatcg cacagcctca catatgagct 1860
cacccctgct ggtctgcagg tcaggatttc atctaatggc ttggattgta ccgccgtatt 1920
ccctcccggc ggcgccccta gcgccgcacc gggggaggtg gcagccttct gcagcgccct 1980
ttatagatat aacaggttca cccaacggca ctcgctaacc ggtggattat ggttacaccc 2040
tgaggggttg ctgggcatct tccccccttt ctctcctgga cacatctggg agtctgctaa 2100
cccattttgt ggggagggga ccttgtatac ccgaacctgg tcaacatctg gcttctctag 2160
cgacttctcc ccccctgagg cggccgcccc tgttccggct gctgccccgg ggctgcccca 2220
ccccacccca cctgttagtg acatttgggt gctgccacca ccctcagagg agtcccagat 2280
cgatgcggca cctgtgcccc ctgtccctaa gactgttgga ttgcctagcc ccattgtact 2340
tgctcctccc tcccctcttc cttcccccgt gcgtaagcca ccatcacccc cgccttctcg 2400

cactcgtcgt ctcctctaca cctatcctga cggcgctagg gtatatgcgg ggtcgttgtt 2460
tgaatcagac tgtgactggc tagttaacgc ctcaaatccg ggccaccgtc ctggaggtgg 2520
cctctgccac gccttttacc aacgcttccc agaggcgttt tacccaactg aattcattat 2580
gcgtgagggc cttgcagcat ataccctgac cccgcgccct atcattcatg cagtggctcc 2640
cgactatagg gtcgagcaga atccgaagag gcttgaggca gcgtaccggg aaacttgctc 2700
ccgtcgcggc accgctgcct atccgctttt gggctcgggt atataccagg tccctgttag 2760
tctcagtttt gatgcctggg aacgcaatca tcgccccggc gacgagcttt acttgactga 2820
gcccgctgca gcttggtttg aggctaataa gccatcgcag ccggcgctta ctataactga 2880
ggacacggct cgtacggcca atctggcatt agagattgat gccgccacag aggttggccg 2940
tgcttgtgcc ggctgcacta tcagcccggg ggttgtgcat taccagttta ctgccggggt 3000
cccgggctcg ggcaagtcaa ggtccataca acagggagac gtcgatgtgg tggttgtgcc 3060
cacccgggag cttcgtaata gttggcgccg ccggggtttt gcggctttca cacctcacac 3120
agcggcccgt gttactattg gtcgccgcgt tgtgattgat gaggctccgt ccctcccgcc 3180
gcacttgctg ctgctacaca tgcaacgggc ctcctcggtc catctcctcg gcgacccaaa 3240
tcagattcct gctattgatt ttgaacatgc cggcctggtc cccgcgatcc gtcccgagct 3300
tgcaccaacg agctggtggc atgttacaca ccgctgcccg gcagatgtgt gtgagcttat 3360
acgtggggcc taccctaaga tccagaccac gagtcgtgtg ctacggtccc tgttttggaa 3420
cgaaccggcc attggccaga agctggtttt cacgcaggct gctaaggctg ctaatcctgg 3480
tgcgatcacg gttcatgagg ctcagggtgc caccttcacg gagaccacaa tcatagccac 3540
ggctgatgct aggggcctta ttcagtcatc ccgagctcac gctatagtcg cactcacccg 3600
ccacactgag aagtgtgtta ttttagatgc ccccggccta ctgcgcgagg tcggtatttc 3660
agatgtgatt gtcaataact ttttccttgc tggtggagag gttggccacc accgcccctc 3720
cgtgatacct cgcggtaacc ccgatcagaa tctcgggact ctacaggcat tcccgccgtc 3780
ttgccagatt agtgcctacc accagttggc tgaggaatta ggccaccgcc cagctcctgt 3840
cgccgccgtc ttaccccctt gcccggagct tgagcagggc ctgctctaca tgccacaaga 3900
gcttactgtg tccgatagtg tgttggtatt tgaactcaca gatatagtcc attgccgtat 3960
ggccgctcca agccagcgaa aggctgttct ctcaacactt gtcgggaggt atggccgtag 4020
aacgaaatta tatgaggcgg cacattcaga tgttcgtgag tccctagcta ggttcatccc 4080
cactatcggg cctgttcagg ccaccacatg tgagttgtat gagttggttg aggccatggt 4140

ggagaagggt caggacggct ctgccgtcct agagcttgac ctttgcaatc gtgacgtctc 4200
gcgtatcaca tttttccaaa aggattgcaa taaattcaca actggtgaga ctattgccca 4260
tggcaaggtt ggccagggta tatcggcctg gagtaagacc ttctgtgccc tgtttggccc 4320
gtggttccgc gctatagaaa aagagatatt ggccctgctc ccgcctaata tcttttatgg 4380
cgacgcttat gaagagtcag tgtttgctgc cgctgtgtct ggggcggggt catgtatggt 4440
atttgaaaat gatttttcgg aatttgacag tactcagaac aacttctctc tcggccttga 4500
gtgtgtggtc atggaggagt gcggcatgcc ccagtggttg attaggttgt accatctggt 4560
tcggtcagcc tggattttgc aggcgccgaa ggagtctctt aagggttttt ggaagaagca 4620
ctctggtgag cctggtaccc ttctctggaa caccgtctgg aacatggcga ttatagcgca 4680
ctgttacgag ttccgtgact ttcgcgttgc cgccttcaag ggtgatgatt cggtggtcct 4740
ttgcagcgac tatcggcaga gccgcaatgc ggctgcctta attgcaggct gtgggctcaa 4800
attgaaggtc gattatcgtc ctattgggct gtatgctggg gtggtggtgg cccctggttt 4860
ggggacactg cccgacgtgg tgcgttttgc tggtcggttg tctgaaaaga attggggccc 4920
cggccctgaa cgtgctgagc agctgcgtct cgctgtttgt gatttccttc gagggttgac 4980
gaatgttgcg caggtttgtg ttgatgttgt gtcccgtgtt tacggagtca gccccgggct 5040
ggtacataac cttattggca tgctgcagac cattgccgat ggcaaggctc acttcacaga 5100
gaccattaaa cctgtgcttg accttacgaa ttccatcata cagcgggaag aatgaataac 5160
atgtcttttg catcgcccat gggatcacca tgcgccctag ggctgttctg ttgttgttcc 5220
tcgtgctttt gcctatgctg cccgcgccac cggccggtca gccgtctggc cgtcgccgtg 5280
ggcggcgcag cggcggtgcc ggcggtggtt tctggggtga cagggttgat tctcagccct 5340
tcgccctccc ctatattcat ccaaccaacc ccttcgccgc cgatgtcgtt tcacaacccg 5400
gggctggaac tcgccctcga cagccgcccc gccccttggc ctccgcttgg cgtgaccagt 5460
cccagcgccc ctccgctgcc ccccgccgtc gatctgcccc agctggggct gcgccgttga 5520
ccgctgtatc accggctcct gacacagccc ctgtgcctga tgttgattca cgcggtgcta 5580
tcctgcgccg gcagtacaat ctgtccacgt ccccgctcac gtcatctgtc gcctcgggca 5640
caaatctggt tctctatgct gccccgctta atcctctcct gccccttcag gatggcacca 5700
acactcatat tatggccact gaggcatcca attatgccca gtatcgggtt gttcgagcta 5760
cgatccgtta tcgcccgttg gtgccgaatg cagttggcgg ttatgctatt tctatttctt 5820
tttggcctca aaccacaact actcccacct ctgtcgacat gaattctatc acttccactg 5880

atgttaggat tttggttcag cccggcattg cctccgagtt agtcatccct agtgagcgcc 5940

tccactaccg caatcaaggc tggcgctctg ttgagaccac gggtgtggcc gaggaggagg 6000

ctacttccgg tctggtaatg ctttgtattc atggctctcc tgttaattcc tacactaata 6060

caccttatac tggtgcactg ggctccttgg attttgcatt agagcttgaa tttagaaatc 6120

tgacacccgg gaacacaaac acccgtgttt cccggtatac cagcacagcc cgtcaccggc 6180

tgcgccgcgg tgctgatggg actgctgagc ttaccaccac agcagccaca cgtttcatga 6240

aggatttaca ttttactggc acgaatggtg ttggtgaggt gggtcgtggc atcgctctga 6300

cattgtttaa tctcgctgac acgcttctcg gtggtttacc gacagaattg atttcgtcgg 6360

ccgggggtca actgttttac tcccgccctg ttgtctcggc caatggcgag ccaacagtaa 6420

agttatacac atctgttgag aatgcgcagc aagataaggg cattaccatc ccacacgata 6480

tagatctggg tgactcccgt gtggttattc aggattatga taaccagcac gagcaagatc 6540

ggcctactcc gtcacctgcc ccctcccgcc ctttctcagt tcttcgtgct aatgatgttc 6600

tgtggctctc cctcaccgcc gctgagtatg accagactac gtatgggtcg tccaccaacc 6660

ctatgtatgt ctccgacaca gtcacgctcg ttaatgtggc cactggagcc caggctgtgg 6720

cccgctctct tgattggtct aaagttacct tggatggccg cccccttact accattcagc 6780

agtattctaa gacattctat gtccttccgc tccgcgggaa gctgtctttc tgggaggctg 6840

gtacgactaa ggccggttac ccgtataatt ataatactac tgctagtgat cagatcttga 6900

ttgagaacgc ggccggccac cgtgtcgcta tttctaccta tactactagc ttgggtgccg 6960

gccctacctc gatctctgcg gtcggtgtac tagctccaca ttcggccctc gccgttctag 7020

aggacaccgt tgattacccc gcccgcgctc acacttttga tgatttctgc ccggagtgcc 7080

gtaccctcgg tttgcagggt tgtgcattcc agtctactat cgctgagctt cagcgtctta 7140

aaatgaaggt aggtaaaacc cgggagtctt aattaattcc ttttgtgccc ccttcgcagc 7200

tttctctggc tttatttctt atttctgctt ttcgcgctcc ctggaaaaaa aaaaaa 7256

<210> 45

<211> 7233

<212> RNA

<213> Hepatitis E Virus Japan JMY-Haw(AB074920)

<400> 45
   ccatggaggc ccatcaattc attaaggctc ctggcattac tactgccatt gagcaggctg 60
   ctctggctgc ggccaattcc gccttggcga atgctgtggt ggtccggccg tttttatctc 120
   gtgtacaaac cgagattctt attaatttga tgcaaccccg gcagttggtt ttccgtcctg 180
   aggtgctctg gaatcatcct attcagcggg ttatacataa tgaattggaa cagtactgcc 240
   gggcccgggc cggccgctgc ctggaggttg gggcccaccc gagatccatt aatgataatc 300
   ccaatgtcct gcacaggtgc tttcttagac cggtcggccg agatgtccaa cgctggtact 360
   ctgcccccac ccgcggtcct gcggccaatt gccgccgctc cgcgttgcgt ggtctccctc 420
   ccgctgaccg cacttattgc tttgatggat tctcccgctg tgcttttgct gcagagaccg 480
   gcgtggctct ttactctttg catgaccttt ggccagctga tgttgcagag gctatggccc 540
   gccatgggat gacacgccta tatgctgtac tacaccttcc tcctgaggtg ctgctaccac 600
   ccggcactta ccacacaact tcgtatctcc tgatccacga cggcgaccgt gccgttgtaa 660
   cttatgaggg tgatactagt gcgggctaca accacgatgt ttccatactt cgtgcgtgga 720
   tccgcactac taaaatagtt ggtgaccacc cgctggttat agagcgtgtg cgggccattg 780
   gatgtcactt tgtgctgctg ctcaccgcag cccctgagcc gtcacctatg ccttatgtcc 840
   cctaccctcg ttcaactgag gtgtatgtac gatctatatt tggccctggt ggctccccat 900
   ctctgttccc gtcagcctgc tctactaaat ctacttttca tgctgtcccg gttcatatct 960
   gggatcggct tatgcttttt ggtgccaccc tggacgatca ggcgttttgt tgttcacggc 1020
   tcatgactta cctccgtggt attagctaca aggtcactgt cggcgcgctg gtcgctaatg 1080
   aaggatggaa cgcctctgag gacgccctta ctgcagtgat cactgcggct tatctgacta 1140
   tttgccacca gcgctacctc cgaacccagg cgatatccaa gggtatgcgc cggttggagg 1200
   ttgagcatgc ccagaaattt attacaaggc tctacagctg gctatttgag aaatctggcc 1260
   gtgattatat tcccggccgc cagcttcagt tctatgcaca atgccggcgg tggctatctg 1320
   caggtttcca tctcgacccc agggtgcttg tctttgatga atcagtgcca tgccgttgta 1380
   ggacgttttt gaagaaggtc gcgggtaaat tctgctgttt tatgcggtgg ctggggcagg 1440
   agtgcacctg ttttttggag cccgccgagg gtttagttgg agaccaaggc catgacaacg 1500
   aggcctatga gggttctgag gtcgacccgg ccgaacctgc acatcttgat gtttcgggta 1560
   cctatgctgt ccatgggcac cagcttgagg ccctttatag agcacttaat gtcccacatg 1620
   atattgccgc tcgagcctcc cggctaacgg ctactgtcga gcttgttgca agtccagacc 1680
   gcttggagtg ccgtactgtg ctcggtaata agaccttccg gacaacggtg gttgatggcg 1740
   cccatcttga agcaaatggc cctgagcagt atgttctatc atttgacgcc tcccgtcagt 1800
   ctatgggggc cgggtcgcat agcctcactt atgagctcac ccctgccggt ctgcaggtca 1860
   ggatttcatc taatggcctg gattgcaccg ctgtattccc tcccggcggc gcccctagcg 1920
   ccgcaccggg ggaggtggca gccttctgca gcgcccttta tagatataac aggttcaccc 1980
   aacggcactc gctgactggt ggattatggt tacaccctga ggggttgctg ggtatcttcc 2040
   cccctttctc acctgggcac atctgggagt ctgctaatcc attttgtggg gaggggacct 2100
   tgtatacccg aacctggtca acatctggct tttctagtga cttctccccc cctgaggcgg 2160
   ccgcccctgt tccggctgct gccccgggat tgccccaccc tactccacct gttagtgata 2220
   tttgggtgct gccacccccc tcagaggggt cccagatcga tgcggcacct gtgccccctg 2280
   ttcctaagac tgttggattg cctagcccca ttgtacttgc tcctcccccc ccctttcctt 2340
   cccccgtgcg taagccatca tcacccccgc cttctcgcac ccgtcgtctc ctctacacct 2400
   atcctgacgg cgctagggta tatgcggggt cattgtttga atcagattgt gactggctgg 2460
   ttaatgcctc aaacccggga caccgtcctg gaggtggcct ttgccatgcc ttttaccagc 2520
   gtttcccaga ggcgttttac ccgactgaat tcattatgcg tgagggcctc gcagcatata 2580
   ccctgacccc gcgccctatc attcatgcag tggctcccga ctatagggtt gagcagaacc 2640
   cgaagaggct tgaggcagcg tatcgggaaa cttgctctcg tcgtggcacc gctgcctatc 2700
   cgcttttggg ctcgggtata taccaggtcc ctgttagtct cagttttgat gcctgggaac 2760
   gcaatcatcg ccccggcgac gagctttatc tgactgagcc cgctgcagcc tggtttgagg 2820
   ctaataagcc ctcgcagccg gcgcttacta taactgagga cacggctcgt acggccaacc 2880
   tggcattaga gattgatgcc gccacagagg ttggccgtgc ttgtgccggc tgcactatta 2940
   gcccagggat tgtgcattat cagtttactg ccggggtccc gggctcgggc aagtcaaggt 3000
   ccatacaaca gggagacgtc gatgtggtgg ttgtgcccac ccgggagctt cgtaatagtt 3060
   ggcgtcgccg gggttttgcg gctttcacgc ctcacacagc ggcccgtgtt actatcggtc 3120
   gccgcgttgt gattgatgag gccccatccc tcccgccgca tctgctgctg ttacacatgc 3180
   aacgggcctc ctcggtccat ctcctcggcg acccaaatca gattcctgct attgattttg 3240
   agcatgccgg cttggtcccc gcgatccgcc ccgagcttgc gccaacgagc tggtggcacg 3300
   ttacacaccg ctgcccggca gacgtgtgcg agcttatacg tggagcctac cctaagatcc 3360
   agaccacgag ccgtgtgcta cggtctctgt tttggaacga accggccatt ggccagaagc 3420
   tggttttcac gcaggctgct aaggctgcta atcctggtgc gatcacggtt catgaagctc 3480
   agggtgccac cttcacggag accacaatta tagccacggc tgacgccagg ggccttattc 3540
   agtcatcccg ggctcacgct atagttgcac tcactcgcca cactgagaag tgtgttattt 3600
   tagatgcccc cggcctgttg cgcgaggtcg gtatttcgga tgtgattgtt aacaactttt 3660
   tccttgctgg tggagaggtc ggccaccacc gcccctctgt gataccccgc ggcaaccccg 3720
   atcagaatct cgggacttta caggcattcc cgccgtcttg ccagattagt gcctaccacc 3780
   agttggctga ggaattaggc caccgcccgg cccctgtcgc tgccgtctta cccccttgcc 3840
   cggagcttga gcagggccta ctctacatgc cacaagagct tactgtgtcc gatagtgtgt 3900
   tggtttttga gctcacagat atagtccact gtcgtatggc cgccccaagt cagcgaaagg 3960
   ctgttctctc tacacttgtg gggaggtatg gccgtagaac gaaattatat gaggcggcgc 4020
   attcagatgt tcgtgagtcc ttagctaggt tcatccccac tatcgggcct gttcaggcca 4080
   ccacatgtga gttgtatgag ttggtcgagg ccatggtgga gaagggtcag gacggctctg 4140
   ccgtcctaga gcttgatctt tgcaatcgtg acgtctcgcg tatcacattt ttccaaaagg 4200
   attgcaataa gtttacaact ggtgagacta tagcccatgg caaggttggc cagggtatat 4260
   cggcctggag caagaccttc tgcgccctgt tcggcccgtg gttccgcgct atagaaaaag 4320
   aaatattggc cctgctcccg cctaatattt tttatggcga cgcttatgag gagtcagtgt 4380
   ttgctgccgc tgtgtctggg gcggggtcat gtatggtatt tgaaaatgat ttctcggagt 4440
   ttgacagtac tcagaataat ttctctctcg gtcttgagtg tgtggtcatg gaggagtgcg 4500
   gcatgcccca atggttgatt aggttgtacc atctggttcg gtcagcctgg attttgcagg 4560
   cgccgaagga gtcccttaag ggtttttgga agaagcactc tggtgagccc ggcacccttc 4620
   tctggaatac cgtctggaac atggcgatta tagcgcattg ttacgagttc cgtgactttc 4680
   gcgttgccgc cttcaagggt gatgattcgg tggtgctttg tagcgactac cggcagagcc 4740
   gcaatgcggc tgccttaatt gcaggctgtg ggctcaaatt gaaggtcgat taccgcccta 4800
   ttgggctgta cgctggggtg gtggtggccc ctggtttagg gacactgcct gatgtggtgc 4860
   gttttgctgg tcggttgtct gaaaagaatt ggggccccgg ccctgaacgc gctgagcagc 4920
   tgcgtctcgc cgtttgtgat ttccttcgag ggttgacgaa tgtcgcgcag gtctgtgttg 4980
   atgttgtgtc ccgtgtctac ggagtcagcc ccgggctggt acataacctt attggcatgc 5040
   tgcagactat cgccgatggc aaggcccact ttacagagac cattaaacct gtgcttgacc 5100
   ttacgaattc catcatacag cgggaggaat gaataacatg tcttttgcac cgcccatggg 5160
   atcaccatgc gccctagggc tgttctgttg ttgttcctcg tgcttctgcc tatgctgccc 5220
   gcgccaccgg ccggccagcc gtctggccgt cgtcgtgggc ggcgcagcgg cggtaccggc 5280
   ggtggtttct ggggtgacag ggttgattct cagcccttcg ccctccccta tattcatcca 5340
   accaacccct tcgccgccga tgtcgtttca caacccgggg ctggagctcg ccctcgacag 5400
   ccgccccgcc cccttggctc cgcttggcgt gaccagtccc agcgcccctc cgctgccccc 5460
   cgccgtcgat ctgccccagc tggggctgcg ccgttgaccg ctgtatcacc ggctcctgac 5520
   acagcccctg tacctgatgt tgattcacgc ggcgctattc tgcgccggca gtacaatttg 5580
   tccacgtccc cgcttacgtc atccgttgcc tcgggtacaa atctggttct ttatgctgcc 5640
   ccgcttaatc ctctccttcc ccttcaggat ggcaccaaca ctcatattat ggccactgag 5700
   gcatctaatt atgcccagta tcgggttgtt cgagctacga tccgttatcg cccgttggtg 5760
   ccgaacgctg ttggcggtta tgctatttct atctcttttt ggcctcaaac cacaactact 5820
   ccaacttctg ttgatatgaa ttccattact tccactgatg ttaggatttt ggttcagccc 5880
   ggtattgcct ccgagttagt catccctagt gagcgcctcc actaccgcaa tcaaggctgg 5940
   cgctctgttg agaccacggg tgtggccgag gaggaggcta cttccggtct ggtaatgctt 6000
   tgcattcatg gttctcctgt taattcctat actaatacac cttatactgg tgcactgggg 6060
   ctccttgatt ttgcattaga gcttgaattt agaaatctga cacccgggaa cacaaacacc 6120
   cgtgtttccc ggtataccag cacagcccgt caccggctgc gacgcggtgc tgatgggact 6180
   gctgagctca ccaccacagc agccacacgt tttatgaagg atttgcattt tactggcacg 6240
   aacggtgttg gtgaggtggg tcgtggcatc gctctgacat tgtttaatct cgctgatacg 6300
   cttctcggtg gtttaccgac agaattgatt tcgtcggccg ggggtcagct gttttactcc 6360
   cgccctgttg tctcagccaa tggcgagcca acagtaaagt tatatacatc tgttgagaac 6420
   gcgcagcaag ataagggcat taccatccca cacgatatag atttgggtga ctcccgtgtg 6480
   gttatccagg attatgataa ccagcacgag caagatcggc ctaccccgtc acctgccccc 6540
   tcccgccctt tctcagttct tcgtgccaat gatgttctgt ggctctccct caccgccgct 6600
   gagtacgacc agactacata tgggtcgtcc accaacccta tgtatgtctc cgacacggtc 6660
   acgctcgtta atgtggccac tggtgctcag gctgttgccc gctctctcga ttggtctaaa 6720
   gttaccttgg atggccgccc ccttactact attcagcagt attctaagac attctatgtc 6780
   ctcccgcttc gcgggaagct gtctttttgg gaggctggca ctaccaaggc cggctacccg 6840
   tataattata atactactgc tagtgatcaa attttgattg agaacgcggc cggccaccgt 6900
   gtcgctattt ctacctatac tactagtttg ggtgccggtc ctacctcgat ctctgcggtc 6960
   ggtgttctag ccccacactc ggccctcgcc gctctagagg acaccgttga ttaccccgcc 7020
   cgcgctcaca cttttgatga tttttgcccg gagtgccgta ccctcggttt gcagggttgt 7080
   gcattccagt ctactatcgc tgagcttcag cgtcttaaaa tgaaggtagg taaaacccgg 7140
   gagtcttaat taattccttt tgtgccccct tcgtagttcc ctctggcttt atttcttatt 7200
   tctgctcccc gcgctccctg gaaaaaaaaa aaa 7233

<210> 46
<211> 7229
<212> RNA
<213> Hepatitis E Virus Japan JAK-Sai(AB074915)

<400> 46
ccatggaggc ccatcagttt ataaaggctc ctggcgtcac tactgctatt gagcaggcag 60
ctctagcagc ggccaactcc gccttggcga atgctgtggt ggttcggcct ttcttatccc 120
ggctacagac agagatactc attaacctga tgcagccccg gcagcttgtt ttccggcctg 180
aggttttatg gaatcaccca attcagcgtg tgatccacaa tgagctcgaa cagtattgtc 240
gggctcgcgc tggccgctgc cttgaagtgg gcgctcatcc gcgctccatt aacgacaatc 300
ccaatgttct tcaccgctgc tttttaaaac ctgttggccg cgatgttcag cggtggtaca 360
ccgcccctac ccgtggccct gcagcgaact gtcgccgatc ggcccttcgt ggccttccac 420
ctgccgaccg gacgtattgc tttgatggtt tctccggctg tacattcgct gctgagacgg 480
gcgtagccct ctactcactg cacgatctct ggcccgccga tgtcgctgag gcgatggctc 540
gccacggcat gactcggttg tatgcagccc tccacctccc cccggaggtg ctactccccc 600
ccggcaccta tcatactacc tcgtatctcc tcatccatga tggggaccgt gcagtgatta 660
cttatgaggg ggactctagc gctgggtaca accatgatgt atccatcctg cgcgcctgga 720
tccgcactac caaggtcact ggtgaccacc cgttggtaat tgagcgggtt cgggcggtgg 780
gttgtcactt tgtgctcctc ctcacagccg cgcctgaacc atcaccaatg ccctatgttc 840
catatcctcg ttctaccgag gtttatgtgc ggtccatttt tggccctggc ggttcacctt 900
ccctgttccc atctgcctgc tctactaagt caacatttca tgctgtccct gtgcatatat 960

gggacagact catgctcttt ggtgcgaccc tcgacgacca ggccttctgt tgctcgcggc 1020
taatgacata cctccgtggc attagttaca aggttacggt cggtgctctc gtcgccaatg 1080
agggttggaa tgcctccgaa gatgcattga ctgccgtgat tactgcggcc tatctcacca 1140
tttgccacca gaggtatctc cgcacgcagg ctatctcgaa agggatgaaa agattggagc 1200
ttgagcatgc acagaagttt ataacacgcc tttacagctg gctgtttgaa aagtccggcc 1260
gtgactacat ccccggccgt caactgcagt tctacgctca gtgccgtcgg tggctatctg 1320
ctggtttcca ccttgatcct cgtgtgctcg tctttgacga agcagccccc tgccgctgcc 1380
gtagccttct ccgtaaagca gcacataaat tttgctgctt tatgcggtgg ctggggcagg 1440
actgtacctg tttcctccag cccatcgaag gaagggttgg tgagcagggt tatgacaatg 1500
aggcatttga ggggtcggac gtcgatcctg ctgaggaggc tactgtgagc atttctgggt 1560
catatatcgt cactggcagc cagctgcagc ccctctacca ggcgcttggc atcccttctg 1620
atcttgccgc ccgagcgggt cggcttactg ccaccgtcga agtctctgat gccgacggcc 1680
gccttacttg taagaccatc atgggcaata agacttttac aacagttttt actgatggtg 1740
cccagctaga ggtcaacggg ccagagcaat atgtgctgtc gtttgatttg gctaaacaga 1800
cgatggccgc tggcccgcat agccttagtt atgttctgac atccgcgggc cttgaggtgc 1860
gtgttgtttc tgctgggctt gactgtaagg ctgtttttcc atctggggtt gcaaccccct 1920
ccgcttctgg cgaggtgtca gccttctgtt cagctttata caggtttaac cgttgcgttc 1980
agcggcattc tcttattggg ggcttgtggt attaccctga ggggctaatc ggcctgttcc 2040
caccgtttgc tcccggccac acctgggagt caaccaaccc tttctgtgga gaaagtaccc 2100
tttatactcg cacctggtcg gtgtcagggt tttctagctg tttctcccca cttgagcccc 2160
ctgcctcggg ttcactaccc cctgcggaga tcgacccacc cgtgactgtt gatgccccat 2220
ctccatctat tttggcattg ccccggccct cagtttttga acagaccacg cccccattgg 2280
atccagctgg tgacgctgct gcttcggccc cgccaggtgc ccctggtgtg cccccggcac 2340
cagcgcggcc tgtaacccac ccatccgggc cccgccggcg gttactccat acttatcctg 2400
atggctcaaa ggtgtatgcc ggttctctct ttgagtccga gtgtacttgg ctggttaatg 2460
catccaatcc cggtcaccgc cctggtggcg gtctctgcca tgcgttttac cagcggtttc 2520
cggagtcatt tgatcccgcc gagtttgtca tgtctgatgg gtttgcggcc tacaccttga 2580
caccccggcc cataattcac gctgttgctc ccgattatcg agtagaacat aatcctaaga 2640
ggctcgaggc tgcctatcgg gagacgtgct ctcgccgcgg gacggccgct taccctctgc 2700

tcggcgccgg tatatataag gtacctgttg ggctgagttt tgatgcctgg gaacgtaacc 2760
accggcccgg ggatgagttg tacctgaccg agccagctat agcttggttt gaggctaacc 2820
ggcctactct ccctgcgctt accattactg aggacacggc acgaacagca aatctggcgc 2880
tggagttgga ttcggccact gaggttggcc gggcatgtgc cggctgtcgt gttgagcctg 2940
gtgtcgtcca ctaccagttt acggcaggtg tccctggctc cggcaagtca cggtcaattc 3000
agcagggtga ggtcgatgtg gtggtggtgc caactcgtga gctgcgtaac tcctggcgac 3060
gccgtgggtt tgcagcttac acaccccaca ccgcggcccg cgtcacccgc ggtcgcagga 3120
ttgttattga cgaggccccg gcacttccac cacatttact tctgctacat atgcagcggg 3180
cctcgtcagt ccacctcctc ggcgacccca accagatccc tgctattgac ttcgagcatg 3240
ccggtcttgt cccggcaatc cggcctgagc ttgtcccaac aaagtggtgg catcttaccc 3300
ataggtgccc agcagacgtc tgtgagctaa ttcgtggcgc gtatccaaag atccagacgg 3360
caagccgtgt cctccgctct ctgttttggg gagagccccc tgtaggccaa aagttagtat 3420
tcactcaggc ggcaaaggcc gctaaccctg gtgcaattac agtccatgag gcccagggtg 3480
ccacatttac tgagaccact attattgcta cggcagacgc ccgcgggctg atccagtctt 3540
ctagggccca cgctattgta gccctgactc gccacacaga gaaatgtgta gttgttgatg 3600
ccccggggct ccttcgtgag gttggtattt ctgatgctat tgttaataac tttttccttt 3660
ccggcggcca gattggtcaa caccgcccat cagttatacc gcgcggcact gttgacagca 3720
atgttgatac gctcgatgcg tttccacctt cctgtcaatt tagcgcctac catcagcttg 3780
cggaggaact cggtcatcga ccggctccga tcgctgccgt tttgcccccc tgcccagagc 3840
tcgaacaggg tctgctttat atgccccagg aactaactac gtccgacagc gtgcttacat 3900
ttgaattaac agatatagtg cactgccgta tggcggctcc aagccagcgc aaggcagtcc 3960
tgtcgaccct tgtcggcagg tatggccgtc gtacgaagtt gtatgaggcc gcccatgcgg 4020
acgttcgcgg atccctgaac cactttatcc ccgagctcgg ccccattagt gttaccacct 4080
gtgagcttta cgagcttgtg gaggctatgg tagagaaggg gcaggatggc tctgcggtct 4140
tggagcttga tctatgtagc cgtgatgtgt cgcgtataac attctttcag aaggattgta 4200
ataaatttac gacaggagag acaatagcac acggcaaagt tgggcagggg atatctgcgt 4260
ggagcaaaac gttttgtgcc ctatttggcc cttggttccg tgccattgag aaggagattc 4320
ttgcagcact tgctcccaat gttttctatg gtgatgcata tgaagacaca gtcctggccg 4380
ctgctgttgc tggggctcct ggctgcaagg tttttgagaa tgatttttct gagtttgata 4440

gcactcaaaa taacttctca cttggtttgg agtgtataat catggaggag tgcggcatgc 4500
cgcaatggat gatccgactt taccatcttg tccgctctgc ctgggtcctg caagccccaa 4560
aggagtccct gcggggtttc tggaagaagc actcaggcga gcctggcacc ttgctttgga 4620
acacggtctg gaacatggcg gtaatagcac actgttacga gttccgcgac ttgaaagtcg 4680
cggcgtttaa aggggatgac tctgttgtgc tctgtagtga ctaccggcag agccgcaatg 4740
cggccgcctt aatcactggt tgtgggctga agcttaaggt ggattttagg cccatcgggc 4800
tgtatgctgg tgttgttgtg gccccgggtc tcgggaccct tcctgatgtt gttaggttcg 4860
ctgggcggct ctcagagaaa aactgggggc cgggtccaga gagggctgag cagctacggc 4920
tggcagtttg tgatttcctg cgaaagttaa cgaatgtggc tcaagtctgt gtggatgttg 4980
tttcgcgggt ttatggtgtt agccccggct tagtacataa cctgattggg atgctccaga 5040
ctatcgccga tggcaaggcg cattttactg aaactgttaa acctgttttg gatctcacca 5100
attccattat acatcgggtg gaatgaataa catgttcttt tgctctgtgc atggagatgc 5160
caccatgcgc tctcgggctc ttctgtttct gctcttcgtg tttttgccta tgctgcccgc 5220
gccaccggcc ggtcagccgt ctggccgtcg ccgcgggcgg cgcagcggcg gtgccggcgg 5280
tggtttctgg ggtgaccggg ttgattctca gcccttcgcc ctcccctata ttcatccaac 5340
caaccccttc gcatctgaca ttccaaccgc agccggggct ggagctcgcc ctcggcaacc 5400
ggcccgtcca ctcggctccg cttggcgtga ccaatcccag cgccccaccg cttccgcccg 5460
tcgtcgacct gccccagctg gggctgcgcc gttgactgct gtggccccgg ctcctgatac 5520
cgctcctgtc cccgatgttg attctcgcgg cgcaatatta cgccgccaat ataatttatc 5580
tacatccccg cttacatcta ctattgctac tggcactaac cttgttttat atgctgcccc 5640
gttgagccct ctgctcccgc tccaagacgg aactaatact catattatgg ctactgaagc 5700
atcaaactac gcccagtatc gcgttgtccg cgccactatt cggtaccgtc cactcgtgcc 5760
gaatgccgtt ggcgggtacg ctatatctat ttctttctgg cctcagacga caactactcc 5820
gacatctgtc gatatgaatt ccatcacttc caccgatgtt cggattcttg tccagcctgg 5880
tattgcctct gaacttgtga tccctagcga gcgcctgcat taccgtaatc aaggctggcg 5940
ttcggttgag acctctggtg tcgcagagga ggaggcaacc tccggccttg ttatgctttg 6000
cattcatgga tcgcctgtga attcttatac taatacaccc tatactggtg cccttggttt 6060
gctcgatttt gcactcgagc ttgagttccg caatttgaca cctggtaata caaatacgcg 6120
cgtttcccga tactcgagca gcgcgcgcca caagcttcgc cgcgggcccg acggcactgc 6180

tgaattgact accactgctg ctacacgctt tatgaaagac cttcacttta cagggactaa 6240
tggtgttggt gaggttggtc gtggtatagc gctaactctg tttaatcttg ctgatacgct 6300
tcttggcggg cttccgacag aattaatttc gtcggctggt ggccagttgt tttactcccg 6360
ccccgtcgtc tcagccaatg gcgagccgac tgtgaaactt tacacctcag tcgagaatgc 6420
tcagcaggac aagggtatag ctattccaca tgatattgat cttggcgagt cccgagttgt 6480
tatccaggac tatgacaacc aacatgagca agaccgtcct actccctctc cagctccctc 6540
ccgtcctttt tctgtccttc gtgctaatga tgtgctttgg ctttcactta cagctgctga 6600
gtatgatcag actacttatg gctcttctac taaccctatg tatgtctctg ataccgtgac 6660
atttgtcaat gtggctactg gcgcccaggg ggtttcccgc tccttggact ggtctaaagt 6720
taccctcgat gggcgcccgc ttactactat tcagcagtac tctaagacct tctttgtcct 6780
accccttcgc ggtaagcttt ccttctggga ggctggtact actaaggctg gctatccgta 6840
caattataat accactgcta gtgaccagat cctaattgag aatgcggccg gtcatcgtgt 6900
ttgtatttca acttatacta ctaatcttgg ctctggccct gtttctatct ctgctgttgg 6960
tgtcctcgca cctcattctg cgctggccgc tctagaggac actgttgatt atcccgctcg 7020
tgctcatact tttgacgatt tctgccctga gtgccgtacg ctcggccttc agggctgtgc 7080
ttttcagtca actgtcgctg agctacagcg tcttaaaatg aaggtgggta aaacccggga 7140
gtattgattt attttgcttg tgccttcctt ctgttttgtt tcttttattt ctttcttctg 7200
cgcttcgcgc tccctggaaa aaaaaaaaa 7229

<210> 47
<211> 7228
<212> RNA
<213> Hepatitis E Virus Japan JKK-Sap(AB074917)

<400> 47
   ccatggaggc ccaccagttc ataaaggctc ctggcgtcac tactgctatt gagcaggcag 60
   ctctagcagc ggccaactcc gccttggcga atgctgtggt ggttcggcct ttcctatccc 120
   ggctacagac agagatactt attaacctga tgcagccccg gcagcttgtt ttccggcctg 180
   aagtcttgtg gaaccaccca attcagcgcg tgatccacaa cgagcttgag cagtactgcc 240
   gggctcgggc tggccgctgc cttgaggtgg gtgctcaccc acgttctatt aatgacaacc 300
   ccaatgttct gcaccgctgt ttcttgaaac ctgtcggccg cgatgtccag cggtggtaca 360
   ccgcccccac tcgtggcccc gcagcaaatt gccgccggtc ggccctccgt ggcctcccac 420
   ctgccgatcg aacctactgt tttaatggtt tttccggctg cacgtttgct gctgagacag 480
   ggatagccct ttactcactg cacgacctct ggcctgccga tgttgccgag gcaatggctc 540
   gccatggtat gacccggctg tatgcagctc tccacctccc cccggaggta ctgctccccc 600
   ctggcaccta tcataccact tcgtacctcc tcatccatga tggggaccgt gcagtgatta 660
   catatgaggg ggactctagt gctggataca accatgatgt gtccattctg cgcgcctgga 720
   tccgcactac taaggtcacc ggcgaccacc cgctggtaat tgagcgggtc cgggcggtgg 780
   gctgtcactt tgtgcttctt ctcacagctg cacctgaacc atcgccaatg ccctatgtcc 840
   cataccctcg ttccaccgag gtttatgttc ggtccatttt tggccctggt ggttcaccat 900
   ccctgtttcc aactgcctgc tctactaagt caacatttca tgccgtccct gtgcatatat 960
   gggacaggct tatgctcttc ggtgcgaccc tcgatgacca ggccttttgc tgttcaaggc 1020
   taatgacata ccttcgtggt attagttaca aagtcacagt tggcgctctt gtcgccaacg 1080
   agggctggaa tgcctccgaa gacgcattaa ctgctgtcat tactgcggct tatcttacca 1140
   tctgccatca gaggtacctc cgcacacagg ctatctcgaa agggatgaaa agattggagc 1200
   ttgagcatgc acagaagttt ataacacgtc tttatagctg gttgtttgaa aaatctggcc 1260
   gtgattacat ccccggccgt cagttgcagt tttacgccca gtgccgtcga tggctatctg 1320
   ctggtttcca ccttgatcct cgtgtgctcg tttttgatga agcggccccc tgccgctgtc 1380
   gtagcttgct tcgcaaagct gcccataaat tttgctgctt tatgcgatgg ctagggcagg 1440
   attgcacctg cttccttcag cctgttgagg ggagggtcgg tgaacagggt tatgataacg 1500
   aggcatttga ggggtcggat gtcgaccctg ctgaagaggc cactgtgagt atctctggct 1560
   cgtacattgt caccggcagc cagttgcagc ctctttacca ggcgctcggt attccttctg 1620
   accttgccgc ccgagcgggc cggctcactg ctaccgtcga agtttcagat accgacggcc 1680
   gtctcacttg taagaccact atgggcaata agaccttcac aacagttttt accgacggtg 1740
   cccagctgga ggctaatggg ccggagcagt atgtgctatc atttgatttg gctaaacaga 1800
   ctatggccgc cggcccgcat agccttagtt acgccttgac acctgcgggt cttgaggtgc 1860
   gtgttgtttc tgccggactc gactgtaggg ctgttttccc atctggggtt gcaaccccct 1920
   ctgcttccgg ggaggtgtcc gccttctgtt cggctttata caggtttaac cgctgtgtcc 1980
   agcggcattc tctcattggt ggcttgtggt attatcctga ggggctaatt ggtttgtttc 2040
   caccgttcgc ccctggccac acctgggagt cagccaatcc cttctgtggg gagagcaccc 2100
   tttatactcg cacctggtcg gtgtcgggct tttctagctg tttttctcca ttcgagcccc 2160
   ccgctctgga ctcaccaccc cccgccgagg ctgatacacc tatggctgtc gatgttccac 2220
   ccccagctac cttgacatta ccacaacctc cggctcctga acgggcagta cccccgcaag 2280
   atcttgccga tggcgatgtc gctcgggcct ctccgggtgt ctctgctgca ccccctgtgc 2340
   cggcgcaatc cgtgactgat ccacccgtgt cccgccggcg gttacttcat acttatcctg 2400
   atgggtcaaa agtgtacgct ggctccctct ttgagtctga gtgtacttgg ttggtcaatg 2460
   cgtccaatcc cggccaccgt cccggtggtg gcctttgcca tgcattttac cagcggttcc 2520
   cggagtcatt tgaccccgcc gagttcgtca tgtccgatgg gtttgcagct tataccttga 2580
   cgccccgacc tattatccat gctgttgctc ccgactatcg ggtagaacat aaccccaaga 2640
   ggctcgaggc tgcttatcga gagacgtgct cccgccgtgg cacggctgct taccccctgc 2700
   ttggtgccgg catatataag gtgcctgttg ggcttagttt tgatgcctgg gaacgcaacc 2760
   atcggcccgg ggatgagttg taccttactg agccggctat agcttggttc gaggctaacc 2820
   gacccactct ccccgcgctc actatcactg aggacacggc acgaacagct aatctggcgt 2880
   tggagctaga ttcggccact gaggtcggtc gggcgtgtgc tggttgccgt gttgagccag 2940
   gcgtcgttca ctaccagttc acggcgggtg tccctggttc cggcaagtca cggtcaattc 3000
   agcagggcga ggtggatgtg gtggtagtac caactcgtga gctgcgtaac tcatggcgac 3060
   gccgcgggtt tgcagcctac acaccccaca ctgcggcccg tgttactcgt ggccgtaggg 3120
   ttgtcattga tgaggccccg tcacttccac cacatctgct cttgctgcac atgcagcggg 3180
   cctcgtcggt ccaccttctt ggcgacccca accaaatccc tgccattgat ttcgagcatg 3240
   ctggtcttgt acccgcaatc cggcctgagc ttgtaccaac aaagtggtgg catcttaccc 3300
   acaggtgccc ggcagatgtt tgtgagctaa ttcgcggcgc gtaccctaag attcagacag 3360
   cgagccgtgt ttttcgctcc ctgttttggg gggagccccc tgtcggccaa aaattagtgt 3420
   tcactcaggc ggcgaaggcc gctaaccctg gtgcgattac ggtccatgag gcccagggcg 3480
   ctacattcac tgagactact attattgcta cggcggatgc tcgcgggctg attcagtcat 3540
   ctagggctca cgctatcgtg gccctaaccc gtcatacgga gaaatgtgtg gtcgttgatg 3600
   ccccgggact ccttcgtgag gtcggtatct ctgacgccat tgttaataac tttttccttt 3660
   ctggtggcca gattggtcag caccgtccat cagtcatacc gcgtggcact atcaacaaca 3720
   atgttgatac gcttgatgca ttcccaccct cttgccagtt cagcgcttac caccagcttg 3780
   cggaagagct cggccatcga ccggccccga tcgctgccgt tctgcccccc tgtcctgaac 3840
   ttgaacaggg tctactttac atgcctcagg aattgaccac gtctgatagt gtgcttacgt 3900
   ttgaattgac agatatagtg cactgtcgta tggcggcacc tagtcagcgt aaggcggtcc 3960
   tgtcgactct tgtcggcagg tacggccgcc gtacgaagtt gtatgaggct gcccatgcag 4020
   atgtccgtgg gtctctaaac cattttatcc ctgagctcgg ccctgttagt gttaccactt 4080
   gtgaacttta cgagcttgtg gaggccatgg tggagaaagg ccaggatggt tctgcggttc 4140
   tggagctcga tttatgtagc cgtgatgtgt ctcgtataac attcttccag aaggattgta 4200
   ataagtttac aacgggcgag acaatagcac atggcaaagt tggccagggg atatctgcat 4260
   ggagtaaaac cttttgtgct ttgttcggcc catggttccg cgccattgag aaggagatcc 4320
   ttgctgtact tgctcccaat gtattctatg gtgatgcata cgaagataca gttctggccg 4380
   ccgcagtcgc cggagcccct ggttgcaagg ttttcgagaa tgatttctca gagtttgata 4440
   gcactcaaaa taatttttca cttgggctgg agtgtataat catggaggag tgcggcatgc 4500
   cgcagtggat gatccggctt tatcaccttg ttcgctctgc ttgggttttg caagctccaa 4560
   aggagtctct gcgggggttc tggaagaagc actcaggcga gcctggcacc ttgctttgga 4620
   atactgtctg gaacatggcg gtcatagccc attgttatga gttccgcgac ttaaaagtcg 4680
   ccgcatttaa aggagatgac tctgttgtgc tttgtagtga ttatcggcaa agccgcgatg 4740
   cggccgccct aatcgctggc tgtgggttga aacttaaggt ggattttagg cccattgggc 4800
   tgtatgctgg tgttgttgtg gccccgggtc tcgggaccct tcctgatgtt gttaggtttg 4860
   ctgggcggct ctcggagaaa aactgggggc cgggttcaga gagggcagaa cagctacggc 4920
   tggctgtctg tgattttctg cgaaagttaa cgaatgtggc tcaagtctgt gtggacgttg 4980
   tttcgcaggt ttatggtgtt agccccggtt tggtacataa cctgattggg atgctccaga 5040
   ccattgctga tggcaaggcg cactttacag agacagttaa acctgtttta gacctcacca 5100
   attctattat acatcgggtg gaatgaataa catgttcttt tgctctgtgc atggagatgc 5160
   caccatgcgc tctcgggctt ttctgttttt gttcctcgtg cttctgccta tgctgcccgc 5220
   gccaccggcc ggtcagccgt ctggccgtcg ccgcgggcgg cgcagcggcg gtgccggcgg 5280
   tggtttctgg ggtgaccggg ttgattctca gcccttcgcc ctcccctata ttcatccaac 5340
   caaccccttc gcatctgaca ttccagccgc agccggggct ggagctcgcc ctcggcagcc 5400
   agcccgtcca ctcggctccg cttggcgtga ccaatcccag cgccccgcca cttccgcccg 5460
   tcgtcgatct gccccagctg gggcttcgcc gctgactgct gtggccccgg ccccagatac 5520
   tgctcctgtt cccgatgtcg attctcgcgg cgctatatta cgccgccagt ataatttatc 5580
   aacatccccg ctaacatcta ctattgccac tggtactaac cttgttctat atgctgcccc 5640
   gctgagccct ttgcttccgc tccaagatgg aactaacact cacattatgg ccactgaagc 5700
   atcaaattat gcccagtacc gtgttgtccg cgccaccatc cggtaccgtc cgcttgtgcc 5760
   gaacgctgtc ggcggatacg ctatatctat ctctttctgg cctcagacaa ctaccacccc 5820
   gacatctgtg gatatgaatt ctatcacctc cacggatgtc cgaatccttg tccagcctgg 5880
   catcgcttca gaacttgtga tccctagtga gcgcctgcat tatcgtaacc aaggctggcg 5940
   ctcggttgag acctctggtg ttgcggagga ggaggcgacc tccggccttg tcatgctttg 6000
   catccatgga tcacctgtga attcttacac caatacgcct tatactggtg cccttggctt 6060
   gcttgatttc gcactcgagc tcgagttccg caatttgaca cccggtaaca cgaacacacg 6120
   tgtttcccgc tactcgagta gtgcgcgcca caagctacgc cgagggcctg atggcactgc 6180
   tgagttaact accactgctg ctacacgctt tatgaaggac cttcatttta cagggactaa 6240
   tggagttggt gaagtcggtc gtggtatagc gctaactctg ttcaatcttg ctgacacgct 6300
   tctcggcggg ctcccgacag aattgatttc gtcggctggt ggtcagttgt tttactctcg 6360
   ccccgtcgtc tcagccaatg gcgagccgac agtgaagctt tatacttcag tcgagaacgc 6420
   tcagcaggat aagggtatag ctatcccaca tgatattgac cttggtgagt cccgtgttgt 6480
   cattcaggat tacgataacc aacatgagca ggaccgtccc accccttctc ctgctccctc 6540
   tcggcccttt tctgtccttc gtgctaatga tgtgctatgg ctttcactta cagcagctga 6600
   gtatgatcag actacctatg gctcctctac taaccccatg tatgtctctg atactgtgac 6660
   atttgttaat gtggctactg gtgcccaggg ggtatctcgc tctctggact ggtctaaagt 6720
   tacccttgat gggcgcccac ttatgactat ccagcagtat tctaagacct tctttgtcct 6780
   gcctctccgt ggtaagctct cctttgggga ggctggtact actaaggccg gctaccctta 6840
   taattataat actaccgcca gtgaccagat tttaattgag aatgcagctg gtcaccgtgt 6900
   atgcatttca acctacacta ctaatcttgg atctggccct gtttctattt ctgccgtcgg 6960
   tgtcctcgca cctcactctg cgctggccgc tttagaggac actgttgact atcctgcccg 7020
   tgctcatact tttgatgatt tttgccctga gtgccgtgca ctcggccttc agggttgtgc 7080
   tttccaatcg actgttgctg agctacagcg ccttaaaatg aaggtgggta aaacccggga 7140
   gtactgattt attctacttg taccttcctt ctgttctgtt cttttatttt ycttttctgc 7200
   gtyccgcgct ccctggaaaa aaaaaaaa 7228

<210> 48

<211> 7230

<212> RNA

<213> Hepatitis E Virus Japan JRA1(AP003430)

<400> 48

ggcagaccac gtatgtggtc gatgccatgg aggcccacca gttcattaag gctcctggca 60

ttactactgc catagagcag gctgctctgg ctgcggccaa ctccgccttg gcgaatgctg 120

tggtggttcg gccgtttttg tctcgcgtac aaaccgagat ccttattaat ttgatgcaac 180

cccggcagtt ggttttccgc cctgaggtgc tttggaacca tcctattcag agggttatac 240

acaatgaatt agaacagtac tgccgggccc gggccggtcg ttgcctggag attggggctc 300

atccaagatc tattaatgat aaccccaatg ttttgcaccg gtgttttctc aggccggtcg 360

gtagggacgt ccagcgctgg tattctgccc ccacccgcgg ccctgcagcc aactgccgcc 420

gctctgcatt gcgtggcctc ccccctgtcg accgcactta ctgttttgat ggattttcca 480

gttgtgcttt cgccgcagag accggcgtgg ccctttactc tctacatgac ctttggccag 540

ctgatgttgc ggaggctatg gcccgccatg gaatgacacg tctttatgct gcactccacc 600

tccctcccga ggtgttgtta ccacccggta cttaccacac aacctcgtat cttctgatcc 660

atgacggcaa ccgtgctgtt gtgacttatg aaggtgatac tagtgcaggt tacaaccatg 720

acgtttccat acttcgtgca tggattcgca cgactaaaat agttggtgac catccgttgg 780

ttatagagcg tgtgcgggct attggttgtc attttgtgct gctgctcacc gcggcccctg 840

agccatcgcc catgccttac gtcccttacc cccgttccac ggaggtgtat gtccgatcta 900

tatttggccc tggtggctcc ccatccttgt ttccgtcagc ttgctctaca aaatctacct 960

tccatgctgt tccagttcac atatgggacc ggcttatgct ttttggtgcc accttggacg 1020

accaggcgtt ttgctgctca cggctcatga catacctccg tggcattagc tacaaagtca 1080

ctgtcggtgc gcttgttgct aatgaggggt ggaatgcctc tgaagatgcc ctcaccgcag 1140

taatcactgc agcctacttg accatctgtc accagcgcta cctccgtacc caggcgatat 1200

ctaagggtat gcgccggttg gaggttgagc acgcccagaa gtttattaca agactttaca 1260

gctggttatt tgagaagtct ggtcgtgact acatccccgg ccgccaactt cagttctatg 1320
cccagtgccg gcggtggcta tctgcgggct ttcacctaga ccccagagtg ctcgtctttg 1380
atgagtcagt gccctgccgc tgtaggactt tcttaaagaa ggttgcgggt aaattctgtt 1440
gcttcatgcg gtggttaggg caggagtgta cttgtttctt agaaccagct gagggcttag 1500
ttggcgatca aggccatgat aatgaggctt atgagggttc tgaggttgac caggccgagc 1560
ctgtacacct tgatgtgtcg gggacctatg ctgtccacgg gcaccagctt gaggccctct 1620
acagggcact caacattcca cacgatattg ctgctcgagc tgcccgatta acggctactg 1680
ttgagctcgc tgcaggcccc gaccgtctgg agtgccgcac cgtgctcggg aacaagactt 1740
tccggacgac ggtgactgat ggcgcccatc tagaagcgaa cggccctgag caatatgttc 1800
tgtcgttcga tgcctcccgc cagtctatgg gggccgggtc ccatagcctc acttacgagc 1860
ttacacccgc cggcttgcag gttaagatct catctaatgg cctagactgc actgccgtat 1920
tcccccctgg tggcgcccct agcgcgccgc cgggggaggt ggcggctttt tgcagcgccc 1980
tctacaggta taacagattc acccagcggc attcgctgac cggcggtttg tggctacatc 2040
ccgaggggtt gctaggcgtt ttcccccctt tttcccctgg gcacatttgg gagtctgcca 2100
accccttctg cggtgagggt accttgtata ctcgcacctg gtctacatct ggtttttcta 2160
gtgacttttc ccctcctgag gcggccgccc ctgcaccggc tgccgcccca gggtcgtcct 2220
cccccactcc accagtcagt gatatttggg tgttaccacc gctttcagag gagccccagg 2280
tgggtgcgcc gcctgcacct cccacccccg agcctgctcg gctgccctgc cccactaaac 2340
ctaacacccc cgtgcgtaag ccaacggcac cgccgccttc tcgcacccgc cgccttcttt 2400
acacctatcc tgatggtgct aaggtgtatg cggggtcact gtttgagtca gattgtgatt 2460
ggctggtgaa tgcctctaac ccgggccatc gtcctggggg tggcctctgt cacgcctttt 2520
accaacgttt tcccgaggcg ttctatccaa ctgaatttat tatgcgtgag ggtcttgcag 2580
catacacctt gaccccgcgc cctattatcc atgcagtggc ccccgactat agggttgagc 2640
agaatccaaa gaggcttgag gcagcgtacc gagaaacttg ctcccgccgc ggcaccgctg 2700
cctatccact tcttggctca ggtatatacc aggtccctgc tggtcttagt tttgatgcct 2760
gggagcgtaa ccatcgccct ggcgatgagc tttacctgac tgaacccgct gcagcctggt 2820
ttgaagctaa taagccaacg cagccagcac ttacgataac ggaagataca gctcgcacgg 2880
ccaacctagc attagagatc gatgctgcca cagacgttgg ccgtgcttgt gccggctgca 2940
ctattagccc cgggattgtg cattatcagt tcactgccgg ggtcccaggc tcaggcaaat 3000

caaggtccat acagcagggg gatgtcgatg ttgtggttgt ccccacccgg gaacttcgca 3060
atagctggcg ccgtcggggc tttgcggcct ttacacccca cacggcggcc cgtgttacta 3120
taggccgccg tgttgttatt gacgaggccc catcccttcc accgcacttg ttgctgttac 3180
acatgcagcg ggcctcctca gtccatctcc tcggtgaccc aaatcagatc cctgccatcg 3240
acttcgagca tgccggcctg gtccccgcaa ttcgccctga gcttgcacca acgagctggt 3300
ggcatgttac gcatcgctgt ccggccgatg tatgtgagct catacgcgga gcctacccta 3360
aaatccagac cacgagccgt gtgctacggt ctctgttctg gaatgagccg gctattggcc 3420
agaagttggt cttcacgcag gccgctaaag ctgccaaccc tggtgcaatt acggtccatg 3480
aagcccaggg tgctaccttc acagagacca caattatagc cacggctgat gccaggggtc 3540
ttattcagtc atcccgggcc catgctatag ttgcacttac ccgccacaca gagaagtgcg 3600
ttattttgga tgcccctggt ttgttgcgcg aggtcggcat atcagatgta attgttaaca 3660
actttttcct tgctggcgga gaggtgggcc atcaccgccc ctctgtgata cctcgtggca 3720
atcctgaccg gaaccttgac accctacagg ccttcccgcc atcctgccaa attagtgctt 3780
accatcagtt ggctgaggag ttaggccatc gcccggctcc tgttgctgcc gttttgcccc 3840
cctgccccga gctcgagcag ggcttattgt atatgccaca ggagcttaca gtgtccgata 3900
gtgtgttggt ttttgagctc actgacatag tccattgccg catggctgcc ccaagtcagc 3960
ggaaggccgt cctctcaaca cttgtgggga ggtatggccg caagacgaaa ttgtatgagg 4020
cagcccattc agatgttcga gagtctctag ctaggttcat tcccactatc ggacctgtcc 4080
aggccaccac gtgtgagtta tatgaactgg ttgaggccat ggtggaaaag ggccaggacg 4140
gctctgccgt cctggagctt gatctatgta atcgtgatgt ttcgcgcatt acattctttc 4200
agaaagactg taataagttt acaactggtg agaccattgc ccatggtaag gttggccagg 4260
gcatatcggc ctggagtaag accttttgcg ccctgtttgg tccgtggttt cgtgccattg 4320
aaaaagaaat actagccctg ctcccgccta atatcttcta cggcgacgcc tacgaggagt 4380
cggtgtttgc cgcggccgtg tccggggcgg ggtcttgcat ggtatttgaa aatgactttt 4440
cggaatttga tagtactcag aacaatttct cccttggcct tgagtgtgtg gttatggaag 4500
agtgtggtat gccccaatgg ctgatcagat tgtatcacct ggtccggtca gcctggattc 4560
tgcaggcgcc aaaggagtct cttaaaggtt tctggaagaa gcattctggt gagcctggta 4620
cccttctctg gaacaccatc tggaacatgg cgatcatagc ccattgctat gagtttcgcg 4680
atttccgggt cgccgctttt aagggtgatg actcggtagt cctctgtagt gattaccgac 4740

agagtcgtaa cgcggcagcc ttaatcgcag gttgtgggct caagttgaag gttgactatc 4800
gccctattgg gctgtacgct ggtgtggtgg tggcccctgg cttggggaca ctgcctgatg 4860
tagtgcgatt tgctggccgg ctgtccgaaa agaattgggg ccctggccca gagcgtgctg 4920
agcagctgcg tcttgctgtc tgtgacttcc ttcgagggtt aacgaatgtt gcgcaggttt 4980
gtgttgatgt tgtgtcccgt gtttatggag tcagccccgg gctggtacat aaccttattg 5040
gcatgctgca gaccattgct gatggcaagg cccactttac agagtcaatt aaacctgtgc 5100
ttgaccttac aaattctatt atacagcggg tggaatgaat aacatgtctt gtgcatcgcc 5160
catgggatca ccatgcgccc tagggctgtt ctgttgttgc tcttcgtgct tctgcctatg 5220
ctgcccgcgc caccggccgg ccagccgtct ggccgccgtc gtgggcggcg cagcggcggt 5280
gccggcggtg gtttctgggg tgacagggtt gattctcagc ccttcgccct cccctatatt 5340
catccaacca accccttcgc cgccgatgtc gtttcacaat ccggggctgg agctcgccct 5400
cgacagccgc cccgccccct tggctccgct tggcgcgacc agtcccagcg ccccaccgct 5460
gccccccgtc gtcgacctgc cccagctggg gctgcgccgc tgactgctac atcaccagct 5520
cctgacacag ctcctgtgcc tgatgtcgac tcacgcggcg ctattttgcg tcggcagtat 5580
aatctgtcca cgtccccgct tacgtcatct gtcgcctcgg gtactaattt ggttctctat 5640
gccgccccat tgaacccact cctacccctc caggatggca ccaacaccca catcatggct 5700
accgaggcat ctaattatgc ccagtaccgg gtggttcgag ctacaatccg ttatcgcccg 5760
ttggtaccaa atgctgttgg tggttatgcc atctctattt ccttttggcc tcagacaacc 5820
actaccccca cctctgttga tatgaattct attacctcta ctgatgttag gatcttagtc 5880
cagcccggta ttgcctccga gttggtcatt cccagtgagc gtcttcatta tcgtaatcaa 5940
ggctggcgct ctgtcgagac cacgggtgtc gccgaggagg aggctacctc tggcctggta 6000
atgctttgca ttcatgggtc tcctgtcaat tcttacacaa atacacctta tactggagca 6060
ctggggcttc ttgattttgc attagagctt gagtttagga atttgacacc tgggaacact 6120
aatacccgtg tgtcccgata taccagcaca gcccgccacc ggctgcgccg tggtgccgac 6180
gggaccgctg agcttaccac tacggcggcc acgcgtttta tgaaggacct gcatttcacc 6240
ggtacgaatg gagttggtga ggtgggccga ggcatagccc tgacactgtt caatcttgct 6300
gatacgcttc tcggtggtct gccgacagaa ttgatttcgt cggctggggg ccagttgttc 6360
tactcccgcc ctgtcgtctc agccaatggc gaaccgactg taaagttata tacatctgtt 6420
gagaatgcac agcaggataa gggtatcacc atcccacacg atatagacct cggtgattct 6480

cgtgtggtca tccaggacta tgataaccag cacgagcagg accgacctac cccctcaccc 6540
gccccctctc gtccattctc ggttcttcgc gctaatgatg ttctgtggct ctctcttacc 6600
gctgccgagt atgaccagac tacgtatggg tcatctacca accccatgta tgtctcggac 6660
acagtcacat ttgttaacgt ggccactggt gctcaggctg ttgctcgctc ccttgattgg 6720
tctaaagtta ccttggacgg tcgccccctc actactattc agcagtattc taagacattc 6780
tacgttctcc cgcttcgtgg aaagttgtct tttgggaggc ctggcacgac caaggctggt 6840
tatccttata attataatac aactgctagt gatcagattt taatagagaa cgcagctggc 6900
catcgtgtcg ctatctctac ttacactacc agcctgggcg ccggccccac ttctatctct 6960
gcggtcggtg tgttagcccc gcactcagcc cttgctgttc ttgaggatac cgccgattac 7020
cccgctcgtg cccatacttt tgatgacttc tgcccggaat gtcgcaccct tggtttgcag 7080
ggctgcgctt tccagtctac tattgctgag cttcagcgcc ttaaaatgaa ggtaggtaaa 7140
acccgggagt tctaattaat tcgtcctgta ccccccttcgt agtttctttt tgctttattt 7200
ctcttttctg ctttccgcgc tccctggaaa 7230

<210> 49
<211> 7180
<212> RNA
<213> Hepatitis E Virus Mexico(M74506)

<400> 49
gccatggagg cccaccagtt cattaaggct cctggcatca ctactgctat tgagcaagca 60
gctctagcag cggccaactc cgcccttgcg aatgctgtgg tggtccggcc tttcctttcc 120
catcagcagg ttgagatcct tataaatctc atgcaacctc ggcagctggt gtttcgtcct 180
gaggtttttt ggaatcaccc gattcaacgt gttatacata atgagcttga gcagtattgc 240
cgtgctcgct cgggtcgctg ccttgagatt ggagcccacc cacgctccat taatgataat 300
cctaatgtcc tccatcgctg ctttctccac cccgtcggcc gggatgttca gcgctggtac 360
acagccccga ctaggggacc tgcggcgaac tgtcgccgct cggcacttcg tggtctgcca 420
ccagccgacc gcacttactg ttttgatggc tttgccggct gccgttttgc cgccgagact 480

ggtgtggctc tctattctct ccatgacttg cagccggctg atgttgccga ggcgatggct 540
cgccacggca tgacccgcct ttatgcagct ttccacttgc ctccagaggt gctcctgcct 600
cctggcacct accggacatc atcctacttg ctgatccacg atggtaagcg cgcggttgtc 660
acttatgagg gtgacactag cgccggttac aatcatgatg ttgccaccct ccgcacatgg 720
atcaggacaa ctaaggttgt gggtgaacac cctttggtga tcgagcgggt gcggggtatt 780
ggctgtcact ttgtgttgtt gatcactgcg gcccctgagc cctccccgat gccctacgtt 840
ccttacccgc gttcgacgga ggtctatgtc cggtctatct ttgggcccgg cgggtccccg 900
tcgctgttcc cgaccgcttg tgctgtcaag tccacttttc acgccgtccc cacgcacatc 960
tgggaccgtc tcatgctctt tggggccacc ctcgacgacc aggccttttg ctgctccagg 1020
cttatgacgt accttcgtgg cattagctat aaggtaactg tgggtgccct ggtcgctaat 1080
gaaggctgga atgccaccga ggatgcgctc actgcagtta ttacggcggc ttacctcaca 1140
atatgtcatc agcgttattt gcggacccag gcgatttcta agggcatgcg ccggcttgag 1200
cttgaacatg ctcagaaatt tatttcacgc ctctacagct ggctatttga gaagtcaggt 1260
cgtgattaca tcccaggccg ccagctgcag ttctacgctc agtgccgccg ctggttatct 1320
gccgggttcc atctcgaccc ccgcacctta gtttttgatg agtcagtgcc ttgtagctgc 1380
cgaaccacca tccggcggat cgctggaaaa ttttgctgtt ttatgaagtg gctcggtcag 1440
gagtgttctt gtttcctcca gcccgccgag gggctggcgg gcgaccaagg tcatgacaat 1500
gaggcctatg aaggctctga tgttgatact gctgagcctg ccaccctaga cattacaggc 1560
tcatacatcg tggatggtcg gtctctgcaa actgtctatc aagctctcga cctgccagct 1620
gacctggtag ctcgcgcagc ccgactgtct gctacagtta ctgttactga aacctctggc 1680
cgtctggatt gccaaacaat gatcggcaat aagacttttc tcactacctt tgttgatggg 1740
gcacgccttg aggttaacgg gcctgagcag cttaacctct cttttgacag ccagcagtgt 1800
agtatggcag ccggcccgtt ttgcctcacc tatgctgccg tagatggcgg gctggaagtt 1860
catttttcca ccgctggcct cgagagccgt gttgttttcc cccctggtaa tgccccgact 1920
gccccgccga gtgaggtcac cgccttctgc tcagctcttt ataggcacaa ccggcagagc 1980
cagcgccagt cggttattgg tagtttgtgg ctgcaccctg aaggtttgct cggcctgttc 2040
ccgccctttt cacccgggca tgagtggcgg tctgctaacc cattttgcgg cgagagcacg 2100
ctctacaccc gcacttggtc cacaattaca gacacaccct taactgtcgg gctaatttcc 2160
ggtcatttgg atgctgctcc ccactcgggg gggccacctg ctactgccac aggccctgct 2220

gtaggctcgt ctgactctcc agaccctgac ccgctacctg atgttacaga tggctcacgc 2280
ccctctgggg cccgtccggc tggccccaac ccgaatggcg ttccgcagcg ccgcttacta 2340
cacacctacc ctgacggcgc taagatctat gtcggctcca ttttcgagtc tgagtgcacc 2400
tggcttgtca acgcatctaa cgccggccac cgccctggtg gcgggctttg tcatgctttt 2460
tttcagcgtt accctgattc gtttgacgcc accaagtttg tgatgcgtga tggtcttgcc 2520
gcgtataccc ttacaccccg gccgatcatt catgcggtgg ccccggacta tcgattggaa 2580
cataacccca agaggctcga ggctgcctac cgcgagactt gcgcccgccg aggcactgct 2640
gcctatccac tcttaggcgc tggcatttac caggtgcctg ttagtttgag ttttgatgcc 2700
tgggagcgga accaccgccc gtttgacgag ctttacctaa cagagctggc ggctcggtgg 2760
tttgaatcca accgccccgg tcagcccacg ttgaacataa ctgaggatac cgcccgtgcg 2820
gccaacctgg ccctggagct tgactccggg agtgaagtag gccgcgcatg tgccgggtgt 2880
aaagtcgagc ctggcgttgt gcggtatcag tttacagccg gtgtccccgg ctctggcaag 2940
tcaaagtccg tgcaacaggc ggatgtggat gttgttgttg tgcccactcg cgagcttcgg 3000
aacgcttggc ggcgccgggg ctttgcggca ttcactccgc acactgcggc ccgtgtcact 3060
agcggccgta gggttgtcat tgatgaggcc ccttcgctcc ccccacactt gctgctttta 3120
catatgcagc gtgctgcatc tgtgcacctc cttggggacc cgaatcagat ccccgccata 3180
gattttgagc acaccggtct gattccagca atacggccgg agttggtccc gacttcatgg 3240
tggcatgtca cccaccgttg ccctgcagat gtctgtgagt tagtccgtgg tgcttaccct 3300
aaaatccaga ctacaagtaa ggtgctccgt tccctttctt ggggagagcc agctgtcggc 3360
cagaagctag tgttcacaca ggctgctaag gccgcgcacc ccggatctat aacggtccat 3420
gaggcccagg gtgccacttt taccactaca actataattg caactgcaga tgcccgtggc 3480
ctcatacagt cctcccgggc tcacgctata gttgctctca ctaggcatac tgaaaaatgt 3540
gttatacttg actctcccgg cctgttgcgt gaggtgggta tctcagatgc cattgttaat 3600
aatttcttcc tttcgggtgg cgaggttggt caccagagac catcggtcat tccgcgaggc 3660
aaccctgacc gcaatgttga cgtgcttgcg gcgtttccac cttcatgcca aataagcgcc 3720
ttccatcagc ttgctgagga gctgggccac cggccggcgc cggtggcggc tgtgctacct 3780
ccctgccctg agcttgagca gggccttctc tatctgccac aggagctagc ctcctgtgac 3840
agtgttgtga catttgagct aactgacatt gtgcactgcc gcatggcggc ccctagccaa 3900
aggaaagctg ttttgtccac gctggtaggc cggtatggca gacgcacaag gctttatgat 3960

gcgggtcaca ccgatgtccg cgcctccctt gcgcgcttta ttcccactct cgggcgggtt 4020
actgccacca cctgtgaact ctttgagctt gtagaggcga tggtggagaa gggccaagac 4080
ggttcagccg tcctcgagtt ggatttgtgc agccgagatg tctcccgcat aacctttttc 4140
cagaaggatt gtaacaagtt cacgaccggc gagacaattg cgcatggcaa agtcggtcag 4200
ggtatcttcc gctggagtaa gacgttttgt gccctgtttg gcccctggtt ccgtgcgatt 4260
gagaaggcta ttctatccct tttaccacaa gctgtgttct acggggatgc ttatgacgac 4320
tcagtattct ctgctgccgt ggctggcgcc agccatgcca tggtgtttga aaatgatttt 4380
tctgagtttg actcgactca gaataacttt tccctaggtc ttgagtgcgc cattatggaa 4440
gagtgtggta tgccccagtg gcttgtcagg ttgtaccatg ccgtccggtc ggcgtggatc 4500
ctgcaggccc caaaagagtc tttgagaggg ttctggaaga agcattctgg tgagccgggc 4560
agcttgctct ggaatacggt gtggaacatg gcaatcattg cccattgcta tgagttccgg 4620
gacctccagg ttgccgcctt caagggcgac gactcggtcg tcctctgtag tgaataccgc 4680
cagagcccag gcgccggttc gcttatagca ggctgtggtt tgaagttgaa ggctgacttc 4740
cggccgattg ggctgtatgc cggggttgtc gtcgccccgg ggctcggggc cctacccgat 4800
gtcgttcgat tcgccggacg gctttcggag aagaactggg ggcctgatcc ggagcgggca 4860
gagcagctcc gcctcgccgt gcaggatttc ctccgtaggt taacgaatgt ggcccagatt 4920
tgtgttgagg tggtgtctag agtttacggg gtttccccgg gtctggttca taacctgata 4980
ggcatgctcc agactattgg tgatggtaag gcgcatttta cagagtctgt taagcctata 5040
cttgacctta cacactcaat tatgcaccgg tctgaatgaa taacatgtgg tttgctgcgc 5100
ccatgggttc gccaccatgc gccctaggcc tcttttgctg ttgttcctct tgtttctgcc 5160
tatgttgccc gcgccaccga ccggtcagcc gtctggccgc cgtcgtgggc ggcgcagcgg 5220
cggtaccggc ggtggtttct ggggtgaccg ggttgattct cagcccttcg caatccccta 5280
tattcatcca accaacccct ttgccccaga cgttgccgct gcgtccgggt ctggacctcg 5340
ccttcgccaa ccagcccggc cacttggctc cacttggcga gatcaggccc agcgcccctc 5400
cgctgcctcc cgtcgccgac ctgccacagc cggggctgcg gcgctgacgg ctgtggcgcc 5460
tgcccatgac acctcacccg tcccggacgt tgattctcgc ggtgcaattc tacgccgcca 5520
gtataatttg tctacttcac ccctgacatc ctctgtggcc tctggcacta atttagtcct 5580
gtatgcagcc ccccttaatc cgcctctgcc gctgcaggac ggtactaata ctcacattat 5640
ggccacagag gcctccaatt atgcacagta ccgggttgcc cgcgctacta tccgttaccg 5700

gcccctagtg cctaatgcag ttggaggcta tgctatatcc atttctttct ggcctcaaac 5760
aaccacaacc cctacatctg ttgacatgaa ttccattact tccactgatg tcaggattct 5820
tgttcaacct ggcatagcat ctgaattggt catcccaagc gagcgccttc actaccgcaa 5880
tcaaggttgg cgctcggttg agacatctgg tgttgctgag gaggaagcca cctccggtct 5940
tgtcatgtta tgcatacatg gctctccagt taactcctat accaataccc cttataccgg 6000
tgcccttggc ttactggact ttgccttaga gcttgagttt cgcaatctca ccacctgtaa 6060
caccaataca cgtgtgtccc gttactccag cactgctcgt cactccgccc gaggggccga 6120
cgggactgcg gagctgacca caactgcagc caccaggttc atgaaagatc tccactttac 6180
cggccttaat ggggtaggtg aagtcggccg cgggatagct ctaacattac ttaaccttgc 6240
tgacacgctc ctcggcgggc tcccgacaga attaatttcg tcggctggcg ggcaactgtt 6300
ttattcccgc ccggttgtct cagccaatgg cgagccaacc gtgaagctct atacatcagt 6360
ggagaatgct cagcaggata agggtgttgc tatcccccac gatatcgatc ttggtgattc 6420
gcgtgtggtc attcaggatt atgacaacca gcatgagcag gatcggccca ccccgtcgcc 6480
tgcgccatct cggccttttt ctgttctccg agcaaatgat gtactttggc tgtccctcac 6540
tgcagccgag tatgaccagt ccacttacgg gtcgtcaact ggcccggttt atatctcgga 6600
cagcgtgact ttggtgaatg ttgcgactgg cgcgcaggcc gtagcccgat cgcttgactg 6660
gtccaaagtc accctcgacg ggcggcccct cccgactgtt gagcaatatt ccaagacatt 6720
ctttgtgctc ccccttcgtg gcaagctctc cttttgggag gccggcacaa caaaagcagg 6780
ttatccttat aattataata ctactgctag tgaccagatt ctgattgaaa atgctgccgg 6840
ccatcgggtc gccatttcaa cctataccac caggcttggg gccggtccgg tcgccatttc 6900
tgcggccgcg gttttggctc cacgctccgc cctggctctg ctggaggata cttttgatta 6960
tccggggcgg gcgcacacat ttgatgactt ctgccctgaa tgccgcgctt taggcctcca 7020
gggttgtgct ttccagtcaa ctgtcgctga gctccagcgc cttaaagtta aggtgggtaa 7080
aactcgggag ttgtagttta tttggctgtg cccacctact tatatctgct gatttccttt 7140
atttcctttt tctcggtccc gcgctccctg aaaaaaaaaa 7180

<210> 50
<211> 1709
<212> PRT

<213> Hepatitis E Virus JKN-Sap (ORF1)

<400> 50

Met Glu Ala His Gln Phe Ile Lys Ala Pro Gly Ile Thr Thr Ala Ile
1               5                   10                  15

Glu Gln Ala Ala Leu Ala Ala Ala Asn Ser Ala Leu Ala Asn Ala Val
            20                  25                  30

Val Val Arg Pro Phe Leu Ser Arg Val Gln Thr Glu Ile Leu Ile Asn
        35                  40                  45

Leu Met Gln Pro Arg Gln Leu Val Phe Arg Pro Glu Val Leu Trp Asn
    50                  55                  60

His Pro Ile Gln Arg Val Ile His Asn Glu Leu Glu Gln Tyr Cys Arg
65                  70                  75                  80

Ala Arg Ala Gly Arg Cys Leu Glu Val Gly Ala His Pro Arg Ser Ile
                85                  90                  95

Asn Asp Asn Pro Asn Val Leu His Arg Cys Phe Leu Arg Pro Val Gly
            100                 105                 110

Arg Asp Val Gln Arg Trp Tyr Ser Ala Pro Thr Arg Gly Pro Ala Ala
        115                 120                 125

Asn Cys Arg Arg Ser Ala Leu Arg Gly Leu Pro Pro Ala Asp Arg Thr
    130                 135                 140

Tyr Cys Phe Asp Gly Phe Ser Arg Cys Ala Phe Ala Ala Glu Thr Gly
145                 150                 155                 160

Val Ala Leu Tyr Ser Leu His Asp Leu Trp Pro Ala Asp Val Ala Glu
                165                 170                 175

Ala Met Ala Arg His Gly Met Thr Arg Leu Tyr Ala Ala Leu His Leu
            180                 185                 190

Pro Pro Glu Val Leu Leu Pro Pro Gly Thr Tyr His Thr Thr Ser Tyr
        195                 200                 205

Leu Leu Ile His Asp Gly Asp Arg Ala Val Val Thr Tyr Glu Gly Asp
    210                 215                 220

Thr Ser Ala Gly Tyr Asn His Asp Val Ser Ile Leu Arg Ala Trp Ile
225                 230                 235                 240

Arg Thr Thr Lys Ile Val Gly Asp His Pro Leu Val Ile Glu Arg Val
                245                 250                 255

Arg Ala Ile Gly Cys His Phe Val Leu Leu Leu Thr Ala Ala Pro Glu
            260                 265                 270

Pro Ser Pro Met Pro Tyr Val Pro Tyr Pro Arg Ser Thr Glu Val Tyr
        275                 280                 285

Val Arg Ser Ile Phe Gly Pro Gly Gly Ser Pro Ser Leu Phe Pro Ser

    290                 295                 300

Ala Cys Ser Thr Lys Ser Thr Phe His Ala Val Pro Val His Ile Trp
305                 310                 315                 320

Asp Arg Leu Met Leu Phe Gly Ala Thr Leu Asp Asp Gln Ala Phe Cys
                325                 330                 335

Cys Ser Arg Leu Met Thr Tyr Leu Arg Gly Ile Ser Tyr Lys Val Thr
            340                 345                 350

Val Gly Ala Leu Val Ala Asn Glu Gly Trp Asn Ala Ser Glu Asp Ala
        355                 360                 365

Leu Thr Ala Val Ile Thr Ala Ala Tyr Leu Thr Ile Cys His Gln Arg
    370                 375                 380

Tyr Leu Arg Thr Gln Ala Ile Ser Lys Gly Met Arg Arg Leu Glu Val
385                 390                 395                 400

Glu His Ala Gln Lys Phe Ile Thr Arg Leu Tyr Ser Trp Leu Phe Glu
                405                 410                 415

Lys Ser Gly Arg Asp Tyr Ile Pro Gly Arg Gln Leu Gln Phe Tyr Ala
            420                 425                 430

Gln Cys Arg Arg Trp Leu Ser Ala Gly Phe His Leu Asp Pro Arg Val
        435                 440                 445

Leu Val Phe Asp Glu Ala Val Pro Cys Arg Cys Arg Thr Phe Leu Lys
    450                 455                 460

Lys Val Ala Gly Lys Phe Cys Cys Phe Met Arg Trp Leu Gly Gln Glu
465                 470                 475                 480

Cys Thr Cys Phe Leu Glu Pro Ala Glu Gly Leu Ile Gly Asp Gln Gly
                485                 490                 495

His Asp Asn Glu Ala Tyr Glu Gly Ser Glu Val Asp Pro Ala Glu Pro
            500                 505                 510

Ala His Leu Asp Val Ser Gly Thr Tyr Ala Val His Gly His Gln Leu
        515                 520                 525

Glu Ala Leu Tyr Arg Ala Leu Asn Val Pro His Asp Ile Ala Ala Arg
    530                 535                 540

Ala Ser Arg Leu Thr Ala Thr Val Glu Leu Val Ala Ser Pro Asp Arg
545                 550                 555                 560

Leu Glu Cys Arg Thr Val Leu Gly Asn Lys Thr Phe Arg Thr Thr Val
                565                 570                 575

Val Asp Gly Ala His Leu Glu Ala Asn Gly Pro Glu Glu Tyr Val Leu
            580                 585                 590

Ser Phe Asp Ala Ser Arg Gln Ser Met Gly Ala Gly Ser His Ser Leu
        595                 600                 605

Thr Tyr Glu Leu Thr Pro Ala Gly Leu Gln Val Arg Ile Ser Ser Asn
    610                 615                 620

Gly Leu Asp Cys Thr Ala Val Phe Pro Pro Gly Gly Ala Pro Ser Ala
625                 630                 635                 640

Ala Pro Gly Glu Val Ala Ala Phe Cys Ser Ala Leu Tyr Arg Tyr Asn
                645                 650                 655

Arg Phe Thr Gln Arg His Ser Leu Thr Gly Gly Leu Trp Leu His Pro
            660                 665                 670

Glu Gly Leu Leu Gly Ile Phe Pro Pro Phe Ser Pro Gly His Ile Trp
        675                 680                 685

Glu Ser Ala Asn Pro Phe Cys Gly Glu Gly Thr Leu Tyr Thr Arg Thr
    690                 695                 700

Trp Ser Thr Ser Gly Phe Ser Ser Asp Phe Ser Pro Pro Glu Ala Ala
705                 710                 715                 720

Ala Pro Val Pro Ala Ala Ala Pro Gly Leu Pro His Pro Thr Pro Pro
                725                 730                 735

Val Ser Asp Ile Trp Val Leu Pro Pro Pro Ser Glu Glu Ser Gln Ile
            740                 745                 750

Asp Ala Ala Pro Val Pro Pro Val Pro Lys Thr Val Gly Leu Pro Ser

        755                 760                 765

Pro Ile Val Leu Ala Pro Pro Ser Pro Leu Pro Ser Pro Val Arg Lys
    770                 775                 780

Pro Pro Ser Pro Pro Pro Ser Arg Thr Arg Arg Leu Leu Tyr Thr Tyr
785                 790                 795                 800

Pro Asp Gly Ala Arg Val Tyr Ala Gly Ser Leu Phe Glu Ser Asp Cys
                805                 810                 815

Asp Trp Leu Val Asn Ala Ser Asn Pro Gly His Arg Pro Gly Gly Gly
            820                 825                 830

Leu Cys His Ala Phe Tyr Gln Arg Phe Pro Glu Ala Phe Tyr Pro Thr
        835                 840                 845

Glu Phe Ile Met Arg Glu Gly Leu Ala Ala Tyr Thr Leu Thr Pro Arg
    850                 855                 860

Pro Ile Ile His Ala Val Ala Pro Asp Tyr Arg Val Glu Gln Asn Pro
865                 870                 875                 880

Lys Arg Leu Glu Ala Ala Tyr Arg Glu Thr Cys Ser Arg Arg Gly Thr
                885                 890                 895

Ala Ala Tyr Pro Leu Leu Gly Ser Gly Ile Tyr Gln Val Pro Val Ser
            900                 905                 910

Leu Ser Phe Asp Ala Trp Glu Arg Asn His Arg Pro Gly Asp Glu Leu
        915                 920                 925

Tyr Leu Thr Glu Pro Ala Ala Ala Trp Phe Glu Ala Asn Lys Pro Ser
    930                 935                 940

Gln Pro Ala Leu Thr Ile Thr Glu Asp Thr Ala Arg Thr Ala Asn Leu
945                 950                 955                 960

Ala Leu Glu Ile Asp Ala Ala Thr Glu Val Gly Arg Ala Cys Ala Gly
                965                 970                 975

Cys Thr Ile Ser Pro Gly Val Val His Tyr Gln Phe Thr Ala Gly Val
            980                 985                 990

Pro Gly Ser Gly Lys Ser Arg Ser Ile Gln Gln Gly Asp Val Asp Val
        995                 1000                1005

Val Val Val Pro Thr Arg Glu Leu Arg Asn Ser Trp Arg Arg Arg Gly
    1010                1015                1020

Phe Ala Ala Phe Thr Pro His Thr Ala Ala Arg Val Thr Ile Gly Arg
1025                1030                1035                1040

Arg Val Val Ile Asp Glu Ala Pro Ser Leu Pro Pro His Leu Leu Leu
                1045                1050                1055

Leu His Met Gln Arg Ala Ser Ser Val His Leu Leu Gly Asp Pro Asn
            1060                1065                1070

Gln Ile Pro Ala Ile Asp Phe Glu His Ala Gly Leu Val Pro Ala Ile
        1075                1080                1085

Arg Pro Glu Leu Ala Pro Thr Ser Trp Trp His Val Thr His Arg Cys
    1090                1095                1100

Pro Ala Asp Val Cys Glu Leu Ile Arg Gly Ala Tyr Pro Lys Ile Gln
1105                1110                1115                1120

Thr Thr Ser Arg Val Leu Arg Ser Leu Phe Trp Asn Glu Pro Ala Ile
                1125                1130                1135

Gly Gln Lys Leu Val Phe Thr Gln Ala Ala Lys Ala Ala Asn Pro Gly
            1140                1145                1150

Ala Ile Thr Val His Glu Ala Gln Gly Ala Thr Phe Thr Glu Thr Thr
        1155                1160                1165

Ile Ile Ala Thr Ala Asp Ala Arg Gly Leu Ile Gln Ser Ser Arg Ala
    1170                1175                1180

His Ala Ile Val Ala Leu Thr Arg His Thr Glu Lys Cys Val Ile Leu
1185                1190                1195                1200

Asp Ala Pro Gly Leu Leu Arg Glu Val Gly Ile Ser Asp Val Ile Val
                1205                1210                1215

Asn Asn Phe Phe Leu Ala Gly Gly Glu Val Gly His His Arg Pro Ser

1220                1225                1230

Val Ile Pro Arg Gly Asn Pro Asp Gln Asn Leu Gly Thr Leu Gln Ala
        1235                1240                1245

Phe Pro Pro Ser Cys Gln Ile Ser Ala Tyr His Gln Leu Ala Glu Glu
    1250                1255                1260

Leu Gly His Arg Pro Ala Pro Val Ala Ala Val Leu Pro Pro Cys Pro
1265                1270                1275                1280

Glu Leu Glu Gln Gly Leu Leu Tyr Met Pro Gln Glu Leu Thr Val Ser
                1285                1290                1295

Asp Ser Val Leu Val Phe Glu Leu Thr Asp Ile Val His Cys Arg Met
            1300                1305                1310

Ala Ala Pro Ser Gln Arg Lys Ala Val Leu Ser Thr Leu Val Gly Arg
        1315                1320                1325

Tyr Gly Arg Arg Thr Lys Leu Tyr Glu Ala Ala His Ser Asp Val Arg
    1330                1335                1340

Glu Ser Leu Ala Arg Phe Ile Pro Thr Ile Gly Pro Val Gln Ala Thr
1345                1350                1355                1360

Thr Cys Glu Leu Tyr Glu Leu Val Glu Ala Met Val Glu Lys Gly Gln
                1365                1370                1375

Asp Gly Ser Ala Val Leu Glu Leu Asp Leu Cys Asn Arg Asp Val Ser
            1380                1385                1390

Arg Ile Thr Phe Phe Gln Lys Asp Cys Asn Lys Phe Thr Thr Gly Glu
        1395                1400                1405

Thr Ile Ala His Gly Lys Val Gly Gln Gly Ile Ser Ala Trp Ser Lys
    1410                1415                1420

Thr Phe Cys Ala Leu Phe Gly Pro Trp Phe Arg Ala Ile Glu Lys Glu
1425                1430                1435                1440

Ile Leu Ala Leu Leu Pro Pro Asn Ile Phe Tyr Gly Asp Ala Tyr Glu
                1445                1450                1455

Glu Ser Val Phe Ala Ala Ala Val Ser Gly Ala Gly Ser Cys Met Val
            1460                1465                1470

Phe Glu Asn Asp Phe Ser Glu Phe Asp Ser Thr Gln Asn Asn Phe Ser
        1475                1480                1485

Leu Gly Leu Glu Cys Val Val Met Glu Glu Cys Gly Met Pro Gln Trp
    1490                1495                1500

Leu Ile Arg Leu Tyr His Leu Val Arg Ser Ala Trp Ile Leu Gln Ala
1505                1510                1515                1520

Pro Lys Glu Ser Leu Lys Gly Phe Trp Lys Lys His Ser Gly Glu Pro
                1525                1530                1535

Gly Thr Leu Leu Trp Asn Thr Val Trp Asn Met Ala Ile Ile Ala His
            1540                1545                1550

Cys Tyr Glu Phe Arg Asp Phe Arg Val Ala Ala Phe Lys Gly Asp Asp
        1555                1560                1565

Ser Val Val Leu Cys Ser Asp Tyr Arg Gln Ser Arg Asn Ala Ala Ala
    1570                1575                1580

Leu Ile Ala Gly Cys Gly Leu Lys Leu Lys Val Asp Tyr Arg Pro Ile
1585                1590                1595                1600

Gly Leu Tyr Ala Gly Val Val Val Ala Pro Gly Leu Gly Thr Leu Pro
                1605                1610                1615

Asp Val Val Arg Phe Ala Gly Arg Leu Ser Glu Lys Asn Trp Gly Pro
            1620                1625                1630

Gly Pro Glu Arg Ala Glu Gln Leu Arg Leu Ala Val Cys Asp Phe Leu
        1635                1640                1645

Arg Gly Leu Thr Asn Val Ala Gln Val Cys Val Asp Val Val Ser Arg
    1650                1655                1660

Val Tyr Gly Val Ser Pro Gly Leu Val His Asn Leu Ile Gly Met Leu
1665                1670                1675                1680

Gln Thr Ile Ala Asp Gly Lys Ala His Phe Thr Glu Thr Ile Lys Pro

1685                1690                1695

Val Leu Asp Leu Thr Asn Ser Ile Ile Gln Arg Glu Glu
            1700                1705

<210> 51
<211> 660
<212> PRT
<213> Hepatitis E Virus JKN-Sap (ORF2)

<400> 51
Met Arg Pro Arg Ala Val Leu Leu Leu Phe Leu Val Leu Leu Pro Met
1               5                   10                  15

Leu Pro Ala Pro Pro Ala Gly Gln Pro Ser Gly Arg Arg Arg Gly Arg
            20                  25                  30

Arg Ser Gly Gly Ala Gly Gly Gly Phe Trp Gly Asp Arg Val Asp Ser
        35                  40                  45

Gln Pro Phe Ala Leu Pro Tyr Ile His Pro Thr Asn Pro Phe Ala Ala
    50                  55                  60

Asp Val Val Ser Gln Pro Gly Ala Gly Thr Arg Pro Arg Gln Pro Pro
65                  70                  75                  80

Arg Pro Leu Gly Ser Ala Trp Arg Asp Gln Ser Gln Arg Pro Ser Ala
                85                  90                  95

Ala Pro Arg Arg Arg Ser Ala Pro Ala Gly Ala Ala Pro Leu Thr Ala
            100                 105                 110

Val Ser Pro Ala Pro Asp Thr Ala Pro Val Pro Asp Val Asp Ser Arg
        115                 120                 125

Gly Ala Ile Leu Arg Arg Gln Tyr Asn Leu Ser Thr Ser Pro Leu Thr
    130                 135                 140

Ser Ser Val Ala Ser Gly Thr Asn Leu Val Leu Tyr Ala Ala Pro Leu
145                 150                 155                 160

Asn Pro Leu Leu Pro Leu Gln Asp Gly Thr Asn Thr His Ile Met Ala
                165                 170                 175

Thr Glu Ala Ser Asn Tyr Ala Gln Tyr Arg Val Val Arg Ala Thr Ile
            180                 185                 190

Arg Tyr Arg Pro Leu Val Pro Asn Ala Val Gly Gly Tyr Ala Ile Ser
        195                 200                 205

Ile Ser Phe Trp Pro Gln Thr Thr Thr Thr Pro Thr Ser Val Asp Met
    210                 215                 220

Asn Ser Ile Thr Ser Thr Asp Val Arg Ile Leu Val Gln Pro Gly Ile
225                 230                 235                 240

Ala Ser Glu Leu Val Ile Pro Ser Glu Arg Leu His Tyr Arg Asn Gln

                245                 250                 255

Gly Trp Arg Ser Val Glu Thr Thr Gly Val Ala Glu Glu Glu Ala Thr
            260                 265                 270

Ser Gly Leu Val Met Leu Cys Ile His Gly Ser Pro Val Asn Ser Tyr
        275                 280                 285

Thr Asn Thr Pro Tyr Thr Gly Ala Leu Gly Leu Leu Asp Phe Ala Leu
    290                 295                 300

Glu Leu Glu Phe Arg Asn Leu Thr Pro Gly Asn Thr Asn Thr Arg Val
305                 310                 315                 320

Ser Arg Tyr Thr Ser Thr Ala Arg His Arg Leu Arg Arg Gly Ala Asp
                325                 330                 335

Gly Thr Ala Glu Leu Thr Thr Thr Ala Ala Thr Arg Phe Met Lys Asp
            340                 345                 350

Leu His Phe Thr Gly Thr Asn Gly Val Gly Glu Val Gly Arg Gly Ile
        355                 360                 365

Ala Leu Thr Leu Phe Asn Leu Ala Asp Thr Leu Leu Gly Gly Leu Pro
    370                 375                 380

Thr Glu Leu Ile Ser Ser Ala Gly Gly Gln Leu Phe Tyr Ser Arg Pro
385                 390                 395                 400

Val Val Ser Ala Asn Gly Glu Pro Thr Val Lys Leu Tyr Thr Ser Val
                405                 410                 415
Glu Asn Ala Gln Gln Asp Lys Gly Ile Thr Ile Pro His Asp Ile Asp
            420                 425                 430
Leu Gly Asp Ser Arg Val Val Ile Gln Asp Tyr Asp Asn Gln His Glu
        435                 440                 445
Gln Asp Arg Pro Thr Pro Ser Pro Ala Pro Ser Arg Pro Phe Ser Val
    450                 455                 460
Leu Arg Ala Asn Asp Val Leu Trp Leu Ser Leu Thr Ala Ala Glu Tyr
465                 470                 475                 480
Asp Gln Thr Thr Tyr Gly Ser Ser Thr Asn Pro Met Tyr Val Ser Asp
                485                 490                 495
Thr Val Thr Leu Val Asn Val Ala Thr Gly Ala Gln Ala Val Ala Arg
            500                 505                 510
Ser Leu Asp Trp Ser Lys Val Thr Leu Asp Gly Arg Pro Leu Thr Thr
        515                 520                 525
Ile Gln Gln Tyr Ser Lys Thr Phe Tyr Val Leu Pro Leu Arg Gly Lys
    530                 535                 540
Leu Ser Phe Trp Glu Ala Gly Thr Thr Lys Ala Gly Tyr Pro Tyr Asn
545                 550                 555                 560

Tyr Asn Thr Thr Ala Ser Asp Gln Ile Leu Ile Glu Asn Ala Ala Gly
                565                 570                 575

His Arg Val Ala Ile Ser Thr Tyr Thr Thr Ser Leu Gly Ala Gly Pro
            580                 585                 590

Thr Ser Ile Ser Ala Val Gly Val Leu Ala Pro His Ser Ala Leu Ala
        595                 600                 605

Val Leu Glu Asp Thr Val Asp Tyr Pro Ala Arg Ala His Thr Phe Asp
    610                 615                 620

Asp Phe Cys Pro Glu Cys Arg Thr Leu Gly Leu Gln Gly Cys Ala Phe
625                 630                 635                 640

Gln Ser Thr Ile Ala Glu Leu Gln Arg Leu Lys Met Lys Val Gly Lys
                645                 650                 655

Thr Arg Glu Ser
            660

<210> 52
<211> 1709
<212> PRT
<213> Hepatitis E Virus JMY-Haw (ORF1)

<400> 52

Met Glu Ala His Gln Phe Ile Lys Ala Pro Gly Ile Thr Thr Ala Ile
1               5                   10                  15
Glu Gln Ala Ala Leu Ala Ala Ala Asn Ser Ala Leu Ala Asn Ala Val
            20                  25                  30
Val Val Arg Pro Phe Leu Ser Arg Val Gln Thr Glu Ile Leu Ile Asn
        35                  40                  45
Leu Met Gln Pro Arg Gln Leu Val Phe Arg Pro Glu Val Leu Trp Asn
    50                  55                  60
His Pro Ile Gln Arg Val Ile His Asn Glu Leu Glu Gln Tyr Cys Arg
65                  70                  75                  80
Ala Arg Ala Gly Arg Cys Leu Glu Val Gly Ala His Pro Arg Ser Ile
                85                  90                  95
Asn Asp Asn Pro Asn Val Leu His Arg Cys Phe Leu Arg Pro Val Gly
            100                 105                 110
Arg Asp Val Gln Arg Trp Tyr Ser Ala Pro Thr Arg Gly Pro Ala Ala
        115                 120                 125
Asn Cys Arg Arg Ser Ala Leu Arg Gly Leu Pro Pro Ala Asp Arg Thr
    130                 135                 140
Tyr Cys Phe Asp Gly Phe Ser Arg Cys Ala Phe Ala Ala Glu Thr Gly
145                 150                 155                 160

Val Ala Leu Tyr Ser Leu His Asp Leu Trp Pro Ala Asp Val Ala Glu
                165                 170                 175
Ala Met Ala Arg His Gly Met Thr Arg Leu Tyr Ala Val Leu His Leu
            180                 185                 190
Pro Pro Glu Val Leu Leu Pro Pro Gly Thr Tyr His Thr Thr Ser Tyr
        195                 200                 205
Leu Leu Ile His Asp Gly Asp Arg Ala Val Val Thr Tyr Glu Gly Asp
    210                 215                 220
Thr Ser Ala Gly Tyr Asn His Asp Val Ser Ile Leu Arg Ala Trp Ile
225                 230                 235                 240
Arg Thr Thr Lys Ile Val Gly Asp His Pro Leu Val Ile Glu Arg Val
                245                 250                 255
Arg Ala Ile Gly Cys His Phe Val Leu Leu Leu Thr Ala Ala Pro Glu
            260                 265                 270
Pro Ser Pro Met Pro Tyr Val Pro Tyr Pro Arg Ser Thr Glu Val Tyr
        275                 280                 285
Val Arg Ser Ile Phe Gly Pro Gly Gly Ser Pro Ser Leu Phe Pro Ser
    290                 295                 300
Ala Cys Ser Thr Lys Ser Thr Phe His Ala Val Pro Val His Ile Trp

305                 310                 315                 320

Asp Arg Leu Met Leu Phe Gly Ala Thr Leu Asp Asp Gln Ala Phe Cys

                325                 330                 335

Cys Ser Arg Leu Met Thr Tyr Leu Arg Gly Ile Ser Tyr Lys Val Thr

            340                 345                 350

Val Gly Ala Leu Val Ala Asn Glu Gly Trp Asn Ala Ser Glu Asp Ala

        355                 360                 365

Leu Thr Ala Val Ile Thr Ala Ala Tyr Leu Thr Ile Cys His Gln Arg

    370                 375                 380

Tyr Leu Arg Thr Gln Ala Ile Ser Lys Gly Met Arg Arg Leu Glu Val

385                 390                 395                 400

Glu His Ala Gln Lys Phe Ile Thr Arg Leu Tyr Ser Trp Leu Phe Glu

                405                 410                 415

Lys Ser Gly Arg Asp Tyr Ile Pro Gly Arg Gln Leu Gln Phe Tyr Ala

            420                 425                 430

Gln Cys Arg Arg Trp Leu Ser Ala Gly Phe His Leu Asp Pro Arg Val

        435                 440                 445

Leu Val Phe Asp Glu Ser Val Pro Cys Arg Cys Arg Thr Phe Leu Lys

    450                 455                 460

Lys Val Ala Gly Lys Phe Cys Cys Phe Met Arg Trp Leu Gly Gln Glu
465                 470                 475                 480

Cys Thr Cys Phe Leu Glu Pro Ala Glu Gly Leu Val Gly Asp Gln Gly
                485                 490                 495

His Asp Asn Glu Ala Tyr Glu Gly Ser Glu Val Asp Pro Ala Glu Pro
            500                 505                 510

Ala His Leu Asp Val Ser Gly Thr Tyr Ala Val His Gly His Gln Leu
        515                 520                 525

Glu Ala Leu Tyr Arg Ala Leu Asn Val Pro His Asp Ile Ala Ala Arg
    530                 535                 540

Ala Ser Arg Leu Thr Ala Thr Val Glu Leu Val Ala Ser Pro Asp Arg
545                 550                 555                 560

Leu Glu Cys Arg Thr Val Leu Gly Asn Lys Thr Phe Arg Thr Thr Val
                565                 570                 575

Val Asp Gly Ala His Leu Glu Ala Asn Gly Pro Glu Gln Tyr Val Leu
            580                 585                 590

Ser Phe Asp Ala Ser Arg Gln Ser Met Gly Ala Gly Ser His Ser Leu
        595                 600                 605

Thr Tyr Glu Leu Thr Pro Ala Gly Leu Gln Val Arg Ile Ser Ser Asn
    610                 615                 620

Gly Leu Asp Cys Thr Ala Val Phe Pro Pro Gly Gly Ala Pro Ser Ala
625                 630                 635                 640

Ala Pro Gly Glu Val Ala Ala Phe Cys Ser Ala Leu Tyr Arg Tyr Asn
                645                 650                 655

Arg Phe Thr Gln Arg His Ser Leu Thr Gly Gly Leu Trp Leu His Pro
            660                 665                 670

Glu Gly Leu Leu Gly Ile Phe Pro Pro Phe Ser Pro Gly His Ile Trp
        675                 680                 685

Glu Ser Ala Asn Pro Phe Cys Gly Glu Gly Thr Leu Tyr Thr Arg Thr
    690                 695                 700

Trp Ser Thr Ser Gly Phe Ser Ser Asp Phe Ser Pro Pro Glu Ala Ala
705                 710                 715                 720

Ala Pro Val Pro Ala Ala Ala Pro Gly Leu Pro His Pro Thr Pro Pro
                725                 730                 735

Val Ser Asp Ile Trp Val Leu Pro Pro Pro Ser Glu Gly Ser Gln Ile
            740                 745                 750

Asp Ala Ala Pro Val Pro Pro Val Pro Lys Thr Val Gly Leu Pro Ser
        755                 760                 765

Pro Ile Val Leu Ala Pro Pro Pro Pro Phe Pro Ser Pro Val Arg Lys

    770                 775                 780

Pro Ser Ser Pro Pro Pro Ser Arg Thr Arg Arg Leu Leu Tyr Thr Tyr
785                 790                 795                 800

Pro Asp Gly Ala Arg Val Tyr Ala Gly Ser Leu Phe Glu Ser Asp Cys
                805                 810                 815

Asp Trp Leu Val Asn Ala Ser Asn Pro Gly His Arg Pro Gly Gly Gly
            820                 825                 830

Leu Cys His Ala Phe Tyr Gln Arg Phe Pro Glu Ala Phe Tyr Pro Thr
        835                 840                 845

Glu Phe Ile Met Arg Glu Gly Leu Ala Ala Tyr Thr Leu Thr Pro Arg
    850                 855                 860

Pro Ile Ile His Ala Val Ala Pro Asp Tyr Arg Val Glu Gln Asn Pro
865                 870                 875                 880

Lys Arg Leu Glu Ala Ala Tyr Arg Glu Thr Cys Ser Arg Arg Gly Thr
                885                 890                 895

Ala Ala Tyr Pro Leu Leu Gly Ser Gly Ile Tyr Gln Val Pro Val Ser
            900                 905                 910

Leu Ser Phe Asp Ala Trp Glu Arg Asn His Arg Pro Gly Asp Glu Leu
        915                 920                 925

Tyr Leu Thr Glu Pro Ala Ala Ala Trp Phe Glu Ala Asn Lys Pro Ser
930                 935                 940

Gln Pro Ala Leu Thr Ile Thr Glu Asp Thr Ala Arg Thr Ala Asn Leu
945                 950                 955                 960

Ala Leu Glu Ile Asp Ala Ala Thr Glu Val Gly Arg Ala Cys Ala Gly
                965                 970                 975

Cys Thr Ile Ser Pro Gly Ile Val His Tyr Gln Phe Thr Ala Gly Val
            980                 985                 990

Pro Gly Ser Gly Lys Ser Arg Ser Ile Gln Gln Gly Asp Val Asp Val
        995                 1000                1005

Val Val Val Pro Thr Arg Glu Leu Arg Asn Ser Trp Arg Arg Arg Gly
    1010                1015                1020

Phe Ala Ala Phe Thr Pro His Thr Ala Ala Arg Val Thr Ile Gly Arg
1025                1030                1035                1040

Arg Val Val Ile Asp Glu Ala Pro Ser Leu Pro Pro His Leu Leu Leu
                1045                1050                1055

Leu His Met Gln Arg Ala Ser Ser Val His Leu Leu Gly Asp Pro Asn
            1060                1065                1070

Gln Ile Pro Ala Ile Asp Phe Glu His Ala Gly Leu Val Pro Ala Ile
        1075                1080                1085

Arg Pro Glu Leu Ala Pro Thr Ser Trp Trp His Val Thr His Arg Cys
    1090                1095                1100

Pro Ala Asp Val Cys Glu Leu Ile Arg Gly Ala Tyr Pro Lys Ile Gln
1105                1110                1115                1120

Thr Thr Ser Arg Val Leu Arg Ser Leu Phe Trp Asn Glu Pro Ala Ile
                1125                1130                1135

Gly Gln Lys Leu Val Phe Thr Gln Ala Ala Lys Ala Ala Asn Pro Gly
            1140                1145                1150

Ala Ile Thr Val His Glu Ala Gln Gly Ala Thr Phe Thr Glu Thr Thr
        1155                1160                1165

Ile Ile Ala Thr Ala Asp Ala Arg Gly Leu Ile Gln Ser Ser Arg Ala
    1170                1175                1180

His Ala Ile Val Ala Leu Thr Arg His Thr Glu Lys Cys Val Ile Leu
1185                1190                1195                1200

Asp Ala Pro Gly Leu Leu Arg Glu Val Gly Ile Ser Asp Val Ile Val
                1205                1210                1215

Asn Asn Phe Phe Leu Ala Gly Gly Glu Val Gly His His Arg Pro Ser
            1220                1225                1230

Val Ile Pro Arg Gly Asn Pro Asp Gln Asn Leu Gly Thr Leu Gln Ala

        1235                1240                1245

Phe Pro Pro Ser Cys Gln Ile Ser Ala Tyr His Gln Leu Ala Glu Glu
    1250                1255                1260

Leu Gly His Arg Pro Ala Pro Val Ala Ala Val Leu Pro Pro Cys Pro
1265                1270                1275                1280

Glu Leu Glu Gln Gly Leu Leu Tyr Met Pro Gln Glu Leu Thr Val Ser
                1285                1290                1295

Asp Ser Val Leu Val Phe Glu Leu Thr Asp Ile Val His Cys Arg Met
            1300                1305                1310

Ala Ala Pro Ser Gln Arg Lys Ala Val Leu Ser Thr Leu Val Gly Arg
        1315                1320                1325

Tyr Gly Arg Arg Thr Lys Leu Tyr Glu Ala Ala His Ser Asp Val Arg
    1330                1335                1340

Glu Ser Leu Ala Arg Phe Ile Pro Thr Ile Gly Pro Val Gln Ala Thr
1345                1350                1355                1360

Thr Cys Glu Leu Tyr Glu Leu Val Glu Ala Met Val Glu Lys Gly Gln
                1365                1370                1375

Asp Gly Ser Ala Val Leu Glu Leu Asp Leu Cys Asn Arg Asp Val Ser
            1380                1385                1390

Arg Ile Thr Phe Phe Gln Lys Asp Cys Asn Lys Phe Thr Thr Gly Glu
        1395                1400                1405
Thr Ile Ala His Gly Lys Val Gly Gln Gly Ile Ser Ala Trp Ser Lys
    1410                1415                1420
Thr Phe Cys Ala Leu Phe Gly Pro Trp Phe Arg Ala Ile Glu Lys Glu
1425                1430                1435                1440
Ile Leu Ala Leu Leu Pro Pro Asn Ile Phe Tyr Gly Asp Ala Tyr Glu
                1445                1450                1455
Glu Ser Val Phe Ala Ala Ala Val Ser Gly Ala Gly Ser Cys Met Val
            1460                1465                1470
Phe Glu Asn Asp Phe Ser Glu Phe Asp Ser Thr Gln Asn Asn Phe Ser
        1475                1480                1485
Leu Gly Leu Glu Cys Val Val Met Glu Glu Cys Gly Met Pro Gln Trp
    1490                1495                1500
Leu Ile Arg Leu Tyr His Leu Val Arg Ser Ala Trp Ile Leu Gln Ala
1505                1510                1515                1520
Pro Lys Glu Ser Leu Lys Gly Phe Trp Lys Lys His Ser Gly Glu Pro
                1525                1530                1535
Gly Thr Leu Leu Trp Asn Thr Val Trp Asn Met Ala Ile Ile Ala His
            1540                1545                1550

Cys Tyr Glu Phe Arg Asp Phe Arg Val Ala Ala Phe Lys Gly Asp Asp
        1555                1560                1565

Ser Val Val Leu Cys Ser Asp Tyr Arg Gln Ser Arg Asn Ala Ala Ala
    1570                1575                1580

Leu Ile Ala Gly Cys Gly Leu Lys Leu Lys Val Asp Tyr Arg Pro Ile
1585                1590                1595                1600

Gly Leu Tyr Ala Gly Val Val Val Ala Pro Gly Leu Gly Thr Leu Pro
                1605                1610                1615

Asp Val Val Arg Phe Ala Gly Arg Leu Ser Glu Lys Asn Trp Gly Pro
            1620                1625                1630

Gly Pro Glu Arg Ala Glu Gln Leu Arg Leu Ala Val Cys Asp Phe Leu
        1635                1640                1645

Arg Gly Leu Thr Asn Val Ala Gln Val Cys Val Asp Val Val Ser Arg
    1650                1655                1660

Val Tyr Gly Val Ser Pro Gly Leu Val His Asn Leu Ile Gly Met Leu
1665                1670                1675                1680

Gln Thr Ile Ala Asp Gly Lys Ala His Phe Thr Glu Thr Ile Lys Pro
                1685                1690                1695

Val Leu Asp Leu Thr Asn Ser Ile Ile Gln Arg Glu Glu

1700                1705

<210> 53

<211> 660

<212> PRT

<213> Hepatitis E Virus JMY-Haw (ORF2)

<400> 53

Met Arg Pro Arg Ala Val Leu Leu Leu Phe Leu Val Leu Leu Pro Met
1               5                   10                  15

Leu Pro Ala Pro Pro Ala Gly Gln Pro Ser Gly Arg Arg Arg Gly Arg
            20                  25                  30

Arg Ser Gly Gly Thr Gly Gly Gly Phe Trp Gly Asp Arg Val Asp Ser
        35                  40                  45

Gln Pro Phe Ala Leu Pro Tyr Ile His Pro Thr Asn Pro Phe Ala Ala
    50                  55                  60

Asp Val Val Ser Gln Pro Gly Ala Gly Ala Arg Pro Arg Gln Pro Pro
65                  70                  75                  80

Arg Pro Leu Gly Ser Ala Trp Arg Asp Gln Ser Gln Arg Pro Ser Ala
                85                  90                  95

Ala Pro Arg Arg Arg Ser Ala Pro Ala Gly Ala Ala Pro Leu Thr Ala
            100                 105                 110

Val Ser Pro Ala Pro Asp Thr Ala Pro Val Pro Asp Val Asp Ser Arg
        115                 120                 125
Gly Ala Ile Leu Arg Arg Gln Tyr Asn Leu Ser Thr Ser Pro Leu Thr
    130                 135                 140
Ser Ser Val Ala Ser Gly Thr Asn Leu Val Leu Tyr Ala Ala Pro Leu
145                 150                 155                 160
Asn Pro Leu Leu Pro Leu Gln Asp Gly Thr Asn Thr His Ile Met Ala
                165                 170                 175
Thr Glu Ala Ser Asn Tyr Ala Gln Tyr Arg Val Val Arg Ala Thr Ile
            180                 185                 190
Arg Tyr Arg Pro Leu Val Pro Asn Ala Val Gly Gly Tyr Ala Ile Ser
        195                 200                 205
Ile Ser Phe Trp Pro Gln Thr Thr Thr Thr Pro Thr Ser Val Asp Met
    210                 215                 220
Asn Ser Ile Thr Ser Thr Asp Val Arg Ile Leu Val Gln Pro Gly Ile
225                 230                 235                 240
Ala Ser Glu Leu Val Ile Pro Ser Glu Arg Leu His Tyr Arg Asn Gln
                245                 250                 255
Gly Trp Arg Ser Val Glu Thr Thr Gly Val Ala Glu Glu Glu Ala Thr

            260                 265                 270

Ser Gly Leu Val Met Leu Cys Ile His Gly Ser Pro Val Asn Ser Tyr
        275                 280                 285

Thr Asn Thr Pro Tyr Thr Gly Ala Leu Gly Leu Leu Asp Phe Ala Leu
    290                 295                 300

Glu Leu Glu Phe Arg Asn Leu Thr. Pro Gly Asn Thr Asn Thr Arg Val
305 310 315 320

Ser Arg Tyr Thr Ser Thr Ala Arg His Arg Leu Arg Arg Gly Ala Asp
325 330 335

Gly Thr Ala Glu Leu Thr Thr Thr Ala Ala Thr Arg Phe Met Lys Asp
340 345 350

Leu His Phe Thr Gly Thr Asn Gly Val Gly Glu Val Gly Arg Gly Ile
355 360 365

Ala Leu Thr Leu Phe Asn Leu Ala Asp Thr Leu Leu Gly Gly Leu Pro
370 375 380

Thr Glu Leu Ile Ser Ser Ala Gly Gly Gln Leu Phe Tyr Ser Arg Pro
385 390 395 400

Val Val Ser Ala Asn Gly Glu Pro Thr Val Lys Leu Tyr Thr Ser Val
405 410 415

Glu Asn Ala Gln Gln Asp Lys Gly Ile Thr Ile Pro His Asp Ile Asp
420 425 430

Leu Gly Asp Ser Arg Val Val Ile Gln Asp Tyr Asp Asn Gln His Glu
435 440 445

Gln Asp Arg Pro Thr Pro Ser Pro Ala Pro Ser Arg Pro Phe Ser Val
450 455 460

Leu Arg Ala Asn Asp Val Leu Trp Leu Ser Leu Thr Ala Ala Glu Tyr
465 470 475 480

Asp Gln Thr Thr Tyr Gly Ser Ser Thr Asn Pro Met Tyr Val Ser Asp
485 490 495

Thr Val Thr Leu Val Asn Val Ala Thr Gly Ala Gln Ala Val Ala Arg
500 505 510

Ser Leu Asp Trp Ser Lys Val Thr Leu Asp Gly Arg Pro Leu Thr Thr
515 520 525

Ile Gln Gln Tyr Ser Lys Thr Phe Tyr Val Leu Pro Leu Arg Gly Lys
530 535 540

Leu Ser Phe Trp Glu Ala Gly Thr Thr Lys Ala Gly Tyr Pro Tyr Asn
545 550 555 560

Tyr Asn Thr Thr Ala Ser Asp Gln Ile Leu Ile Glu Asn Ala Ala Gly
565 570 575

His Arg Val Ala Ile Ser Thr Tyr Thr Thr Ser Leu Gly Ala Gly Pro
580 585 590

Thr Ser Ile Ser Ala Val Gly Val Leu Ala Pro His Ser Ala Leu Ala
595 600 605

Ala Leu Glu Asp Thr Val Asp Tyr Pro Ala Arg Ala His Thr Phe Asp
610 615 620

Asp Phe Cys Pro Glu Cys Arg Thr Leu Gly Leu Gln Gly Cys Ala Phe
625 630 635 640

Gln Ser Thr Ile Ala Glu Leu Gln Arg Leu Lys Met Lys Val Gly Lys
645 650 655

Thr Arg Glu Ser
660

<210> 54
<211> 1707
<212> PRT
<213> Hepatitis E Virus JKK-Sap (ORF1)

<400> 54
Met Glu Ala His Gln Phe Ile Lys Ala Pro Gly Val Thr Thr Ala Ile
1               5                   10                  15

Glu Gln Ala Ala Leu Ala Ala Ala Asn Ser Ala Leu Ala Asn Ala Val
            20                  25                  30
Val Val Arg Pro Phe Leu Ser Arg Leu Gln Thr Glu Ile Leu Ile Asn
        35                  40                  45
Leu Met Gln Pro Arg Gln Leu Val Phe Arg Pro Glu Val Leu Trp Asn
    50                  55                  60
His Pro Ile Gln Arg Val Ile His Asn Glu Leu Glu Gln Tyr Cys Arg
65                  70                  75                  80
Ala Arg Ala Gly Arg Cys Leu Glu Val Gly Ala His Pro Arg Ser Ile
                85                  90                  95
Asn Asp Asn Pro Asn Val Leu His Arg Cys Phe Leu Lys Pro Val Gly
            100                 105                 110
Arg Asp Val Gln Arg Trp Tyr Thr Ala Pro Thr Arg Gly Pro Ala Ala
        115                 120                 125
Asn Cys Arg Arg Ser Ala Leu Arg Gly Leu Pro Pro Ala Asp Arg Thr
    130                 135                 140
Tyr Cys Phe Asn Gly Phe Ser Gly Cys Thr Phe Ala Ala Glu Thr Gly
145                 150                 155                 160
Ile Ala Leu Tyr Ser Leu His Asp Leu Trp Pro Ala Asp Val Ala Glu
                165                 170                 175

Ala Met Ala Arg His Gly Met Thr Arg Leu Tyr Ala Ala Leu His Leu
            180                 185                 190
Pro Pro Glu Val Leu Leu Pro Pro Gly Thr Tyr His Thr Thr Ser Tyr
        195                 200                 205
Leu Leu Ile His Asp Gly Asp Arg Ala Val Ile Thr Tyr Glu Gly Asp
    210                 215                 220
Ser Ser Ala Gly Tyr Asn His Asp Val Ser Ile Leu Arg Ala Trp Ile
225                 230                 235                 240
Arg Thr Thr Lys Val Thr Gly Asp His Pro Leu Val Ile Glu Arg Val
                245                 250                 255
Arg Ala Val Gly Cys His Phe Val Leu Leu Leu Thr Ala Ala Pro Glu
            260                 265                 270
Pro Ser Pro Met Pro Tyr Val Pro Tyr Pro Arg Ser Thr Glu Val Tyr
        275                 280                 285
Val Arg Ser Ile Phe Gly Pro Gly Gly Ser Pro Ser Leu Phe Pro Thr
    290                 295                 300
Ala Cys Ser Thr Lys Ser Thr Phe His Ala Val Pro Val His Ile Trp
305                 310                 315                 320
Asp Arg Leu Met Leu Phe Gly Ala Thr Leu Asp Asp Gln Ala Phe Cys

                325                 330                 335

Cys Ser Arg Leu Met Thr Tyr Leu Arg Gly Ile Ser Tyr Lys Val Thr
            340                 345                 350

Val Gly Ala Leu Val Ala Asn Glu Gly Trp Asn Ala Ser Glu Asp Ala
        355                 360                 365

Leu Thr Ala Val Ile Thr Ala Ala Tyr Leu Thr Ile Cys His Gln Arg
    370                 375                 380

Tyr Leu Arg Thr Gln Ala Ile Ser Lys Gly Met Lys Arg Leu Glu Leu
385                 390                 395                 400

Glu His Ala Gln Lys Phe Ile Thr Arg Leu Tyr Ser Trp Leu Phe Glu
                405                 410                 415

Lys Ser Gly Arg Asp Tyr Ile Pro Gly Arg Gln Leu Gln Phe Tyr Ala
            420                 425                 430

Gln Cys Arg Arg Trp Leu Ser Ala Gly Phe His Leu Asp Pro Arg Val
        435                 440                 445

Leu Val Phe Asp Glu Ala Ala Pro Cys Arg Cys Arg Ser Leu Leu Arg
    450                 455                 460

Lys Ala Ala His Lys Phe Cys Cys Phe Met Arg Trp Leu Gly Gln Asp
465                 470                 475                 480

Cys Thr Cys Phe Leu Gln Pro Val Glu Gly Arg Val Gly Glu Gln Gly
                485                 490                 495

Tyr Asp Asn Glu Ala Phe Glu Gly Ser Asp Val Asp Pro Ala Glu Glu
            500                 505                 510

Ala Thr Val Ser Ile Ser Gly Ser Tyr Ile Val Thr Gly Ser Gln Leu
        515                 520                 525

Gln Pro Leu Tyr Gln Ala Leu Gly Ile Pro Ser Asp Leu Ala Ala Arg
    530                 535                 540

Ala Gly Arg Leu Thr Ala Thr Val Glu Val Ser Asp Thr Asp Gly Arg
545                 550                 555                 560

Leu Thr Cys Lys Thr Thr Met Gly Asn Lys Thr Phe Thr Thr Val Phe
                565                 570                 575

Thr Asp Gly Ala Gln Leu Glu Ala Asn Gly Pro Glu Gln Tyr Val Leu
            580                 585                 590

Ser Phe Asp Leu Ala Lys Gln Thr Met Ala Ala Gly Pro His Ser Leu
        595                 600                 605

Ser Tyr Ala Leu Thr Pro Ala Gly Leu Glu Val Arg Val Val Ser Ala
    610                 615                 620

Gly Leu Asp Cys Arg Ala Val Phe Pro Ser Gly Val Ala Thr Pro Ser
625                 630                 635                 640

Ala Ser Gly Glu Val Ser Ala Phe Cys Ser Ala Leu Tyr Arg Phe Asn
                645                 650                 655
Arg Cys Val Gln Arg His Ser Leu Ile Gly Gly Leu Trp Tyr Tyr Pro
            660                 665                 670
Glu Gly Leu Ile Gly Leu Phe Pro Pro Phe Ala Pro Gly His Thr Trp
        675                 680                 685
Glu Ser Ala Asn Pro Phe Cys Gly Glu Ser Thr Leu Tyr Thr Arg Thr
    690                 695                 700
Trp Ser Val Ser Gly Phe Ser Ser Cys Phe Ser Pro Phe Glu Pro Pro
705                 710                 715                 720
Ala Leu Asp Ser Pro Pro Pro Ala Glu Ala Asp Thr Pro Met Ala Val
                725                 730                 735
Asp Val Pro Pro Pro Ala Thr Leu Thr Leu Pro Gln Pro Pro Ala Pro
            740                 745                 750
Glu Arg Ala Val Pro Pro Gln Asp Leu Ala Asp Gly Asp Val Ala Arg
        755                 760                 765
Ala Ser Pro Gly Val Ser Ala Ala Pro Pro Val Pro Ala Gln Ser Val
    770                 775                 780
Thr Asp Pro Pro Val Ser Arg Arg Arg Leu Leu His Thr Tyr Pro Asp

785                 790                 795                 800

Gly Ser Lys Val Tyr Ala Gly Ser Leu Phe Glu Ser Glu Cys Thr Trp

                805                 810                 815

Leu Val Asn Ala Ser Asn Pro Gly His Arg Pro Gly Gly Gly Leu Cys

            820                 825                 830

His Ala Phe Tyr Gln Arg Phe Pro Glu Ser Phe Asp Pro Ala Glu Phe

        835                 840                 845

Val Met Ser Asp Gly Phe Ala Ala Tyr Thr Leu Thr Pro Arg Pro Ile

    850                 855                 860

Ile His Ala Val Ala Pro Asp Tyr Arg Val Glu His Asn Pro Lys Arg

865                 870                 875                 880

Leu Glu Ala Ala Tyr Arg Glu Thr Cys Ser Arg Arg Gly Thr Ala Ala

                885                 890                 895

Tyr Pro Leu Leu Gly Ala Gly Ile Tyr Lys Val Pro Val Gly Leu Ser

            900                 905                 910

Phe Asp Ala Trp Glu Arg Asn His Arg Pro Gly Asp Glu Leu Tyr Leu

        915                 920                 925

Thr Glu Pro Ala Ile Ala Trp Phe Glu Ala Asn Arg Pro Thr Leu Pro

    930                 935                 940

Ala Leu Thr Ile Thr Glu Asp Thr Ala Arg Thr Ala Asn Leu Ala Leu
945                 950                 955                 960
Glu Leu Asp Ser Ala Thr Glu Val Gly Arg Ala Cys Ala Gly Cys Arg
                965                 970                 975
Val Glu Pro Gly Val Val His Tyr Gln Phe Thr Ala Gly Val Pro Gly
            980                 985                 990
Ser Gly Lys Ser Arg Ser Ile Gln Gln Gly Glu Val Asp Val Val Val
        995                 1000                1005
Val Pro Thr Arg Glu Leu Arg Asn Ser Trp Arg Arg Arg Gly Phe Ala
    1010                1015                1020
Ala Tyr Thr Pro His Thr Ala Ala Arg Val Thr Arg Gly Arg Arg Val
1025                1030                1035                1040
Val Ile Asp Glu Ala Pro Ser Leu Pro Pro His Leu Leu Leu Leu His
                1045                1050                1055
Met Gln Arg Ala Ser Ser Val His Leu Leu Gly Asp Pro Asn Gln Ile
            1060                1065                1070
Pro Ala Ile Asp Phe Glu His Ala Gly Leu Val Pro Ala Ile Arg Pro
        1075                1080                1085
Glu Leu Val Pro Thr Lys Trp Trp His Leu Thr His Arg Cys Pro Ala
    1090                1095                1100

Asp Val Cys Glu Leu Ile Arg Gly Ala Tyr Pro Lys Ile Gln Thr Ala
1105                1110                1115                1120

Ser Arg Val Phe Arg Ser Leu Phe Trp Gly Glu Pro Pro Val Gly Gln
                1125                1130                1135

Lys Leu Val Phe Thr Gln Ala Ala Lys Ala Ala Asn Pro Gly Ala Ile
            1140                1145                1150

Thr Val His Glu Ala Gln Gly Ala Thr Phe Thr Glu Thr Thr Ile Ile
        1155                1160                1165

Ala Thr Ala Asp Ala Arg Gly Leu Ile Gln Ser Ser Arg Ala His Ala
    1170                1175                1180

Ile Val Ala Leu Thr Arg His Thr Glu Lys Cys Val Val Val Asp Ala
1185                1190                1195                1200

Pro Gly Leu Leu Arg Glu Val Gly Ile Ser Asp Ala Ile Val Asn Asn
                1205                1210                1215

Phe Phe Leu Ser Gly Gly Gln Ile Gly Gln His Arg Pro Ser Val Ile
            1220                1225                1230

Pro Arg Gly Thr Ile Asn Asn Asn Val Asp Thr Leu Asp Ala Phe Pro
        1235                1240                1245

Pro Ser Cys Gln Phe Ser Ala Tyr His Gln Leu Ala Glu Glu Leu Gly

1250                    1255                    1260

His Arg Pro Ala Pro Ile Ala Ala Val Leu Pro Pro Cys Pro Glu Leu
1265                    1270                    1275                    1280

Glu Gln Gly Leu Leu Tyr Met Pro Gln Glu Leu Thr Thr Ser Asp Ser
                1285                    1290                    1295

Val Leu Thr Phe Glu Leu Thr Asp Ile Val His Cys Arg Met Ala Ala
            1300                    1305                    1310

Pro Ser Gln Arg Lys Ala Val Leu Ser Thr Leu Val Gly Arg Tyr Gly
        1315                    1320                    1325

Arg Arg Thr Lys Leu Tyr Glu Ala Ala His Ala Asp Val Arg Gly Ser
    1330                    1335                    1340

Leu Asn His Phe Ile Pro Glu Leu Gly Pro Val Ser Val Thr Thr Cys
1345                    1350                    1355                    1360

Glu Leu Tyr Glu Leu Val Glu Ala Met Val Glu Lys Gly Gln Asp Gly
                1365                    1370                    1375

Ser Ala Val Leu Glu Leu Asp Leu Cys Ser Arg Asp Val Ser Arg Ile
            1380                    1385                    1390

Thr Phe Phe Gln Lys Asp Cys Asn Lys Phe Thr Thr Gly Glu Thr Ile
        1395                    1400                    1405

Ala His Gly Lys Val Gly Gln Gly Ile Ser Ala Trp Ser Lys Thr Phe
    1410                1415                1420
Cys Ala Leu Phe Gly Pro Trp Phe Arg Ala Ile Glu Lys Glu Ile Leu
1425                1430                1435                1440
Ala Val Leu Ala Pro Asn Val Phe Tyr Gly Asp Ala Tyr Glu Asp Thr
                1445                1450                1455
Val Leu Ala Ala Ala Val Ala Gly Ala Pro Gly Cys Lys Val Phe Glu
            1460                1465                1470
Asn Asp Phe Ser Glu Phe Asp Ser Thr Gln Asn Asn Phe Ser Leu Gly
        1475                1480                1485
Leu Glu Cys Ile Ile Met Glu Glu Cys Gly Met Pro Gln Trp Met Ile
    1490                1495                1500
Arg Leu Tyr His Leu Val Arg Ser Ala Trp Val Leu Gln Ala Pro Lys
1505                1510                1515                1520
Glu Ser Leu Arg Gly Phe Trp Lys Lys His Ser Gly Glu Pro Gly Thr
                1525                1530                1535
Leu Leu Trp Asn Thr Val Trp Asn Met Ala Val Ile Ala His Cys Tyr
            1540                1545                1550
Glu Phe Arg Asp Leu Lys Val Ala Ala Phe Lys Gly Asp Asp Ser Val
        1555                1560                1565

Val Leu Cys Ser Asp Tyr Arg Gln Ser Arg Asp Ala Ala Ala Leu Ile
    1570                1575                1580

Ala Gly Cys Gly Leu Lys Leu Lys Val Asp Phe Arg Pro Ile Gly Leu
1585                1590                1595                1600

Tyr Ala Gly Val Val Val Ala Pro Gly Leu Gly Thr Leu Pro Asp Val
                1605                1610                1615

Val Arg Phe Ala Gly Arg Leu Ser Glu Lys Asn Trp Gly Pro Gly Ser
            1620                1625                1630

Glu Arg Ala Glu Gln Leu Arg Leu Ala Val Cys Asp Phe Leu Arg Lys
        1635                1640                1645

Leu Thr Asn Val Ala Gln Val Cys Val Asp Val Val Ser Gln Val Tyr
    1650                1655                1660

Gly Val Ser Pro Gly Leu Val His Asn Leu Ile Gly Met Leu Gln Thr
1665                1670                1675                1680

Ile Ala Asp Gly Lys Ala His Phe Thr Glu Thr Val Lys Pro Val Leu
                1685                1690                1695

Asp Leu Thr Asn Ser Ile Ile His Arg Val Glu
            1700                1705

<210> 55

<211> 674

<212> PRT

<213> Hepatitis E Virus JKK-Sap (ORF2)

<400> 55

Met Asn Asn Met Phe Phe Cys Ser Val His Gly Asp Ala Thr Met Arg
1               5                   10                  15

Ser Arg Ala Phe Leu Phe Leu Phe Leu Val Leu Leu Pro Met Leu Pro
            20                  25                  30

Ala Pro Pro Ala Gly Gln Pro Ser Gly Arg Arg Arg Gly Arg Arg Ser
        35                  40                  45

Gly Gly Ala Gly Gly Gly Phe Trp Gly Asp Arg Val Asp Ser Gln Pro
    50                  55                  60

Phe Ala Leu Pro Tyr Ile His Pro Thr Asn Pro Phe Ala Ser Asp Ile
65                  70                  75                  80

Pro Ala Ala Ala Gly Ala Gly Ala Arg Pro Arg Gln Pro Ala Arg Pro
                85                  90                  95

Leu Gly Ser Ala Trp Arg Asp Gln Ser Gln Arg Pro Ala Thr Ser Ala
            100                 105                 110

Arg Arg Arg Ser Ala Pro Ala Gly Ala Ser Pro Leu Thr Ala Val Ala
        115                 120                 125

Pro Ala Pro Asp Thr Ala Pro Val Pro Asp Val Asp Ser Arg Gly Ala
    130                 135                 140

Ile Leu Arg Arg Gln Tyr Asn Leu Ser Thr Ser Pro Leu Thr Ser Thr
145                 150                 155                 160

Ile Ala Thr Gly Thr Asn Leu Val Leu Tyr Ala Ala Pro Leu Ser Pro
                165                 170                 175

Leu Leu Pro Leu Gln Asp Gly Thr Asn Thr His Ile Met Ala Thr Glu
            180                 185                 190

Ala Ser Asn Tyr Ala Gln Tyr Arg Val Val Arg Ala Thr Ile Arg Tyr
        195                 200                 205

Arg Pro Leu Val Pro Asn Ala Val Gly Gly Tyr Ala Ile Ser Ile Ser
    210                 215                 220

Phe Trp Pro Gln Thr Thr Thr Thr Pro Thr Ser Val Asp Met Asn Ser
225                 230                 235                 240

Ile Thr Ser Thr Asp Val Arg Ile Leu Val Gln Pro Gly Ile Ala Ser
                245                 250                 255

Glu Leu Val Ile Pro Ser Glu Arg Leu His Tyr Arg Asn Gln Gly Trp
            260                 265                 270

Arg Ser Val Glu Thr Ser Gly Val Ala Glu Glu Glu Ala Thr Ser Gly

        275                 280                 285

Leu Val Met Leu Cys Ile His Gly Ser Pro Val Asn Ser Tyr Thr Asn
    290                 295                 300

Thr Pro Tyr Thr Gly Ala Leu Gly Leu Leu Asp Phe Ala Leu Glu Leu
305                 310                 315                 320

Glu Phe Arg Asn Leu Thr Pro Gly Asn Thr Asn Thr Arg Val Ser Arg
                325                 330                 335

Tyr Ser Ser Ser Ala Arg His Lys Leu Arg Arg Gly Pro Asp Gly Thr
            340                 345                 350

Ala Glu Leu Thr Thr Thr Ala Ala Thr Arg Phe Met Lys Asp Leu His
        355                 360                 365

Phe Thr Gly Thr Asn Gly Val Gly Glu Val Gly Arg Gly Ile Ala Leu
    370                 375                 380

Thr Leu Phe Asn Leu Ala Asp Thr Leu Leu Gly Gly Leu Pro Thr Glu
385                 390                 395                 400

Leu Ile Ser Ser Ala Gly Gly Gln Leu Phe Tyr Ser Arg Pro Val Val
                405                 410                 415

Ser Ala Asn Gly Glu Pro Thr Val Lys Leu Tyr Thr Ser Val Glu Asn
            420                 425                 430

Ala Gln Gln Asp Lys Gly Ile Ala Ile Pro His Asp Ile Asp Leu Gly
        435                 440                 445
Glu Ser Arg Val Val Ile Gln Asp Tyr Asp Asn Gln His Glu Gln Asp
    450                 455                 460
Arg Pro Thr Pro Ser Pro Ala Pro Ser Arg Pro Phe Ser Val Leu Arg
465                 470                 475                 480
Ala Asn Asp Val Leu Trp Leu Ser Leu Thr Ala Ala Glu Tyr Asp Gln
                485                 490                 495
Thr Thr Tyr Gly Ser Ser Thr Asn Pro Met Tyr Val Ser Asp Thr Val
            500                 505                 510
Thr Phe Val Asn Val Ala Thr Gly Ala Gln Gly Val Ser Arg Ser Leu
        515                 520                 525
Asp Trp Ser Lys Val Thr Leu Asp Gly Arg Pro Leu Met Thr Ile Gln
    530                 535                 540
Gln Tyr Ser Lys Thr Phe Phe Val Leu Pro Leu Arg Gly Lys Leu Ser
545                 550                 555                 560
Phe Trp Glu Ala Gly Thr Thr Lys Ala Gly Tyr Pro Tyr Asn Tyr Asn
                565                 570                 575
Thr Thr Ala Ser Asp Gln Ile Leu Ile Glu Asn Ala Ala Gly His Arg
            580                 585                 590

Val Cys Ile Ser Thr Tyr Thr Thr Asn Leu Gly Ser Gly Pro Val Ser
        595                 600                 605

Ile Ser Ala Val Gly Val Leu Ala Pro His Ser Ala Leu Ala Ala Leu
    610                 615                 620

Glu Asp Thr Val Asp Tyr Pro Ala Arg Ala His Thr Phe Asp Asp Phe
625                 630                 635                 640

Cys Pro Glu Cys Arg Ala Leu Gly Leu Gln Gly Cys Ala Phe Gln Ser
                645                 650                 655

Thr Val Ala Glu Leu Gln Arg Leu Lys Met Lys Val Gly Lys Thr Arg
            660                 665                 670

Glu Tyr

<210> 56
<211> 1707
<212> PRT
<213> Hepatitis E Virus JAK-Sai (ORF1)

<400> 56
   Met Glu Ala His Gln Phe Ile Lys Ala Pro Gly Val Thr Thr Ala Ile
     1               5                  10                  15
   Glu Gln Ala Ala Leu Ala Ala Ala Asn Ser Ala Leu Ala Asn Ala Val
   20 25 30
   Val Val Arg Pro Phe Leu Ser Arg Leu Gln Thr Glu Ile Leu Ile Asn
   35 40 45
   Leu Met Gln Pro Arg Gln Leu Val Phe Arg Pro Glu Val Leu Trp Asn
   50 55 60
   His Pro Ile Gln Arg Val Ile His Asn Glu Leu Glu Gln Tyr Cys Arg
   65 70 75 80
   Ala Arg Ala Gly Arg Cys Leu Glu Val Gly Ala His Pro Arg Ser Ile
   85 90 95
   Asn Asp Asn Pro Asn Val Leu His Arg Cys Phe Leu Lys Pro Val Gly
   100 105 110
   Arg Asp Val Gln Arg Trp Tyr Thr Ala Pro Thr Arg Gly Pro Ala Ala
   115 120 125
   Asn Cys Arg Arg Ser Ala Leu Arg Gly Leu Pro Pro Ala Asp Arg Thr
   130 135 140
   Tyr Cys Phe Asp Gly Phe Ser Gly Cys Thr Phe Ala Ala Glu Thr Gly
   145 150 155 160
   Val Ala Leu Tyr Ser Leu His Asp Leu Trp Pro Ala Asp Val Ala Glu
   165 170 175
   Ala Met Ala Arg His Gly Met Thr Arg Leu Tyr Ala Ala Leu His Leu
   180 185 190
   Pro Pro Glu Val Leu Leu Pro Pro Gly Thr Tyr His Thr Thr Ser Tyr
   195 200 205
   Leu Leu Ile His Asp Gly Asp Arg Ala Val Ile Thr Tyr Glu Gly Asp
   210 215 220
   Ser Ser Ala Gly Tyr Asn His Asp Val Ser Ile Leu Arg Ala Trp Ile
   225 230 235 240
   Arg Thr Thr Lys Val Thr Gly Asp His Pro Leu Val Ile Glu Arg Val
   245 250 255
   Arg Ala Val Gly Cys His Phe Val Leu Leu Leu Thr Ala Ala Pro Glu
   260 265 270
   Pro Ser Pro Met Pro Tyr Val Pro Tyr Pro Arg Ser Thr Glu Val Tyr
   275 280 285
   Val Arg Ser Ile Phe Gly Pro Gly Gly Ser Pro Ser Leu Phe Pro Ser
   290 295 300
   Ala Cys Ser Thr Lys Ser Thr Phe His Ala Val Pro Val His Ile Trp
   305 310 315 320
   Asp Arg Leu Met Leu Phe Gly Ala Thr Leu Asp Asp Gln Ala Phe Cys
   325 330 335
   Cys Ser Arg Leu Met Thr Tyr Leu Arg Gly Ile Ser Tyr Lys Val Thr
   340 345 350
   Val Gly Ala Leu Val Ala Asn Glu Gly Trp Asn Ala Ser Glu Asp Ala
   355 360 365
   Leu Thr Ala Val Ile Thr Ala Ala Tyr Leu Thr Ile Cys His Gln Arg
   370 375 380
   Tyr Leu Arg Thr Gln Ala Ile Ser Lys Gly Met Lys Arg Leu Glu Leu
   385 390 395 400
   Glu His Ala Gln Lys Phe Ile Thr Arg Leu Tyr Ser Trp Leu Phe Glu
   405 410 415
   Lys Ser Gly Arg Asp Tyr Ile Pro Gly Arg Gln Leu Gln Phe Tyr Ala
   420 425 430
   Gln Cys Arg Arg Trp Leu Ser Ala Gly Phe His Leu Asp Pro Arg Val
   435 440 445
   Leu Val Phe Asp Glu Ala Ala Pro Cys Arg Cys Arg Ser Leu Leu Arg
   450 455 460
   Lys Ala Ala His Lys Phe Cys Cys Phe Met Arg Trp Leu Gly Gln Asp
   465 470 475 480
   Cys Thr Cys Phe Leu Gln Pro Ile Glu Gly Arg Val Gly Glu Gln Gly
   485 490 495
   Tyr Asp Asn Glu Ala Phe Glu Gly Ser Asp Val Asp Pro Ala Glu Glu
   500 505 510
   Ala Thr Val Ser Ile Ser Gly Ser Tyr Ile Val Thr Gly Ser Gln Leu
   515 520 525
   Gln Pro Leu Tyr Gln Ala Leu Gly Ile Pro Ser Asp Leu Ala Ala Arg
   530 535 540
   Ala Gly Arg Leu Thr Ala Thr Val Glu Val Ser Asp Ala Asp Gly Arg
   545 550 555 560
   Leu Thr Cys Lys Thr Ile Met Gly Asn Lys Thr Phe Thr Thr Val Phe
   565 570 575
   Thr Asp Gly Ala Gln Leu Glu Val Asn Gly Pro Glu Gln Tyr Val Leu
   580 585 590
   Ser Phe Asp Leu Ala Lys Gln Thr Met Ala Ala Gly Pro His Ser Leu
   595 600 605
   Ser Tyr Val Leu Thr Ser Ala Gly Leu Glu Val Arg Val Val Ser Ala
   610 615 620
   Gly Leu Asp Cys Lys Ala Val Phe Pro Ser Gly Val Ala Thr Pro Ser
   625 630 635 640
   Ala Ser Gly Glu Val Ser Ala Phe Cys Ser Ala Leu Tyr Arg Phe Asn
   645 650 655
   Arg Cys Val Gln Arg His Ser Leu Ile Gly Gly Leu Trp Tyr Tyr Pro
   660 665 670
   Glu Gly Leu Ile Gly Leu Phe Pro Pro Phe Ala Pro Gly His Thr Trp
   675 680 685
   Glu Ser Thr Asn Pro Phe Cys Gly Glu Ser Thr Leu Tyr Thr Arg Thr
   690 695 700
   Trp Ser Val Ser Gly Phe Ser Ser Cys Phe Ser Pro Leu Glu Pro Pro
   705 710 715 720
   Ala Ser Gly Ser Leu Pro Pro Ala Glu Ile Asp Pro Pro Val Thr Val
   725 730 735
   Asp Ala Pro Ser Pro Ser Ile Leu Ala Leu Pro Arg Pro Ser Val Phe
   740 745 750
   Glu Gln Thr Thr Pro Pro Leu Asp Pro Ala Gly Asp Ala Ala Ala Ser
   755 760 765
   Ala Pro Pro Gly Ala Pro Gly Val Pro Pro Ala Pro Ala Arg Pro Val
   770 775 780
   Thr His Pro Ser Gly Pro Arg Arg Arg Leu Leu His Thr Tyr Pro Asp
   785 790 795 800
   Gly Ser Lys Val Tyr Ala Gly Ser Leu Phe Glu Ser Glu Cys Thr Trp
   805 810 815
   Leu Val Asn Ala Ser Asn Pro Gly His Arg Pro Gly Gly Gly Leu Cys
   820 825 830
   His Ala Phe Tyr Gln Arg Phe Pro Glu Ser Phe Asp Pro Ala Glu Phe
   835 840 845
   Val Met Ser Asp Gly Phe Ala Ala Tyr Thr Leu Thr Pro Arg Pro Ile
   850 855 860
   Ile His Ala Val Ala Pro Asp Tyr Arg Val Glu His Asn Pro Lys Arg
   865 870 875 880
   Leu Glu Ala Ala Tyr Arg Glu Thr Cys Ser Arg Arg Gly Thr Ala Ala
   885 890 895
   Tyr Pro Leu Leu Gly Ala Gly Ile Tyr Lys Val Pro Val Gly Leu Ser
   900 905 910
   Phe Asp Ala Trp Glu Arg Asn His Arg Pro Gly Asp Glu Leu Tyr Leu
   915 920 925
   Thr Glu Pro Ala Ile Ala Trp Phe Glu Ala Asn Arg Pro Thr Leu Pro
   930 935 940
   Ala Leu Thr Ile Thr Glu Asp Thr Ala Arg Thr Ala Asn Leu Ala Leu
   945                 950                 955                 960
   Glu Leu Asp Ser Ala Thr Glu Val Gly Arg Ala Cys Ala Gly Cys Arg
                   965                 970                 975
   Val Glu Pro Gly Val Val His Tyr Gln Phe Thr Ala Gly Val Pro Gly
               980                 985                 990
   Ser Gly Lys Ser Arg Ser Ile Gln Gln Gly Glu Val Asp Val Val Val
           995                 1000                1005
   Val Pro Thr Arg Glu Leu Arg Asn Ser Trp Arg Arg Arg Gly Phe Ala
       1010                1015                1020
   Ala Tyr Thr Pro His Thr Ala Ala Arg Val Thr Arg Gly Arg Arg Ile
   1025                1030                1035                1040
   Val Ile Asp Glu Ala Pro Ala Leu Pro Pro His Leu Leu Leu Leu His
                   1045                1050                1055
   Met Gln Arg Ala Ser Ser Val His Leu Leu Gly Asp Pro Asn Gln Ile
               1060                1065                1070
   Pro Ala Ile Asp Phe Glu His Ala Gly Leu Val Pro Ala Ile Arg Pro
           1075                1080                1085
   Glu Leu Val Pro Thr Lys Trp Trp His Leu Thr His Arg Cys Pro Ala
       1090                1095                1100
   Asp Val Cys Glu Leu Ile Arg Gly Ala Tyr Pro Lys Ile Gln Thr Ala
   1105                1110                1115                1120
   Ser Arg Val Leu Arg Ser Leu Phe Trp Gly Glu Pro Pro Val Gly Gln
                   1125                1130                1135
   Lys Leu Val Phe Thr Gln Ala Ala Lys Ala Ala Asn Pro Gly Ala Ile
               1140                1145                1150
   Thr Val His Glu Ala Gln Gly Ala Thr Phe Thr Glu Thr Thr Ile Ile
           1155                1160                1165
   Ala Thr Ala Asp Ala Arg Gly Leu Ile Gln Ser Ser Arg Ala His Ala
       1170                1175                1180
   Ile Val Ala Leu Thr Arg His Thr Glu Lys Cys Val Val Val Asp Ala
   1185                1190                1195                1200
   Pro Gly Leu Leu Arg Glu Val Gly Ile Ser Asp Ala Ile Val Asn Asn
                   1205                1210                1215
   Phe Phe Leu Ser Gly Gly Gln Ile Gly Gln His Arg Pro Ser Val Ile
               1220                1225                1230
   Pro Arg Gly Thr Val Asp Ser Asn Val Asp Thr Leu Asp Ala Phe Pro
           1235                1240                1245
   Pro Ser Cys Gln Phe Ser Ala Tyr His Gln Leu Ala Glu Glu Leu Gly
   1250                1255                1260
   His Arg Pro Ala Pro Ile Ala Ala Val Leu Pro Pro Cys Pro Glu Leu
   1265                1270                1275                1280
   Glu Gln Gly Leu Leu Tyr Met Pro Gln Glu Leu Thr Thr Ser Asp Ser
                   1285                1290                1295
   Val Leu Thr Phe Glu Leu Thr Asp Ile Val His Cys Arg Met Ala Ala
               1300                1305                1310
   Pro Ser Gln Arg Lys Ala Val Leu Ser Thr Leu Val Gly Arg Tyr Gly
           1315                1320                1325
   Arg Arg Thr Lys Leu Tyr Glu Ala Ala His Ala Asp Val Arg Gly Ser
       1330                1335                1340
   Leu Asn His Phe Ile Pro Glu Leu Gly Pro Ile Ser Val Thr Thr Cys
   1345                1350                1355                1360
   Glu Leu Tyr Glu Leu Val Glu Ala Met Val Glu Lys Gly Gln Asp Gly
                   1365                1370                1375
   Ser Ala Val Leu Glu Leu Asp Leu Cys Ser Arg Asp Val Ser Arg Ile
               1380                1385                1390
   Thr Phe Phe Gln Lys Asp Cys Asn Lys Phe Thr Thr Gly Glu Thr Ile
           1395                1400                1405
   Ala His Gly Lys Val Gly Gln Gly Ile Ser Ala Trp Ser Lys Thr Phe
   1410 1415 1420
   Cys Ala Leu Phe Gly Pro Trp Phe Arg Ala Ile Glu Lys Glu Ile Leu
   1425 1430 1435 1440
   Ala Ala Leu Ala Pro Asn Val Phe Tyr Gly Asp Ala Tyr Glu Asp Thr
   1445 1450 1455
   Val Leu Ala Ala Ala Val Ala Gly Ala Pro Gly Cys Lys Val Phe Glu
   1460 1465 1470
   Asn Asp Phe Ser Glu Phe Asp Ser Thr Gln Asn Asn Phe Ser Leu Gly
   1475 1480 1485
   Leu Glu Cys Ile Ile Met Glu Glu Cys Gly Met Pro Gln Trp Met Ile
   1490 1495 1500
   Arg Leu Tyr His Leu Val Arg Ser Ala Trp Val Leu Gln Ala Pro Lys
   1505 1510 1515 1520
   Glu Ser Leu Arg Gly Phe Trp Lys Lys His Ser Gly Glu Pro Gly Thr
   1525 1530 1535
   Leu Leu Trp Asn Thr Val Trp Asn Met Ala Val Ile Ala His Cys Tyr
   1540 1545 1550
   Glu Phe Arg Asp Leu Lys Val Ala Ala Phe Lys Gly Asp Asp Ser Val
   1555 1560 1565
   Val Leu Cys Ser Asp Tyr Arg Gln Ser Arg Asn Ala Ala Ala Leu Ile
   1570 1575 1580
   Thr Gly Cys Gly Leu Lys Leu Lys Val Asp Phe Arg Pro Ile Gly Leu
   1585 1590 1595 1600
   Tyr Ala Gly Val Val Val Ala Pro Gly Leu Gly Thr Leu Pro Asp Val
   1605 1610 1615
   Val Arg Phe Ala Gly Arg Leu Ser Glu Lys Asn Trp Gly Pro Gly Pro
   1620 1625 1630
   Glu Arg Ala Glu Gln Leu Arg Leu Ala Val Cys Asp Phe Leu Arg Lys
   1635 1640 1645
   Leu Thr Asn Val Ala Gln Val Cys Val Asp Val Val Ser Arg Val Tyr
   1650 1655 1660
   Gly Val Ser Pro Gly Leu Val His Asn Leu Ile Gly Met Leu Gln Thr
   1665 1670 1675 1680
   Ile Ala Asp Gly Lys Ala His Phe Thr Glu Thr Val Lys Pro Val Leu
   1685 1690 1695
   Asp Leu Thr Asn Ser Ile Ile His Arg Val Glu
   1700 1705

<210> 57

<211> 674

<212> PRT

<213> Hepatitis E Virus JAK-Sai (ORF2)

<400> 57

Met Asn Asn Met Phe Phe Cys Ser Val His Gly Asp Ala Thr Met Arg
1               5                   10                  15

Ser Arg Ala Leu Leu Phe Leu Leu Phe Val Phe Leu Pro Met Leu Pro
            20                  25                  30

Ala Pro Pro Ala Gly Gln Pro Ser Gly Arg Arg Arg Gly Arg Arg Ser
        35                  40                  45

Gly Gly Ala Gly Gly Gly Phe Trp Gly Asp Arg Val Asp Ser Gln Pro
    50                  55                  60

Phe Ala Leu Pro Tyr Ile His Pro Thr Asn Pro Phe Ala Ser Asp Ile
65                  70                  75                  80

Pro Thr Ala Ala Gly Ala Gly Ala Arg Pro Arg Gln Pro Ala Arg Pro
                85                  90                  95

Leu Gly Ser Ala Trp Arg Asp Gln Ser Gln Arg Pro Thr Ala Ser Ala
            100                 105                 110

Arg Arg Arg Pro Ala Pro Ala Gly Ala Ala Pro Leu Thr Ala Val Ala
        115                 120                 125

Pro Ala Pro Asp Thr Ala Pro Val Pro Asp Val Asp Ser Arg Gly Ala
    130                 135                 140

Ile Leu Arg Arg Gln Tyr Asn Leu Ser Thr Ser Pro Leu Thr Ser Thr
145                 150                 155                 160

Ile Ala Thr Gly Thr Asn Leu Val Leu Tyr Ala Ala Pro Leu Ser Pro
                165                 170                 175

Leu Leu Pro Leu Gln Asp Gly Thr Asn Thr His Ile Met Ala Thr Glu
            180                 185                 190

Ala Ser Asn Tyr Ala Gln Tyr Arg Val Val Arg Ala Thr Ile Arg Tyr
        195                 200                 205

Arg Pro Leu Val Pro Asn Ala Val Gly Gly Tyr Ala Ile Ser Ile Ser
    210                 215                 220

Phe Trp Pro Gln Thr Thr Thr Thr Pro Thr Ser Val Asp Met Asn Ser
225                 230                 235                 240

Ile Thr Ser Thr Asp Val Arg Ile Leu Val Gln Pro Gly Ile Ala Ser
                245                 250                 255

Glu Leu Val Ile Pro Ser Glu Arg Leu His Tyr Arg Asn Gln Gly Trp
            260                 265                 270

Arg Ser Val Glu Thr Ser Gly Val Ala Glu Glu Glu Ala Thr Ser Gly

        275                 280                 285

Leu Val Met Leu Cys Ile His Gly Ser Pro Val Asn Ser Tyr Thr Asn
    290                 295                 300

Thr Pro Tyr Thr Gly Ala Leu Gly Leu Leu Asp Phe Ala Leu Glu Leu
305                 310                 315                 320

Glu Phe Arg Asn Leu Thr Pro Gly Asn Thr Asn Thr Arg Val Ser Arg
                325                 330                 335

Tyr Ser Ser Ser Ala Arg His Lys Leu Arg Arg Gly Pro Asp Gly Thr
            340                 345                 350

Ala Glu Leu Thr Thr Thr Ala Ala Thr Arg Phe Met Lys Asp Leu His
        355                 360                 365

Phe Thr Gly Thr Asn Gly Val Gly Glu Val Gly Arg Gly Ile Ala Leu
    370                 375                 380

Thr Leu Phe Asn Leu Ala Asp Thr Leu Leu Gly Gly Leu Pro Thr Glu
385                 390                 395                 400

Leu Ile Ser Ser Ala Gly Gly Gln Leu Phe Tyr Ser Arg Pro Val Val
                405                 410                 415

Ser Ala Asn Gly Glu Pro Thr Val Lys Leu Tyr Thr Ser Val Glu Asn
            420                 425                 430

Ala Gln Gln Asp Lys Gly Ile Ala Ile Pro His Asp Ile Asp Leu Gly
        435                 440                 445
Glu Ser Arg Val Val Ile Gln Asp Tyr Asp Asn Gln His Glu Gln Asp
    450                 455                 460
Arg Pro Thr Pro Ser Pro Ala Pro Ser Arg Pro Phe Ser Val Leu Arg
465                 470                 475                 480
Ala Asn Asp Val Leu Trp Leu Ser Leu Thr Ala Ala Glu Tyr Asp Gln
                485                 490                 495
Thr Thr Tyr Gly Ser Ser Thr Asn Pro Met Tyr Val Ser Asp Thr Val
            500                 505                 510
Thr Phe Val Asn Val Ala Thr Gly Ala Gln Gly Val Ser Arg Ser Leu
        515                 520                 525
Asp Trp Ser Lys Val Thr Leu Asp Gly Arg Pro Leu Thr Thr Ile Gln
    530                 535                 540
Gln Tyr Ser Lys Thr Phe Phe Val Leu Pro Leu Arg Gly Lys Leu Ser
545                 550                 555                 560
Phe Trp Glu Ala Gly Thr Thr Lys Ala Gly Tyr Pro Tyr Asn Tyr Asn
                565                 570                 575
Thr Thr Ala Ser Asp Gln Ile Leu Ile Glu Asn Ala Ala Gly His Arg
            580                 585                 590

Val Cys Ile Ser Thr Tyr Thr Thr Asn Leu Gly Ser Gly Pro Val Ser
        595                 600                 605

Ile Ser Ala Val Gly Val Leu Ala Pro His Ser Ala Leu Ala Ala Leu
    610                 615                 620

Glu Asp Thr Val Asp Tyr Pro Ala Arg Ala His Thr Phe Asp Asp Phe
625                 630                 635                 640

Cys Pro Glu Cys Arg Thr Leu Gly Leu Gln Gly Cys Ala Phe Gln Ser
                645                 650                 655

Thr Val Ala Glu Leu Gln Arg Leu Lys Met Lys Val Gly Lys Thr Arg
            660                 665                 670

Glu Tyr